# EUROPEAN PATENT APPLICATION

(11) **EP 0 184 844 A1**
(43) Date of publication of application: **18.06.1986**
(21) Application number: 85115846.9
(22) Date of filing: 12.12.1985
(51) Int. Cl.: C07D 487/04, A61K 31/40, C07F 9/65, C07F 7/18

(54) **1-Methylcarbapenems having a 2-position substituent joined through an alkylenethio bridge**

(30) Priority: 13.12.1984 US 681407
(71) Applicant: Merck & Co., Inc., Rahway New Jersey 07065-0900 (US)
(72) Inventor: Salzmann, Thomas N., North Plainfield New Jersey 07060 (US); Christensen, Burton G., Fanwood New Jersey 07023 (US); Heck, James V., Scotch Plains New Jersey 07076 (US); Shih, David H., Manalpan New Jersey 07726 (US)
(74) Representative: Abitz, Walter, Dr.-Ing.

(57) **Abstract**

1-Methylcarbapenems having the formula: 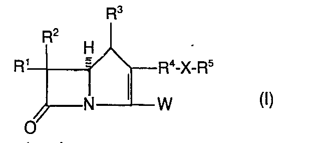
wherein:
R' and R² are independently H, CH₃-, CH₃CH₂-, (CH₃)₂CH-, HOCH₂-, CH₃CH(OH)-, (CH₃)₂C(OH)-, FCH₂, F₂CH-, F₃C-, CH₃CH(F)-, CH₃CF₂- or (CH₃)₂C(F)-;
_{R}³ is CH₃;
R⁴ is a bridging group comprising substituted or unsubstituted C₁-C₄ straight, C₂-C₆ branched or C₃-C₇ cycloalkyl groups wherein the substituents are selected from C₁-C₆ alkyl, O-C₁-C₆ alkyl, S-C₁-C₆ alkyl, CF₃, N(C₁-C₆ alkyl)₂;
X is -S-. 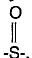 -SO₂-, -O- or -NH: and
R⁵ is, e. g., C₁-C₁₀ straight or branched alkyl; their preparation and antibiotic use are disclosed.

## Description

### BACKGROUND OF THE INVENTION

The present invention is concerned with 1-methylcarbapenem antibiotics having a 2-position substituent joined through an alkylenethio bridge.

Thienamycin is a known carbapenem, broad spectrum antibiotic of the formula: 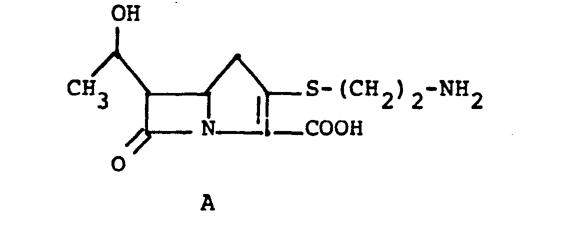 Other derivatives of A are also known.

The present 2-substituted 1-methylcarbapenems have an antibiotic spectrum equal to or better than A. The present 1-methylcarbapenems also are more resistant than A to degradation by the dehydropeptidase enzyme DHP-I. The present 1-methylcarbapenems are also more resistant to DHP-I mediated degradation and are chemically more stable, both in solution and in the solid state, than the corresponding carbapenems lacking the 1-methyl substituent.

### BRIEF DESCRIPTION OF THE PRIOR ART

Sankyo U.S. Pat. No. 4,377,591 and Japanese patent publications 56-199682 and 56-60852; Shionogi Japanese patent publications 57-145086 and 57-145087; and Roche U.K. patent publication 2 092 147A, all describe azabicycloheptene antibiotics having a 2-position substituent joined through an alkylenethio bridge. However, none of these references describe or suggest the 1-methylcarbapenem compounds of the present invention and the superior stability obtained therewith.

### SUMMARY OF THE INVENTION

1-Methylcarbapenems having the formula: 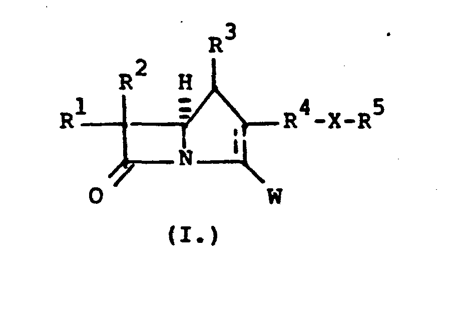 wherein R1 and R2 are independently H, CH₃-, CH₃CH₂-, (CH₃)₂CH-, HOCH₂-, CH₃CH(OH)-, (CH₃)₂C(OH)-, FCH₂-, ^{F}₂^{CH-, F}₃C-, CH₃CH(F)-, CH₃CF₂-, or (CH₃)₂C(F)-;
R⁴ is a bridging group comprising substituted or unsubstituted C₁-C₄ straight, C₂-C₆ branched or C₃-C₇ cycloalkyl groups wherein the substituents are selected from C₁-C₆ alkyl, O-C₁-C₆ alkyl, S-C₁-C₆ alkyl, CF₃, N(C₁-C₆ alkyl)₂;
and X is -S-, 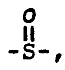 -S0₂-, -O-, or -NH; their preparation and antibiotic use are disclosed.

### DETAILED DESCRIPTION OF THE INVENTION

The invention is embodied in a compound having the formula: 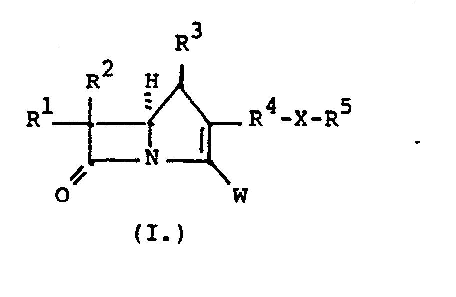 wherein R1 and R2 are independently H, CH₃-,
CH₃CH₂-, (CH₃)₂CH-, HOCH₂-, CH₃CH(OH)-, (CH₃)₂C(OH)-, FCH₂-, F₂CH-, F₃C-, CH₃CH(F)-, CH₃CF₂-, or (CH₃)₂C(F)-;
R³ is CH₃-;
R⁴ is a bridging group comprising substituted or unsubsfituted Cₗ-C₄ straight, C₂-C₆ branched or C₃-C₇ cycloalkyl groups wherein the substituents are selected from Cₗ-C₆ alkyl, O-C₁-C₆ alkyl, S-C₁-C₆ alkyl, CF₃,
N(C₁-C₆ alkyl)₂;
X is -S-, -SO-, -SO₂-, -0-, or -NH-; R⁵ is selected from the group consisting of:

### 1. Aliphatic

-C₁-C₁₀ straight or branched alkyl; C₃-C₈ cycloalkyl; C₂-C₁₀ straight or branched alkenyl; C₃-C₈ cycloalkenyl; or C₂-C₁₀ straight or branched alkynyl;

### 2. Substituted Aliphatic

-C₁-C₁₀ branched or unbranched alkyl; C₂-C₁₀ branched or unbranched alkenyl, or akynyl; or C₃-C₈ cycloalkyl or cycloalkenyl: wherein all of the above groups are substituted by one or more halo, OR,, 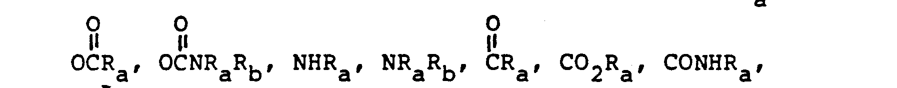 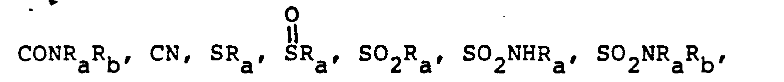 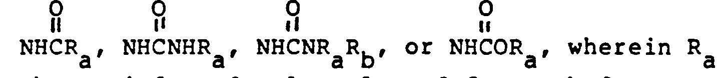 and Rb are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroalkyl, heteroaralkyl, heterocyclyo and heterocyclyoalkyl and wherein the heteroatom or atoms in the above-named heterocyclic moieties are selected from the group consisting of 1-4 oxygen, nitrogen or sulfur atoms and wherein the alkyl moieties associated with said heterocyclic moieties have 1-6 carbon atoms;

### 3. Aryl

-phenyl; or phenyl substituted with 1-3 substituents selected from the group consisting of those substituents set forth above under "Substituted Aliphatic";

### 4. Heterocyclic

-mbno- or bicyclic heterocyclic group containing from 5 to 11 total atoms of which 1 to 5 are heteroatoms selected from nitrogen, oxygen, and sulfur; or substituted heterocyclic where heterocyclic is as defined immediately above and the substituents are 1 to 3 in number and are selected from the group consisting of those substituents set forth above under "Substituted Aliphatic";

### 5. Arylaliphatic

-the group: -Y-Aryl, where "Aryl" is as defined above, including substituted aryl; and Y is C₁-C₆ straight or branched alkyl, C₃-C₈ cycloalkyl, or C₂-C₆ alkenyl or alkynyl;

### 6. Heterocycloaliphatic

-the group: -Y-Heterocyclic, where "Heterocyclic" is as defined above, including substituted heterocyclic; and Y is as defined above;

### 7. Alkyl-Heteroatom-Alkyl

-the group: -(CH₂)ₙ-X (CH₂)ₙ,-R_{c}, where n and n' are independently 2-4; X is NR, O, or S where R is H, CH₃, CH₂CH₃, CH₂CH₂0H, or CH₂CH₂NH₂; and R_{C} is OH, NH_{2'} NHCH₃, N(CH₃)₂, 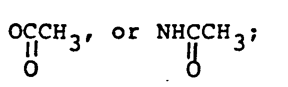 provided that the methylene carbons of the group formula may additidnally be substituted with C₁-C₃ alkyl to form branched alkyl groups;

### 8. Heteroarylium

-the group: 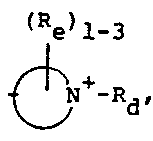 which is a mono- or bicyclic heteroarylium group containing from 5-11 ring atoms of which up to 5 are heteroatoms wherein R_{d} is:
1) an unsubstituted or substituted ^{C}ₗ-C₆ alkyl radical;
2) an unsubstituted or substituted C₂-C₆ alkenyl radical;
3) an unsubstituted or substituted ^{C}₂-C₆ alkynyl radical;
4) a C₃-C₇ cycloalkyl radical in which the ring is substituted or unsubstituted and one or more atoms may be replaced by a heteroatom;
5) a C₃-C₇ cycloalkyl methyl radical in which the ring may be substituted and one or more atoms may be replaced by a heteroatom;
6) an unsubstituted or substituted C₅-C₇ cycloalkenyl radical;
7) an unsubstituted or substituted bivalent C₂-C₆ alkylidene radical, optionally interrupted by a heteroatom, and joined to the heteroarylium group to form a ring which is carbocyclic or in which bne or more atoms is replaced by a heteroatom. The new ring may contain one or more double bonds;
8) an unsubstituted or substituted phenyl or heteroaryl radical;
9) an unsubstituted or substituted phenyl (C₁-C₄ alkyl) or heteroaryl (^{C}₁-^{C}₄ alkyl) radical;
10) a cyano (Cₗ-C₄ alkyl) radical;
11) a carboxy (C₁-C₄ alkyl) radical;
12) a sulfo (C₁₋C₄ alkyl) radical;
13) a carbamoyl (C₁-C₄ alkyl) radical;
14) a phosphonyl (C₁-C₄ alkyl) radical;
15) a hydroxy (C₁-C₄ alkyl) radical;
16) an amino (C₁-C₄ alkyl) radical in which the nitrogen atom is unsubstituted or substituted with one to three C₁-C₄ alkyl groups; wherein the substituents in the above definitions of R_{d} are independently selected from the group consisting of the definitions of Rₑ set out below;

Rₑ is independently selected from:
a) a trifluoromethyl group;
b) a halogen atom;
c) an unsubstituted or substituted C₁-C₄ alkoxyl radical;
d) a hydroxy group;
e) an unsubstituted or substituted (Cₗ-C₆ alkyl) carbonyloxy radical;
f) a carbamoyloxy radical which is unsubstituted or substituted on nitrogen with one or two C₁-C₄ alkyl groups;
^{g}) a C₁-C₆ alkylthio radical, C₁-C₆ alkylsulfinyl radical or C₁-C₆ alkylsulfonyl radical, each of which is unsubstituted or substituted on the alkyl group;
h) a sulfo group;
i) a sulfamoyl group which is unsubstituted or substituted on nitrogen by one or two C₁-C₄ alkyl groups;
j) an amino group;
k) a mono (C₁-C₄ alkyl) amino or di(C₁-C₄ alkyl)amino group, each of which is unsubstituted or substituted on the alkyl group;
1) a formylamino group;
m) an unsubstituted or substituted (C₁-C₆ alkyl)carbonylamino radical;
n) a (C₁-C₄ alkoxyl) carbonylamino radical;
o) a ureido group in which the terminal nitrogen is unsubstituted or substituted with one or two C₁-C₄ alkyl groups;
p) an arylsulfonamido or a (C₁-C₆ alkyl)sulfonamido group;
q) a cyano group;
r) a formyl or acetalized formyl radical;
s) an unsubstituted or substituted (Cₗ-C₆ alkyl)carbonyl radical wherein the carbonyl is free or acetalized;
t) an unsubstituted or substituted phenylcarbonyl or heteroarylcarbonyl radical;
u) a hydroximinomethyl radical in which the oxygen or carbon atom is optionally substituted by a Cₗ-C₄ alkyl group;
v) a carboxyl group;
w) a (C₁-C₆ alkoxy)carbonyl radical;
x) a carbamoyl radical which is unsubstituted or substituted on nitrogen by one or two C₁-C₄ alkyl groups;
y) an N-hydroxycarbamoyl or N(C₁-C₄ alkoxy)carbamoyl radical in which the nitrogen atom may be additionally substituted by a C₁-C₄ alkyl group;
z) a thiocarbamoyl group;
aa) a 5-(lH)-tetrazolyl group;
ab) an amidino group R' 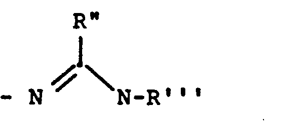 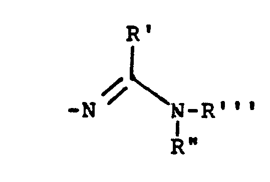 where R', R" and R''' are independently hydrogen, Cₗ-C₄alkyl or wherein two of the alkyl groups together form a C₂-C₆alkylidene radical optionally interrupted by a heteroatom and joined together to form a ring;
ac) a carboxamidino group 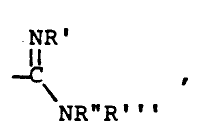 where R', R" and R''' are as defined above;
ad) a guanidinyl group where R" in ab) above is NR^{iv}R^{v} and R^{iv} and R^{v} are as defined for R' through R''' above;
ae) a phosphonate group -P(O)(O⁻)OR^{vi} where R^{vi} is C₁-C₃alkyl;
af) an alkyl phosphonate group -(CH₂)ₙP(O)(O⁻)(OR^{vi}) where n = 1 to 3 and R is C₁-C₃alkyl;
ag) hydrogen;
ah) an unsubstituted or substituted C₁-C₆ alkyl radical;
ai) an unsubstituted or substituted C₂-C₆ alkenyl radical;
aj) an unsubstituted or substituted C₂-C₆ alkynyl radical;
ak) a C₃-C₇ cycloalkyl radical in which the ring is substituted or unsubstituted and one or more atoms may be replaced by a heteroatom;
al) a C₃-C₇ cycloalkyl methyl radical in which the ring may be substituted and one or more atoms may be replaced by a heteroatom;
am) an unsubstituted or substituted C₅-C₇ cycloalkenyl radical;
an) an unsubstituted or substituted phenyl or heteroaryl radical;
ao) an unsubstituted or substituted phenyl (C_{I}-C₄ alkyl) or heteroaryl (C₁-C₄ alkyl) radical; and

### 9. Heteroaryliumaliphatic

-the group: -Y-Heteroarylium, where Y is as defined above; and "Heteroarylium" is as defined above and additionally includes the group: 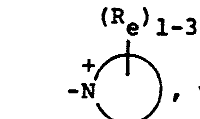
which is a mono- or bicyclic
heteroarylium group containing from 5-11 ring atoms of which up to 5 are heteroatoms, where Rₑ is as defined above;

### 10. Amidino and Amidinium

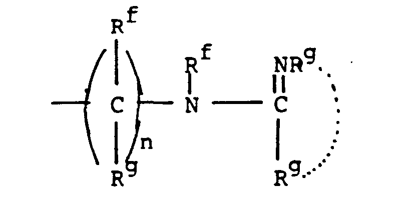

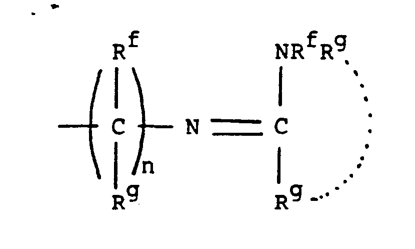

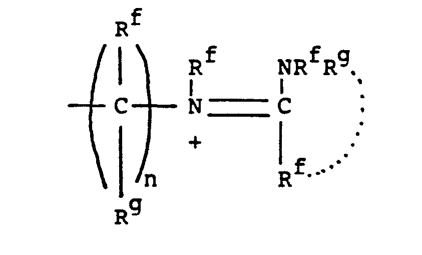

and wherein n = 2-6; R^{f} and R^{g} are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties. have 1-6 carbon atoms; and the dotted line indicates provision for the ring formed by the joinder of substituents carried by the imino carbon atoms; 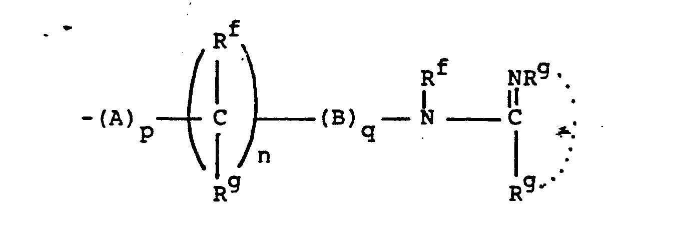 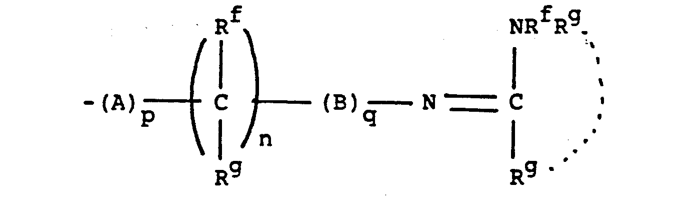 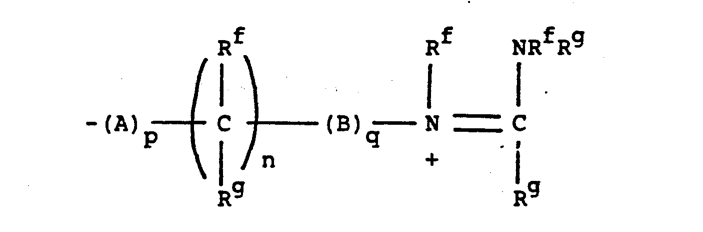
wherein R^{f}, R^{g} and n are as defined immediately above; p and q are 0 or 1; A and B are in bivalent form and are selected from the following definitions above of _{R}⁵: cycloalkyl, alkenyl, cycloalkenyl, alkynyl; substituted: cycloalkyl, alkenyl, cycloalkenyl, alkynyl; phenyl and substituted phenyl; substituted and unsubstituted heteroaryl; arylaliphatic; heteroarylaliphatic, heterocyclylo- aliphatic, and heterocyclic; and alkyl-heteroatom-alkyl; and B can also be selected from -0- and -NR^{f}-.

### 11. Guanidino and Guanidinium

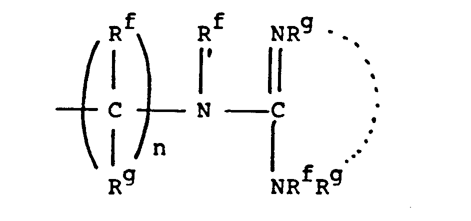

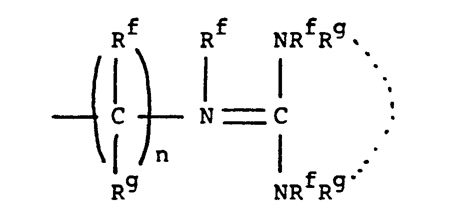

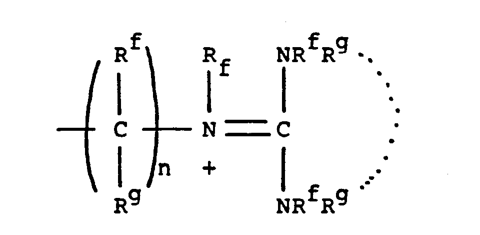

and wherein n = 2-6; R^{f} and R^{g} are as initially defined above under "Amidino and Amidinium"; and the dotted line indicates provision for the ring formed by the joinder of substituents carried by the imino carbon atoms; 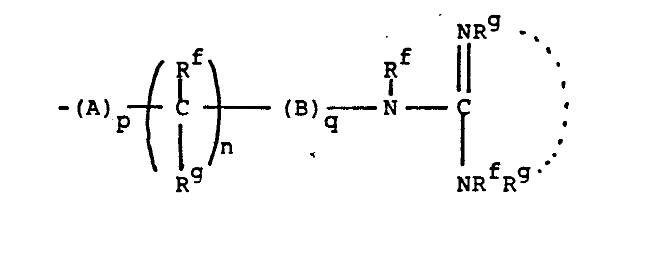 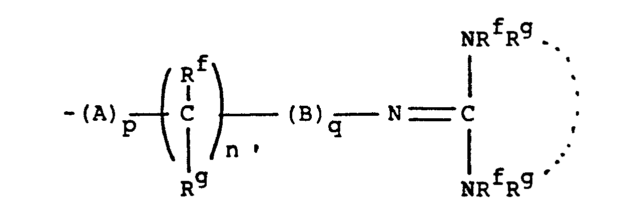 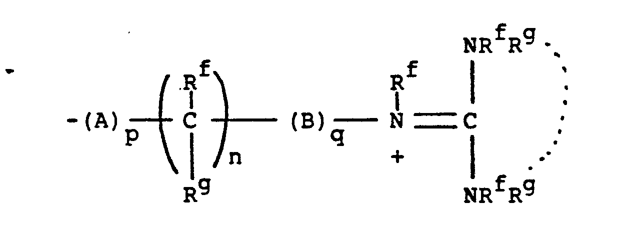
wherein R^{f} and R^{g} and n are as defined immediately above; p and q are 0 or 1; A and B are as defined above under "Amidino and Amidinium".

### 12. Carbamimidoyl and Carbamimidinium

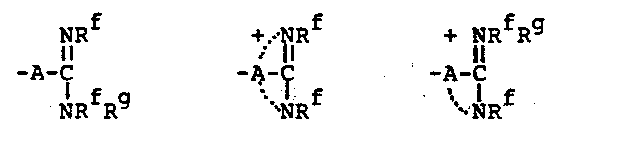

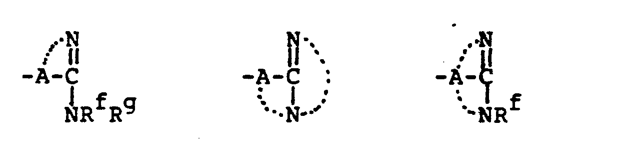

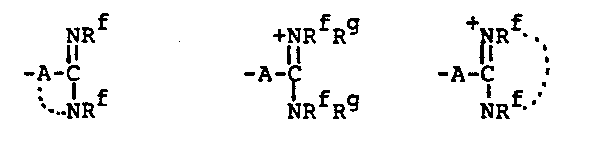

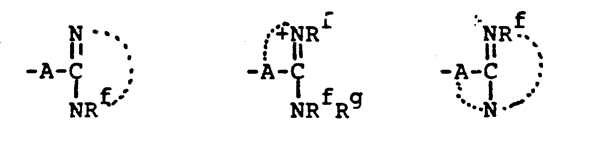

and wherein R and R^{g} are as defined above under "Amidino and Amidinium"; the two nitrogen atoms demonstrated in the above structure may participate in cyclic structures which are indicated by the dotted lines; and A is a connecting group between the sulfur atom and carbamimidoyl function which is bivalent, cyclic or acyclic, is to be read both left to right and right to left, and is selected from: substituted and unsubstituted: loweralkyl having from 1-6 carbon atoms; cycloalkyl having from 3-10 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms, and the alkyl moiety comprises 1 to 6 carbon atoms; alkynyl having from 2 to 10 carbon atoms; aryl, which is defined as phenyl and naphthyl; arylalkyl and alkylaryl; heteroalkyl, alkylheteroalkyl, aryiheteroalkyl and alkylheteroaryl wherein the heteroatoms are selected from the group of sulfur, oxygen and nitrogen, and the alkyl moiety has 1 to 6 carbon atoms, and the aryl moiety is phenyl; heterocyclyo comprising mono-and bicyclic structures having 5 to 10 ring atoms wherein one or more of the heteroatoms is selected from oxygen, nitrogen, or sulphur; heterocycloalkyl wherein the heterocyclyo moiety comprises from 3 to 10 atoms and the alkyl moiety comprises from 1 to 6 atoms; the substituent (or substituents) relative to the above-named radicals are selected from the group consisting of amino, hydroxyl, cyano, carboxyl, nitro, chloro, bromo, fluoro, alkoxy having from 1 to 6 carbon atoms, mercapto, haloloweralkyl, and alkylthio having from 1'-6 carbon atoms;
W is selected from: i) COOH or a pharmaceutically acceptable ester or salt thereof,
   ii) COOR wherein R is a removable carboxy protecting group,
   iii) COOM wherein M is an alkali metal, or
   iv) COO ; provided that when W is other than iv) a counterion Z⁻ is provided.

A preferred group of compounds of the present invention has the structure below: 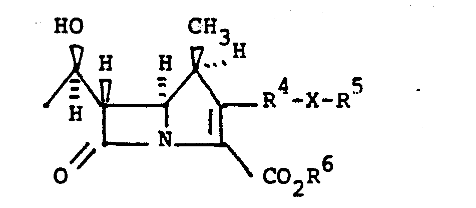
where R⁴, _{R}⁵, R are as defined above.

As used herein, the term "heteroatom" means nitrogen, oxygen, or sulfur, independently selected where more than one heteroatom is involved.

Representative R⁴ groups are -CH₂-, -CH(CH₃)-, -CH(C₂H₅)-, -(CH₂)_{2-4'} -CH(CH₃)-CH₂-, CH₂-CH(OCH₃)-, -CH(CH₃)-(CH₂)₂-, -CH(CH₂OH)-CH₂-, -CH(CF₃)-CH₂-, and the like.

A preferred R⁴ group is -CH₂-, -CH(CH₃)-, (CH₂)₂-, -CH(CH₃)CH₂-, or -CH₂CH(CH₃)-.

A preferred R³ group is -CH₃ in the beta configuration.

Representative R⁵ groups are as follows:
1. Aliphatic 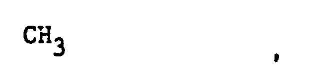 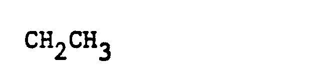 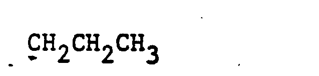 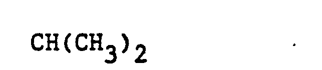 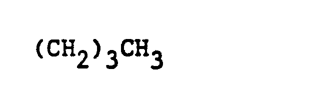 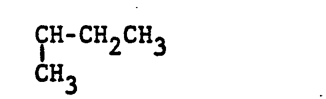 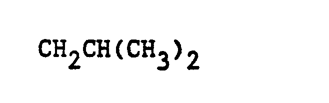 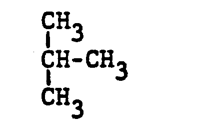 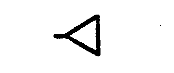 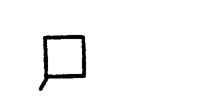 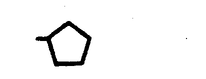 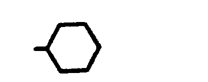 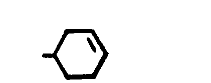 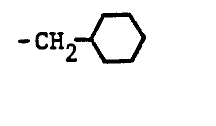 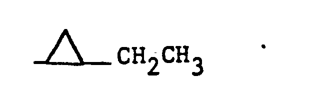 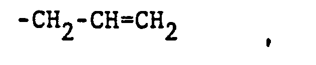 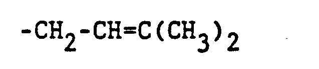 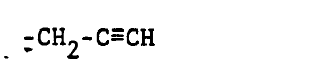 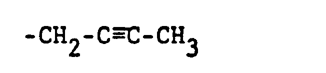
2. Substituted Aliphatic 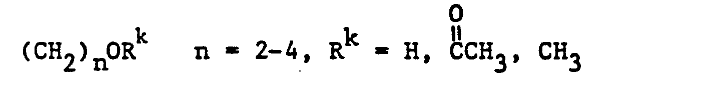 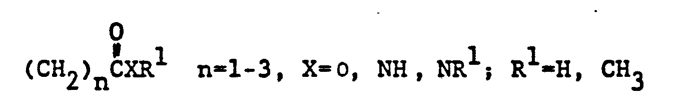 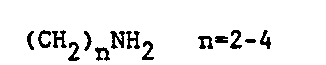 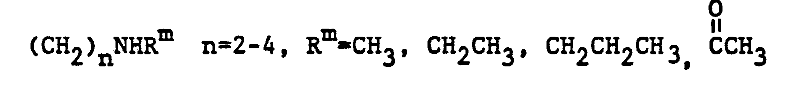 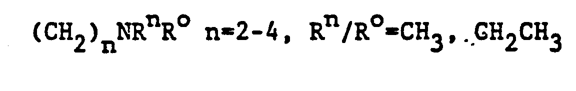 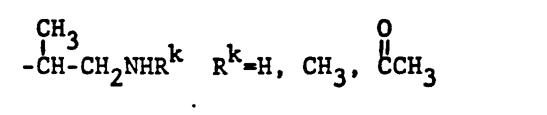 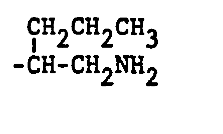 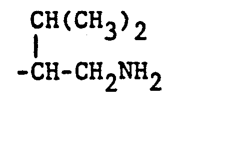 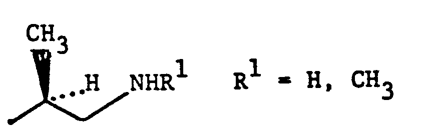 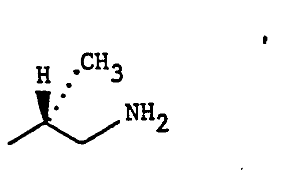 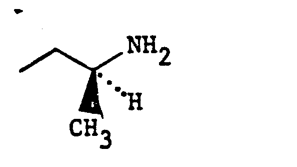 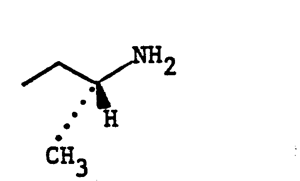 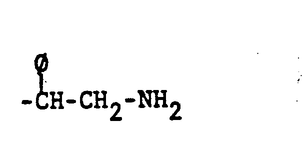 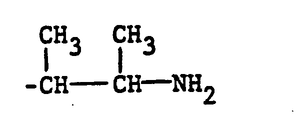 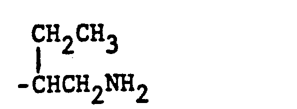 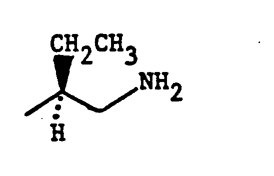 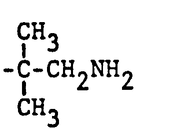 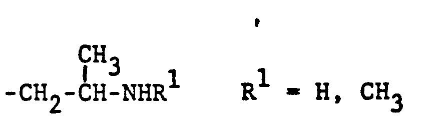 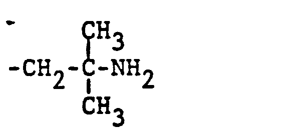 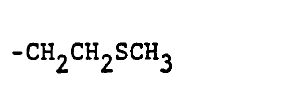 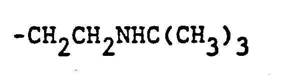 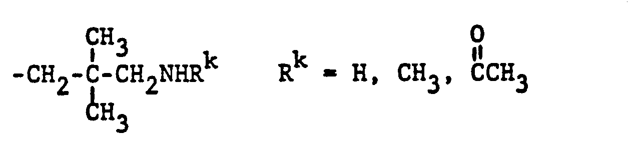 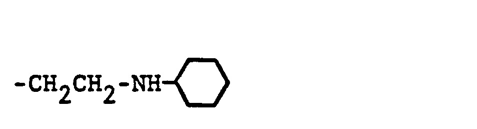 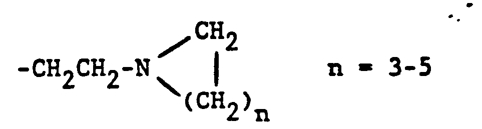 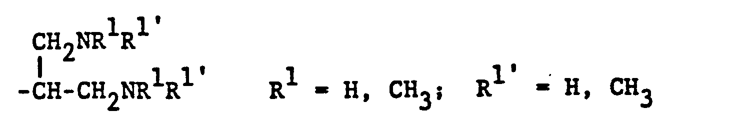 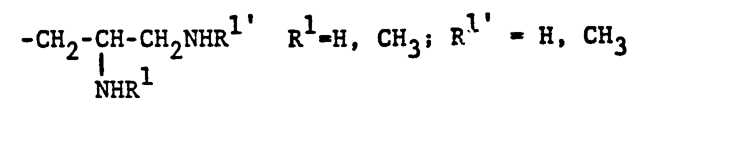 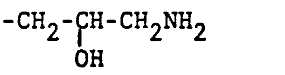 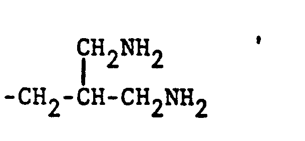 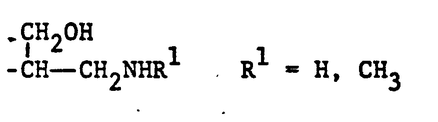 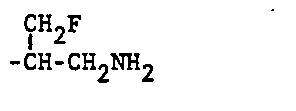 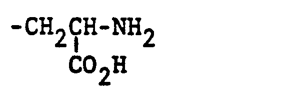 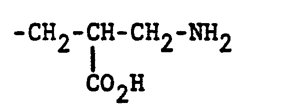 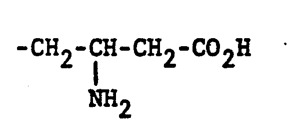 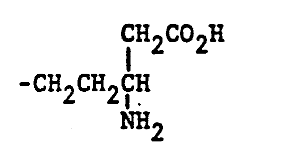 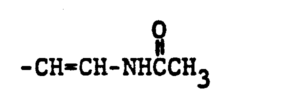 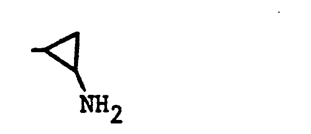 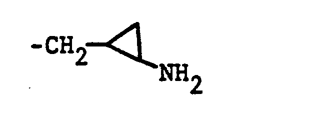 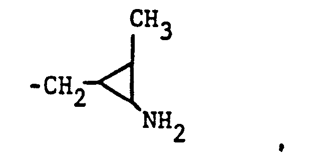 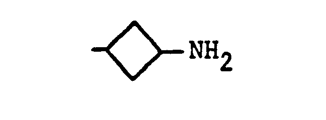 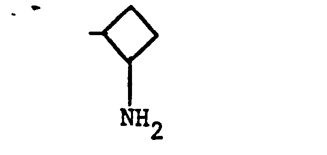 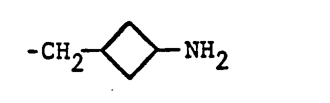 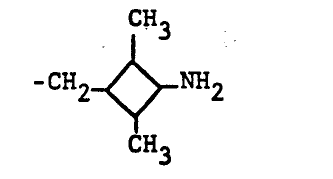 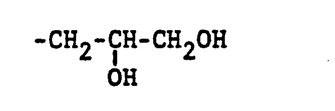 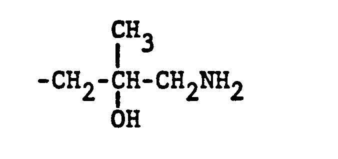 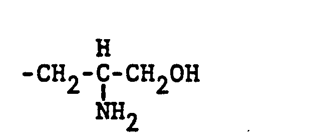 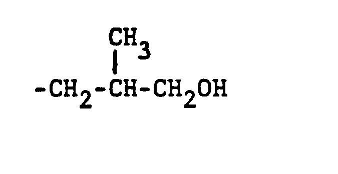 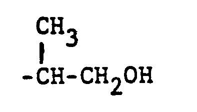 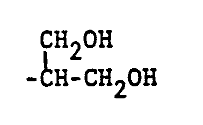 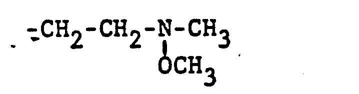 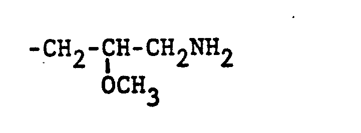 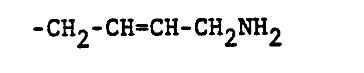 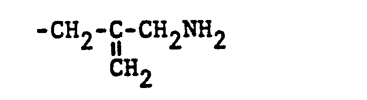 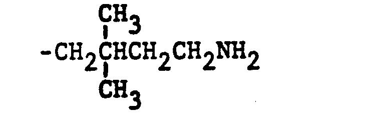 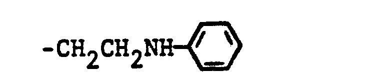 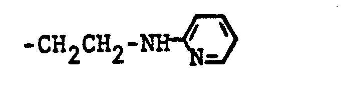 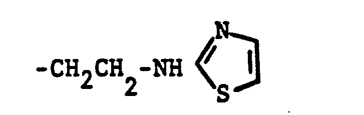 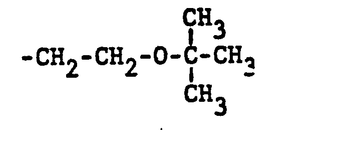
3. Aryl 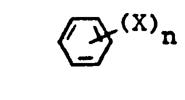 n - 1, 2 or 3, X r F, Cl, Br, OH, OR^{a}, 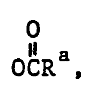 NH₂, NHR^{a}, NR^{a}R^{b}, CH₂NH₂, CH₂NR^{a}R^{b}, CO₂H, CO₂R^{a}, COR^{a}, CONH₂, CONR^{a}R^{b}, R^{a}CONH, _{R}^{a}_{NHCONH}, _{SR}^{a}, 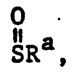 SO₂R^{a}, CH₃, CF₃; R^{a} and _{R}^{b} are as previously defined under "Substituted Aliphatic"; preferred aryl groups are: 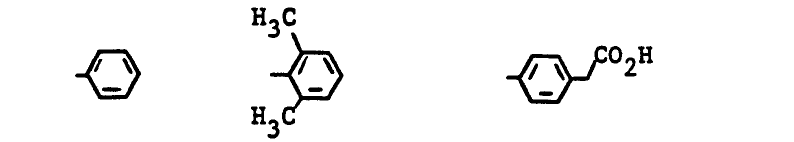 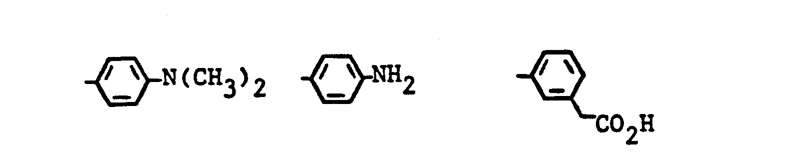 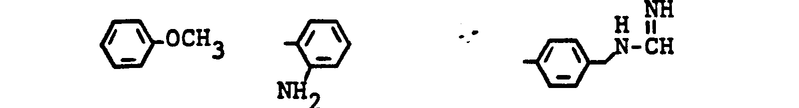 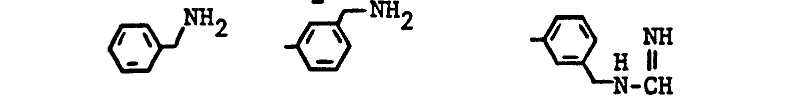 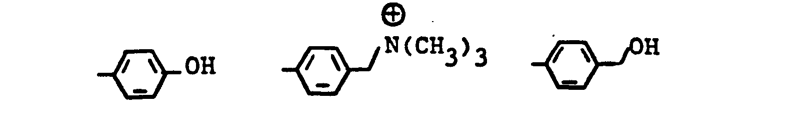 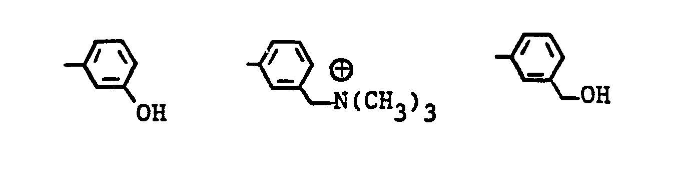 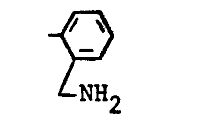 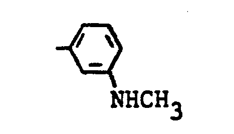 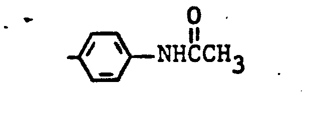 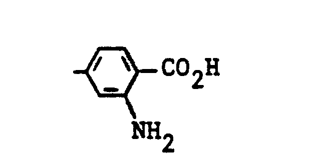
4. Heterocyclic 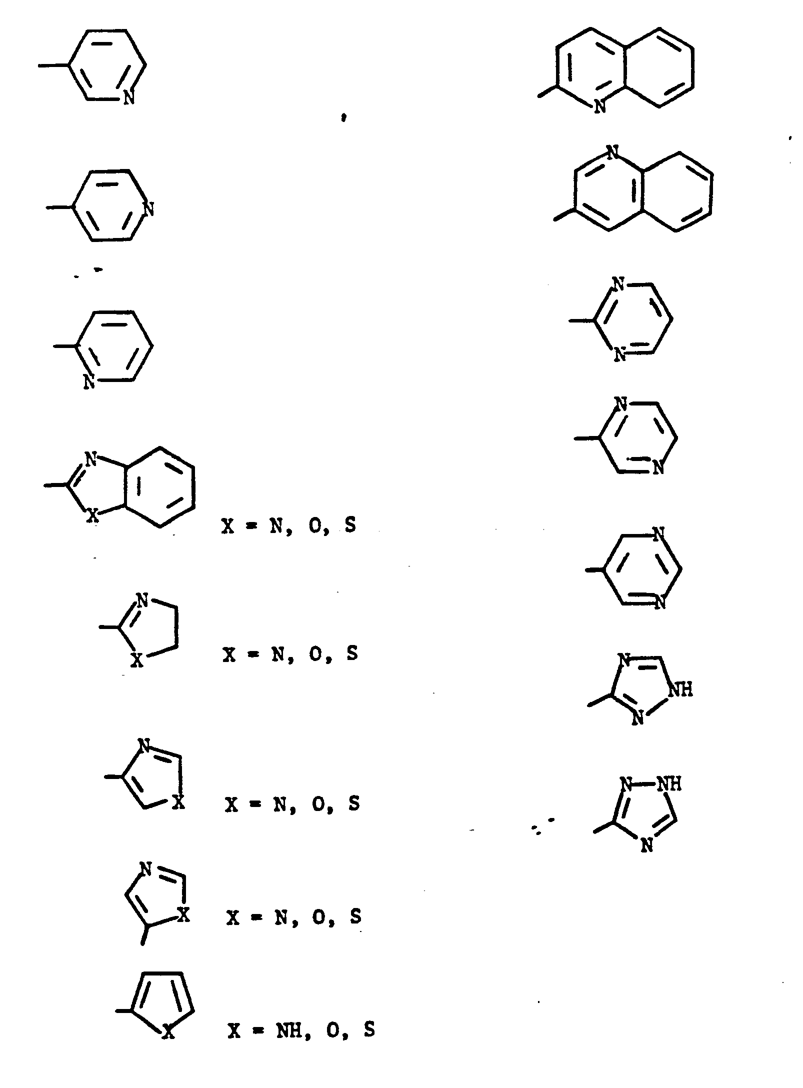 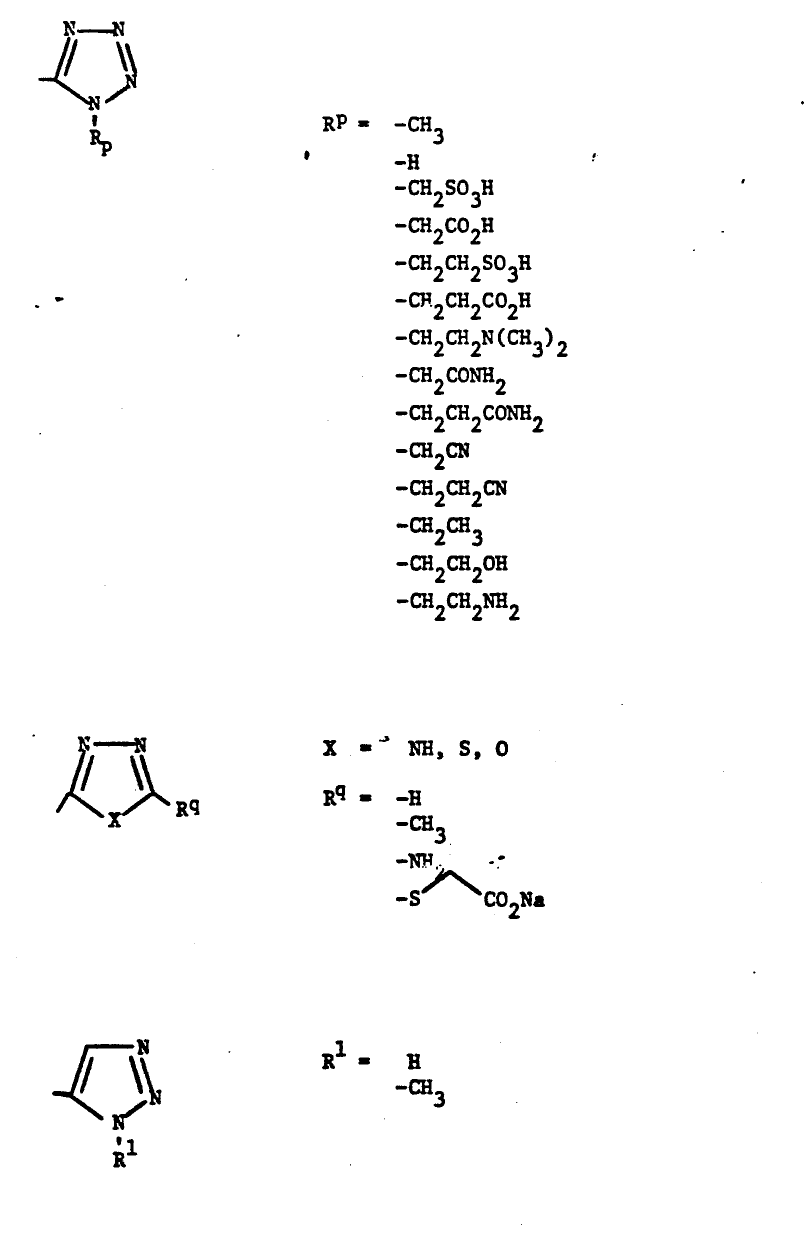 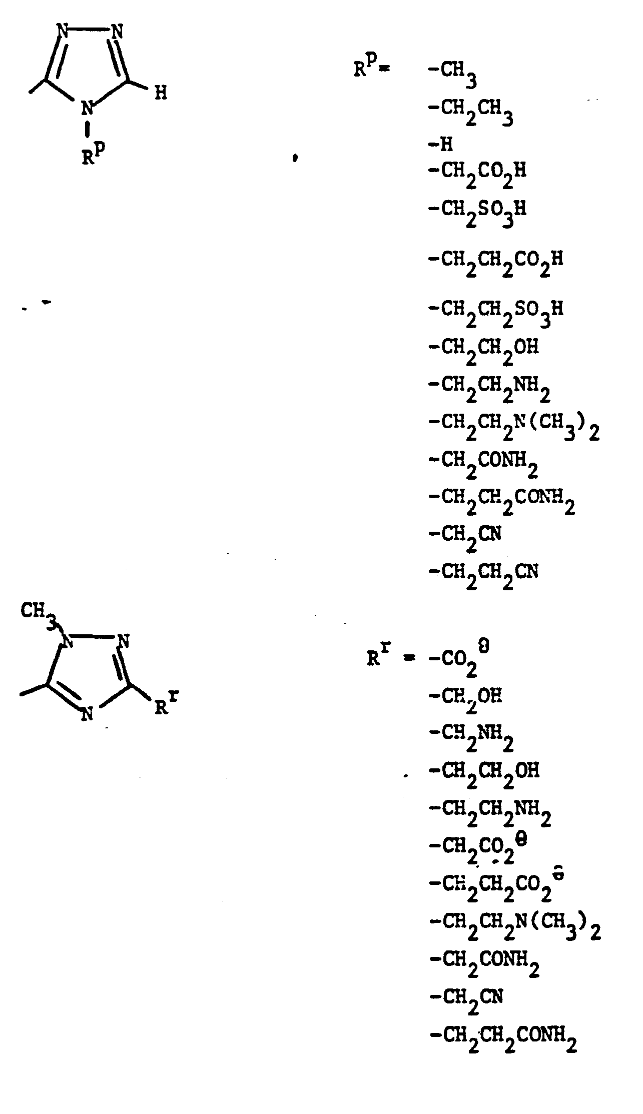 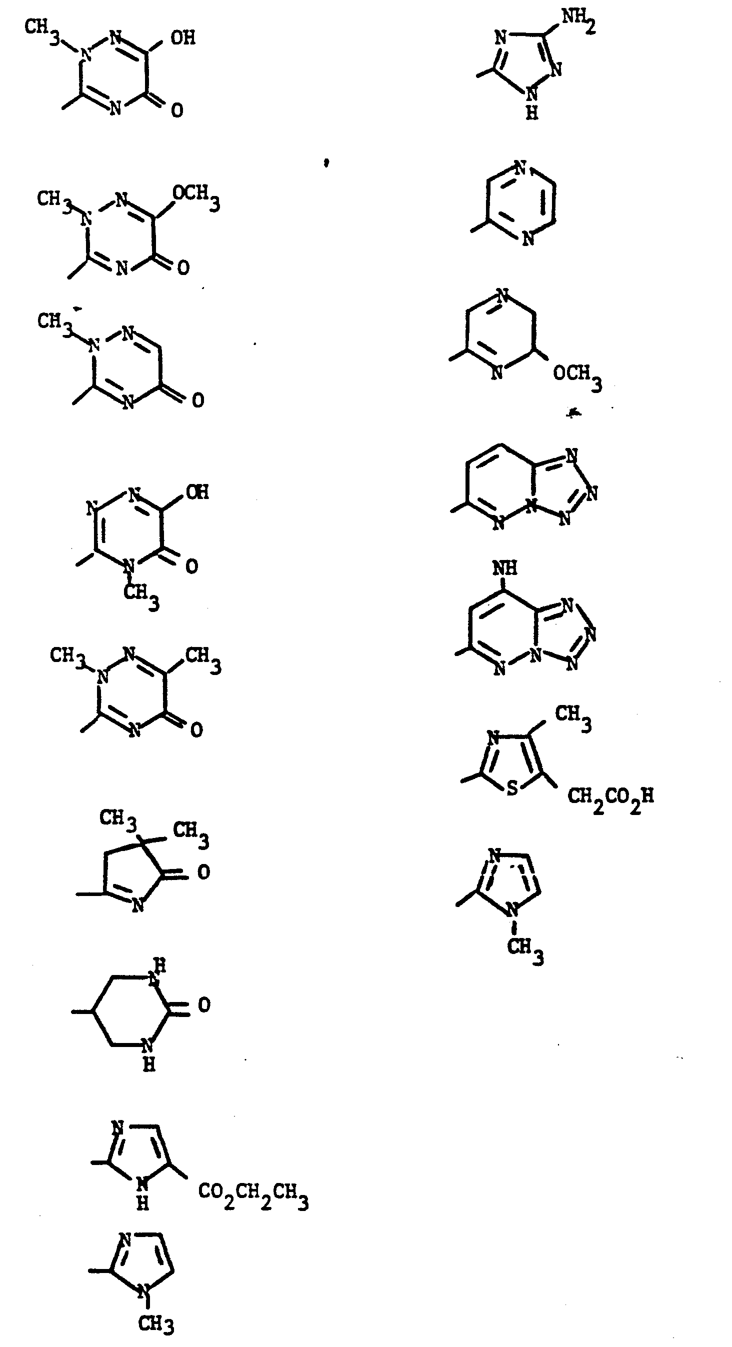 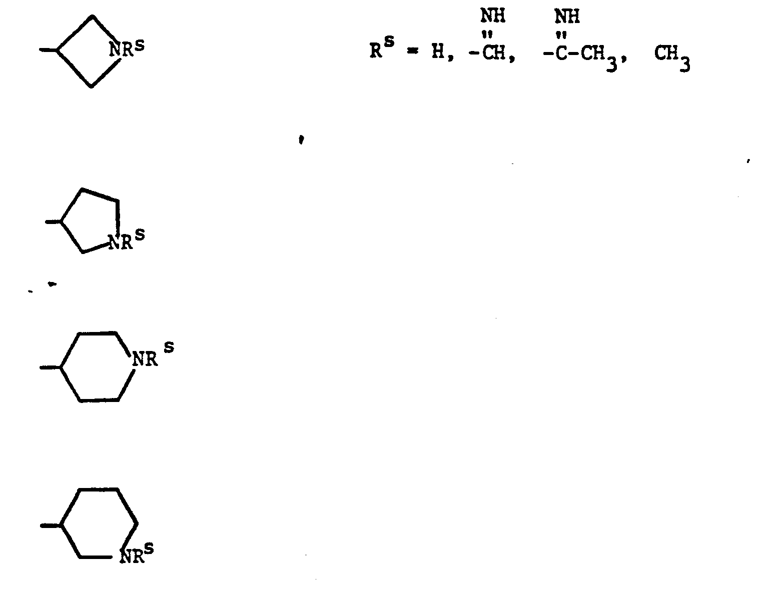
5. Arylaliphatic

Y- Aryl, where the preferred aryl groups are as illustrated above, and -Y- is as originally defined, and is preferably (CH₂)ₙ where n = 1-3.

### 6. Heterocycloaliphatic

Y - Heterocyclic, where the preferred heterocyclic groups are as illustrated above, and -Y- is as originally defined, and is preferably (CH₂)ₙ where n - 1-3; preferred heterocycloaliphatic groups are:

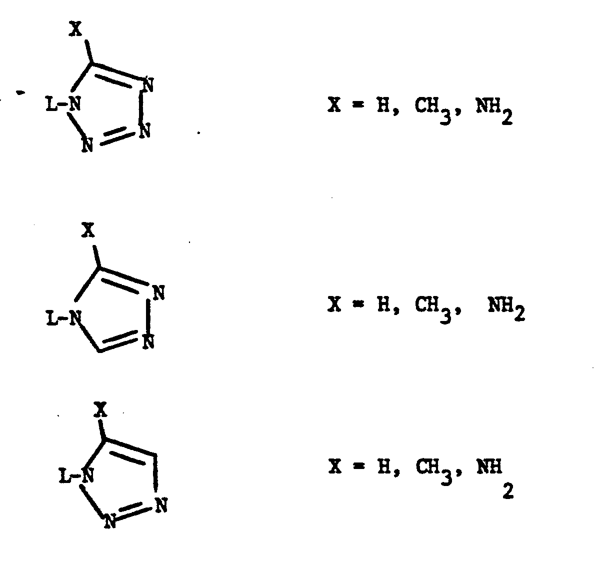

### 7. Alkyl-Heteroatom-Alkyl

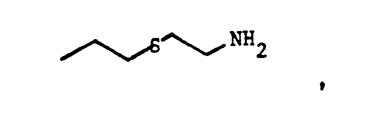

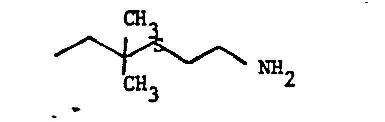

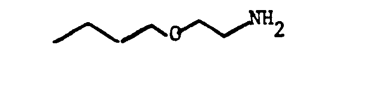

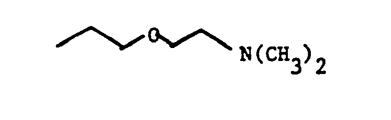

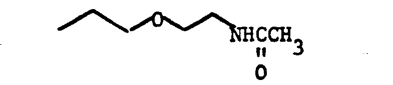

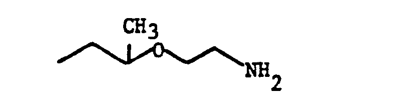

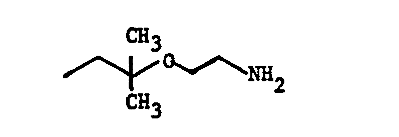

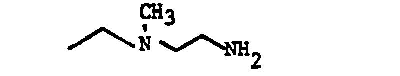

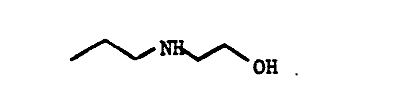

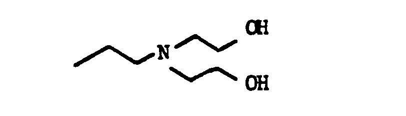

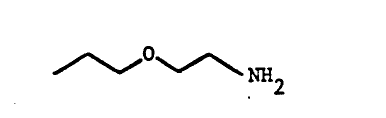

### 8. Heteroarylium

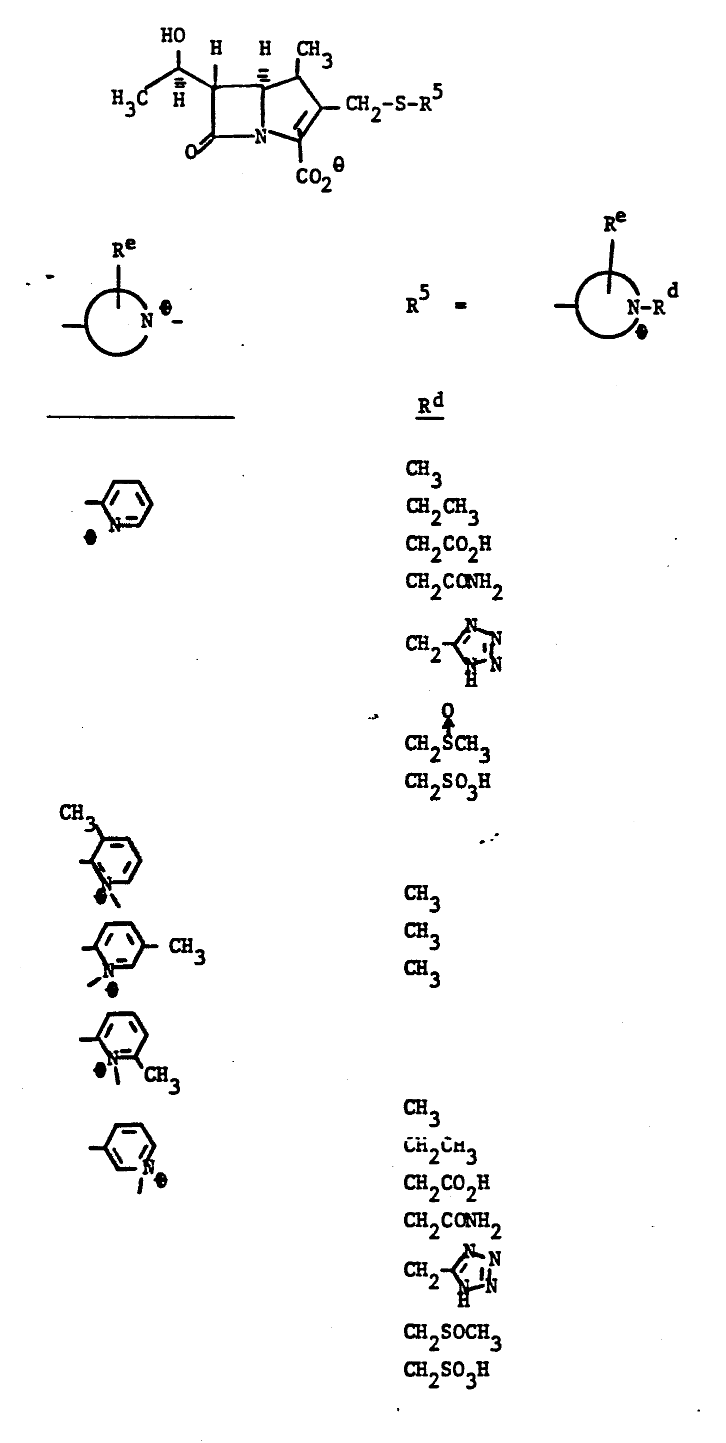

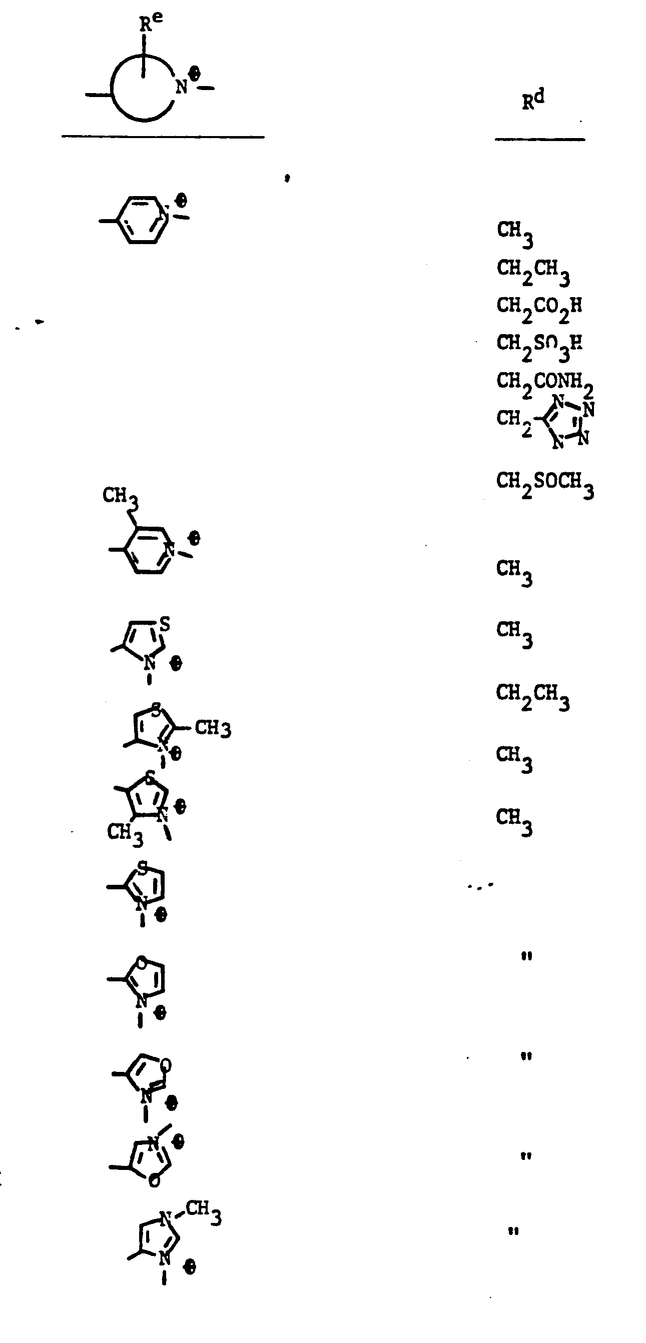

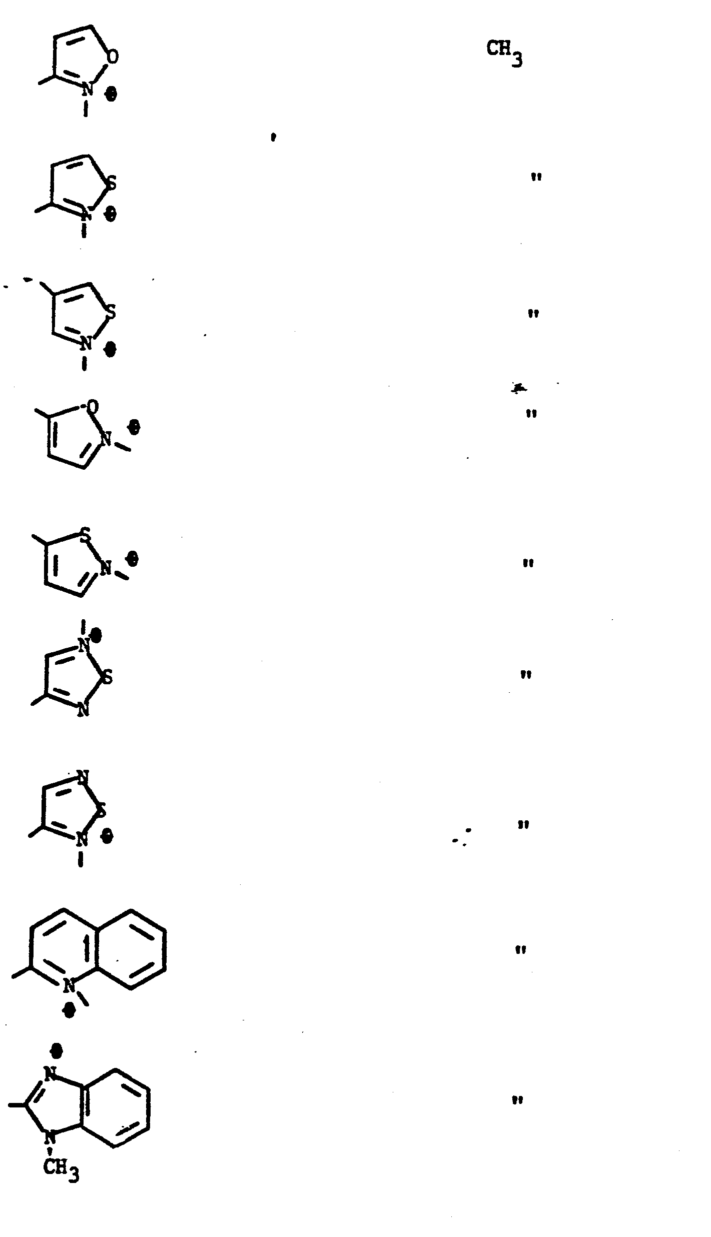

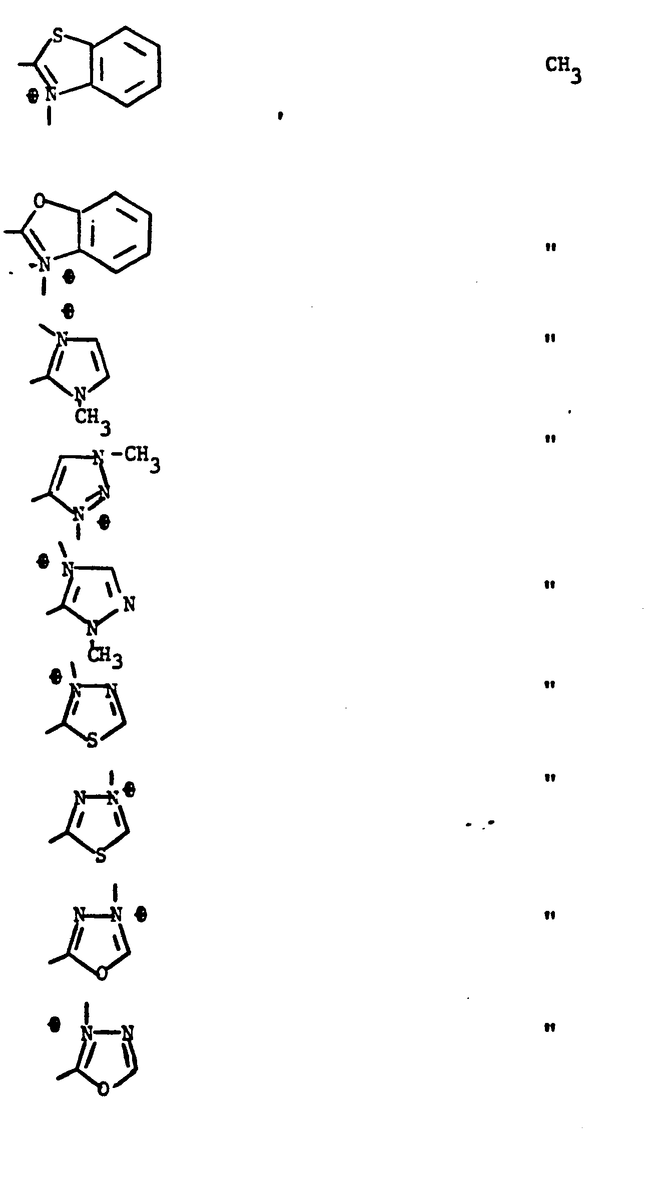

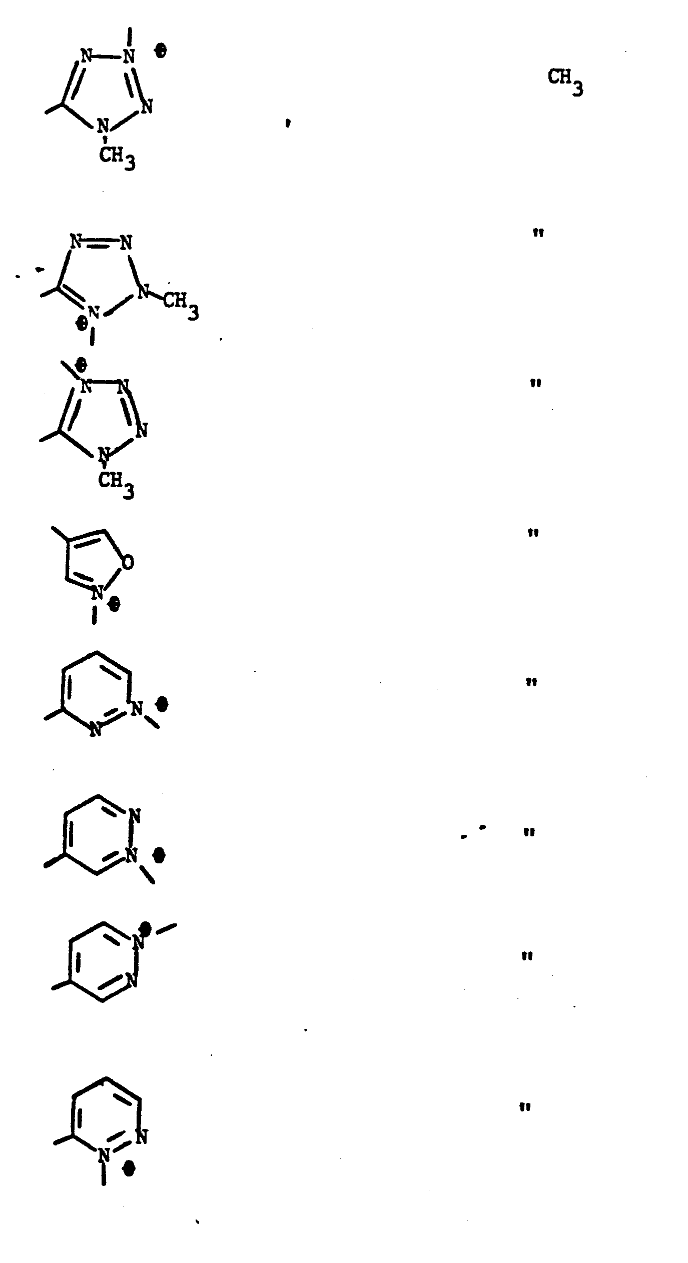

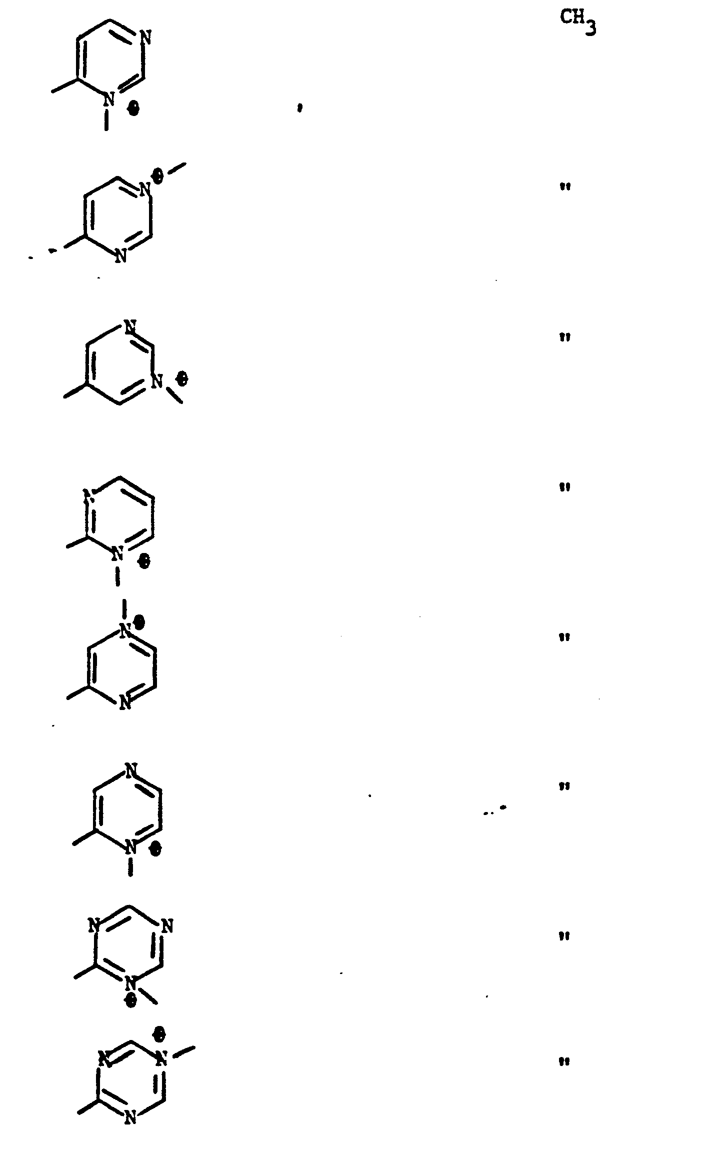

### 9. Heteroar^{y}liumaliphatic

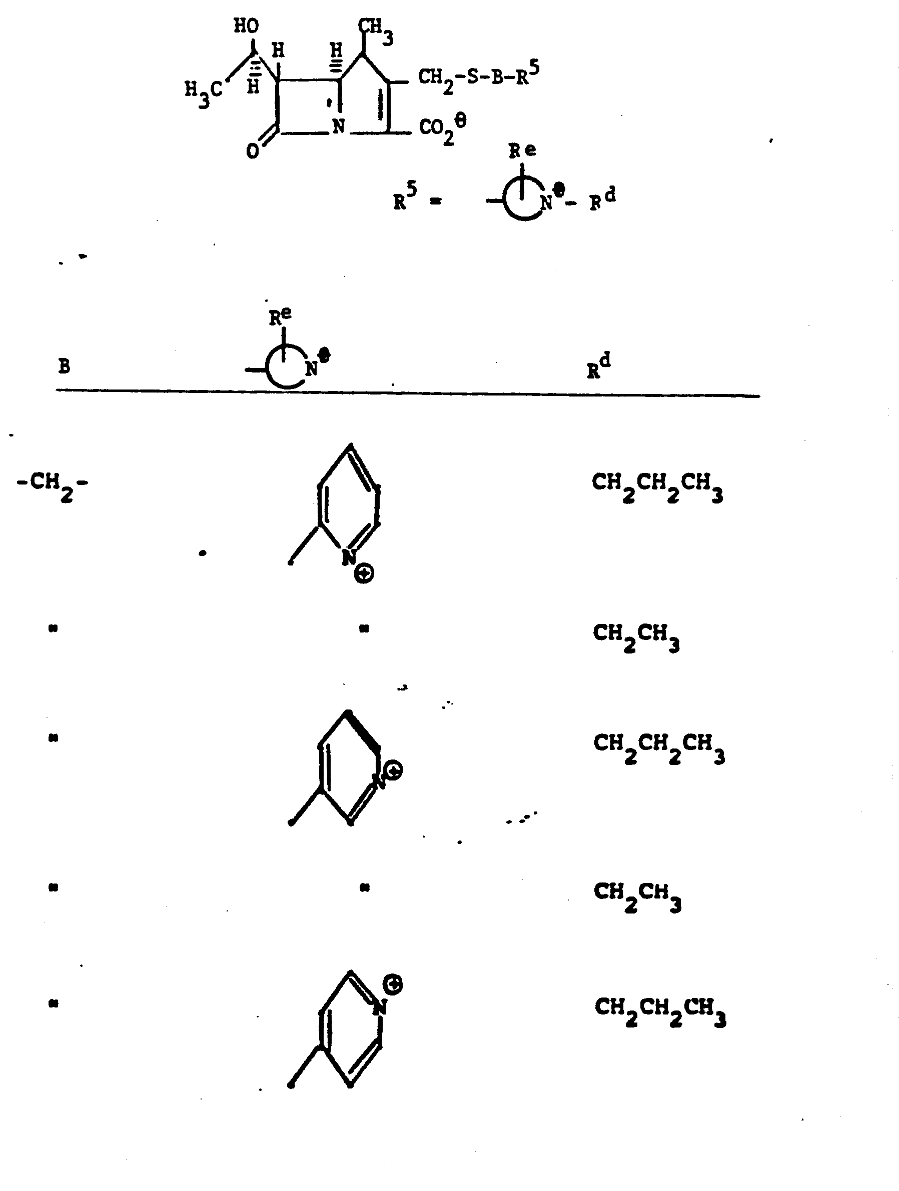

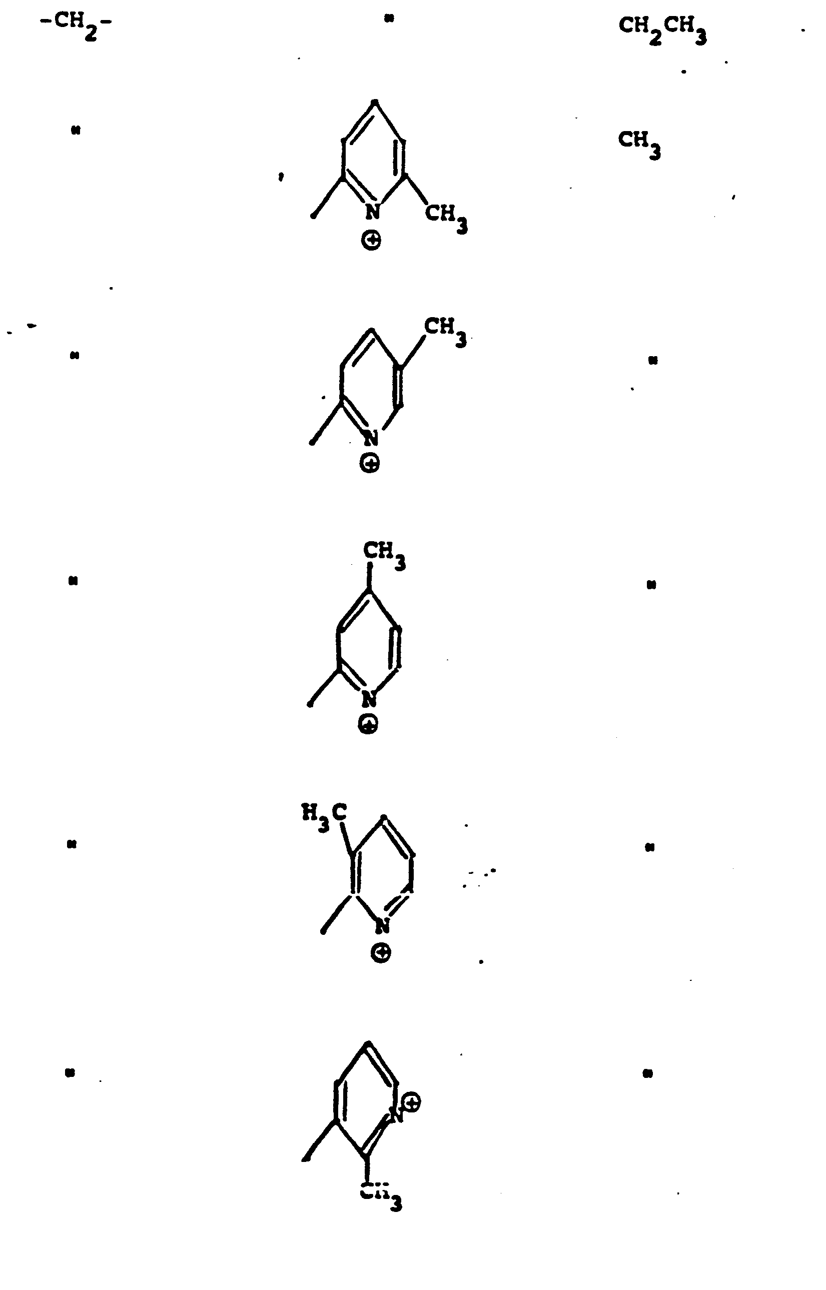

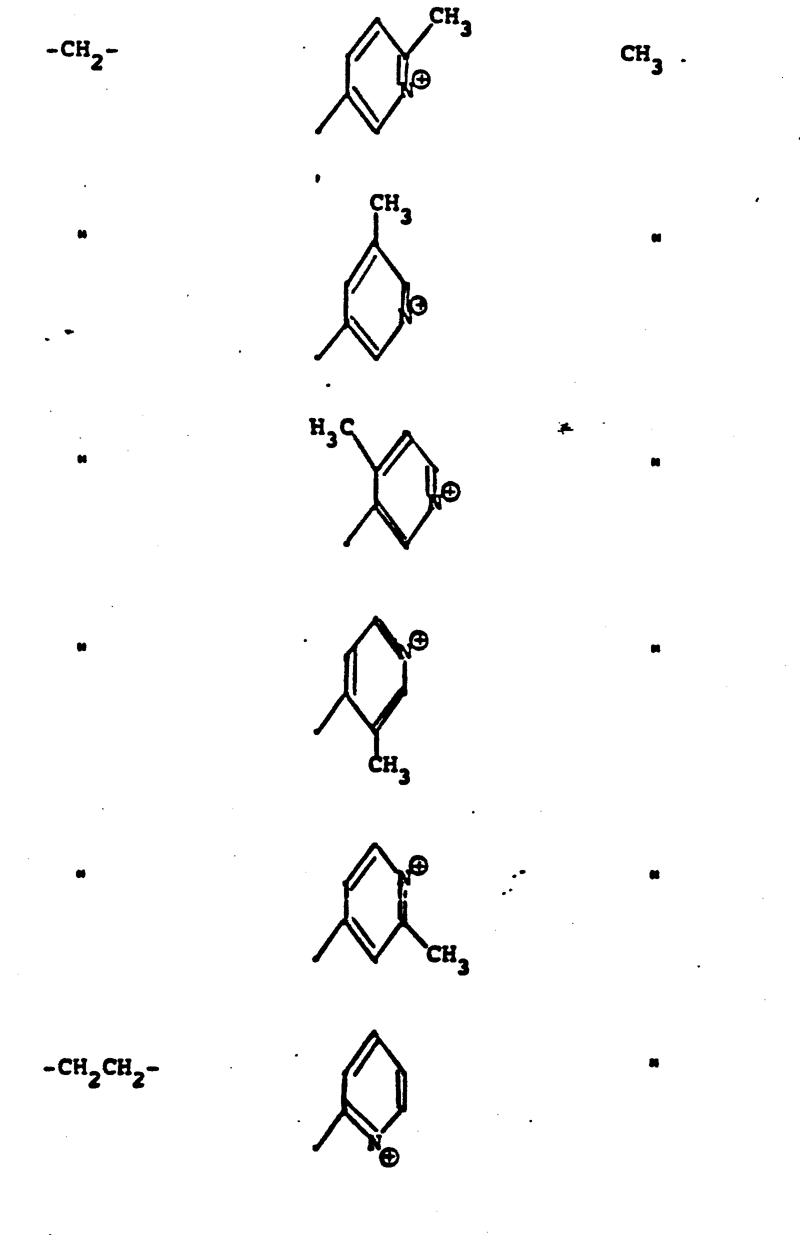

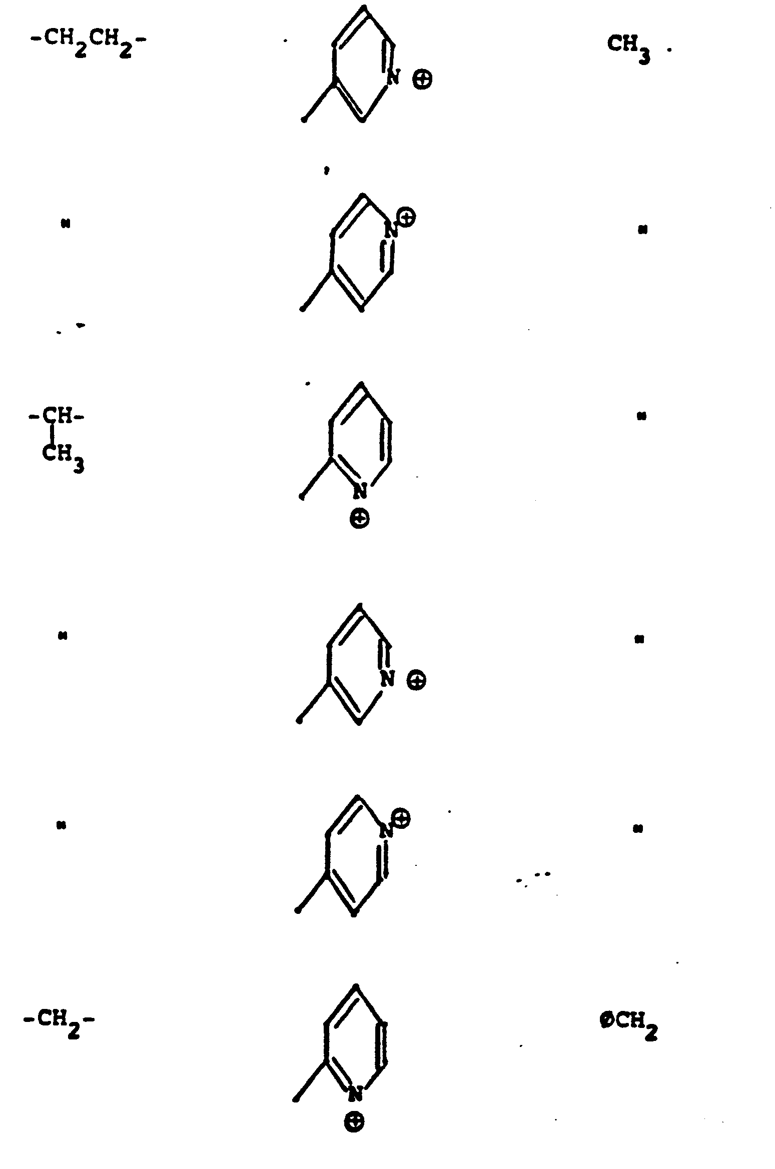

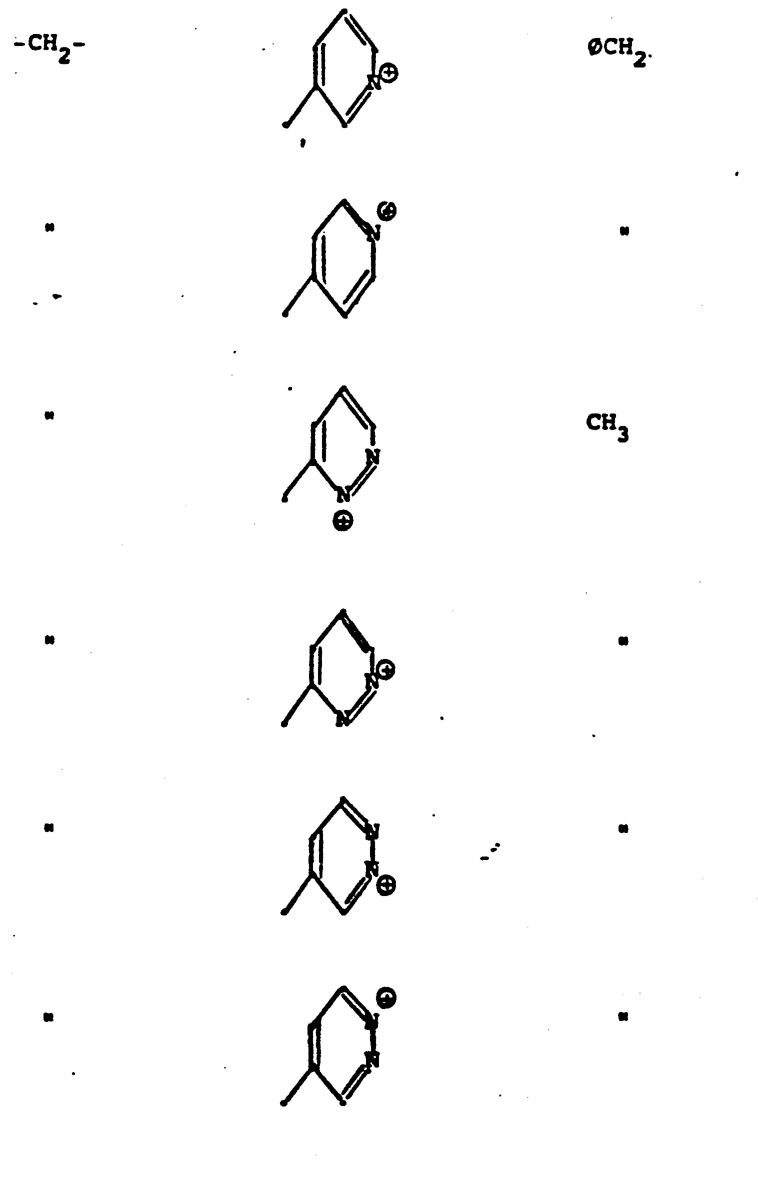

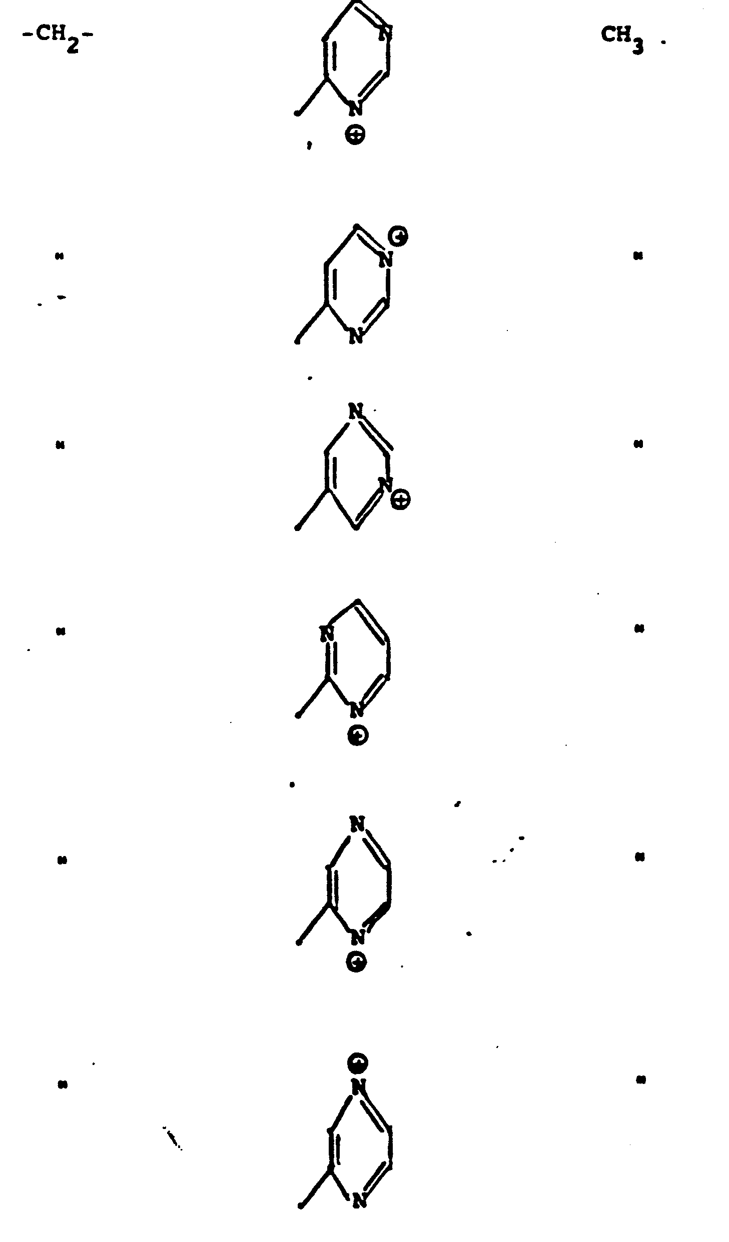

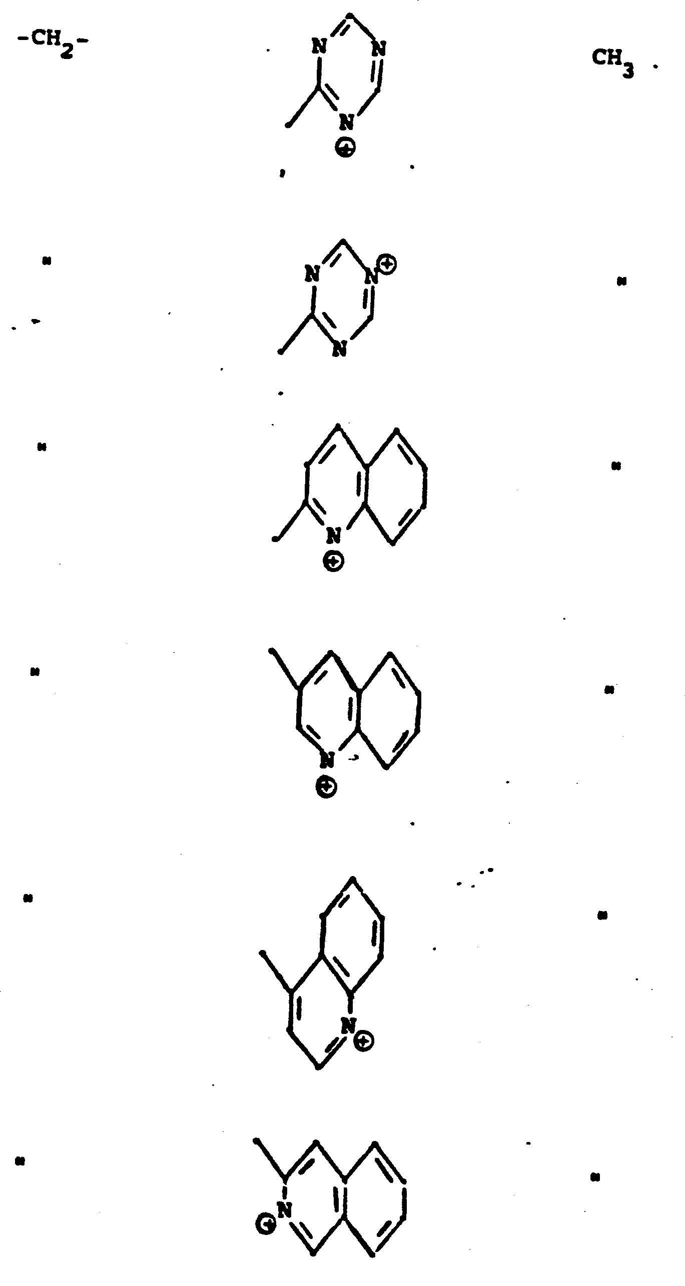

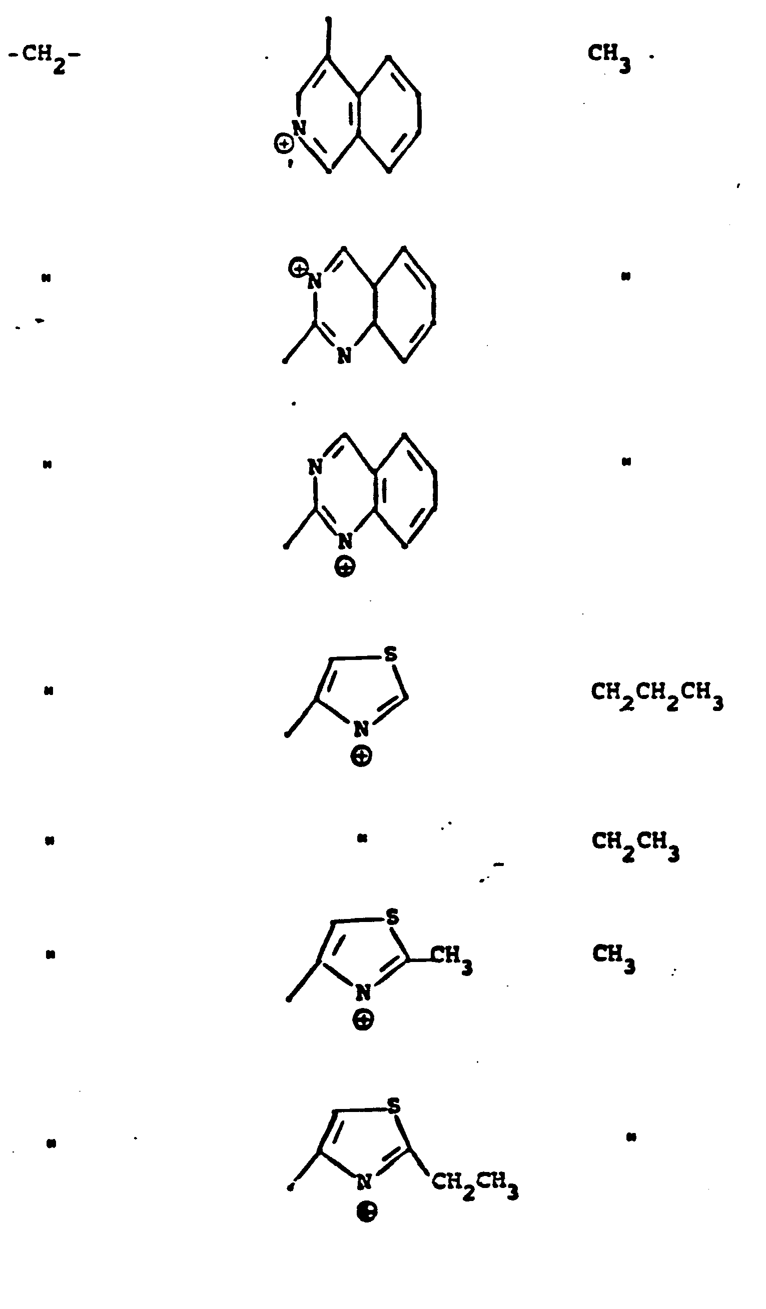

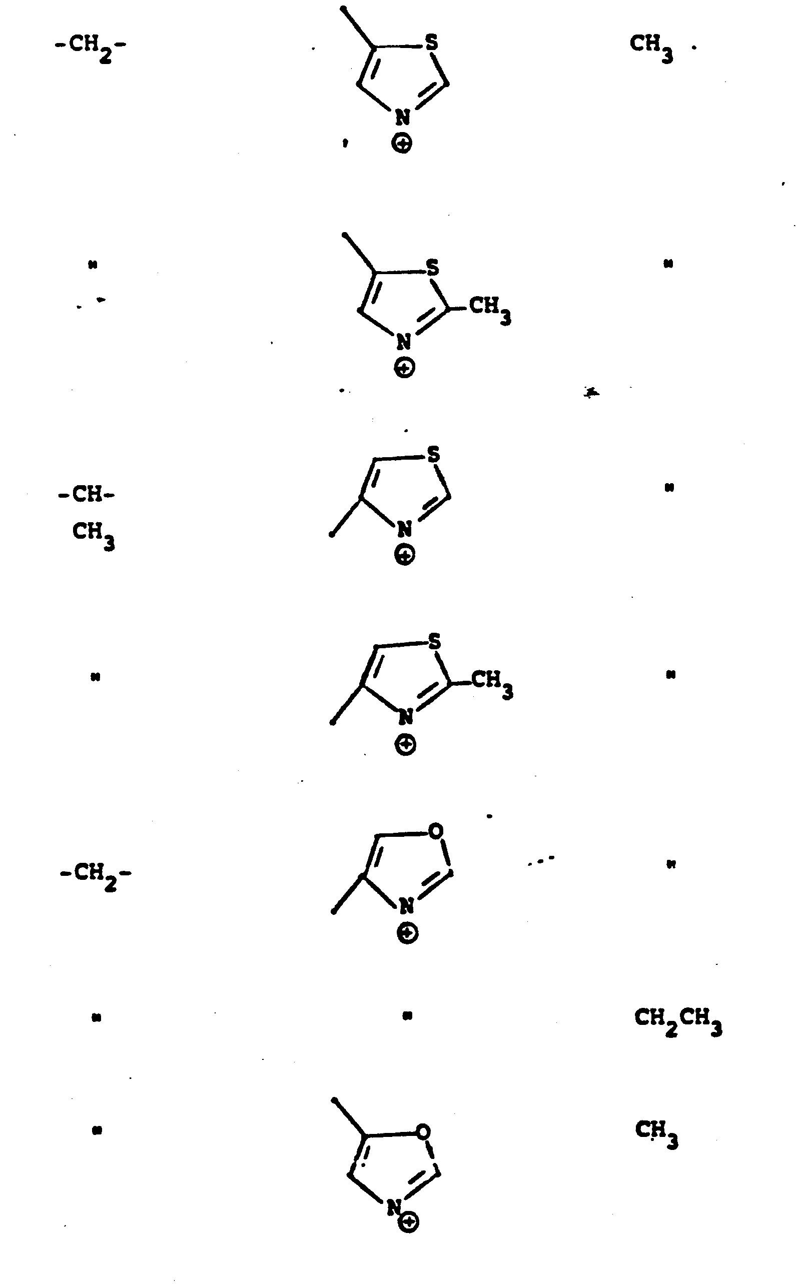

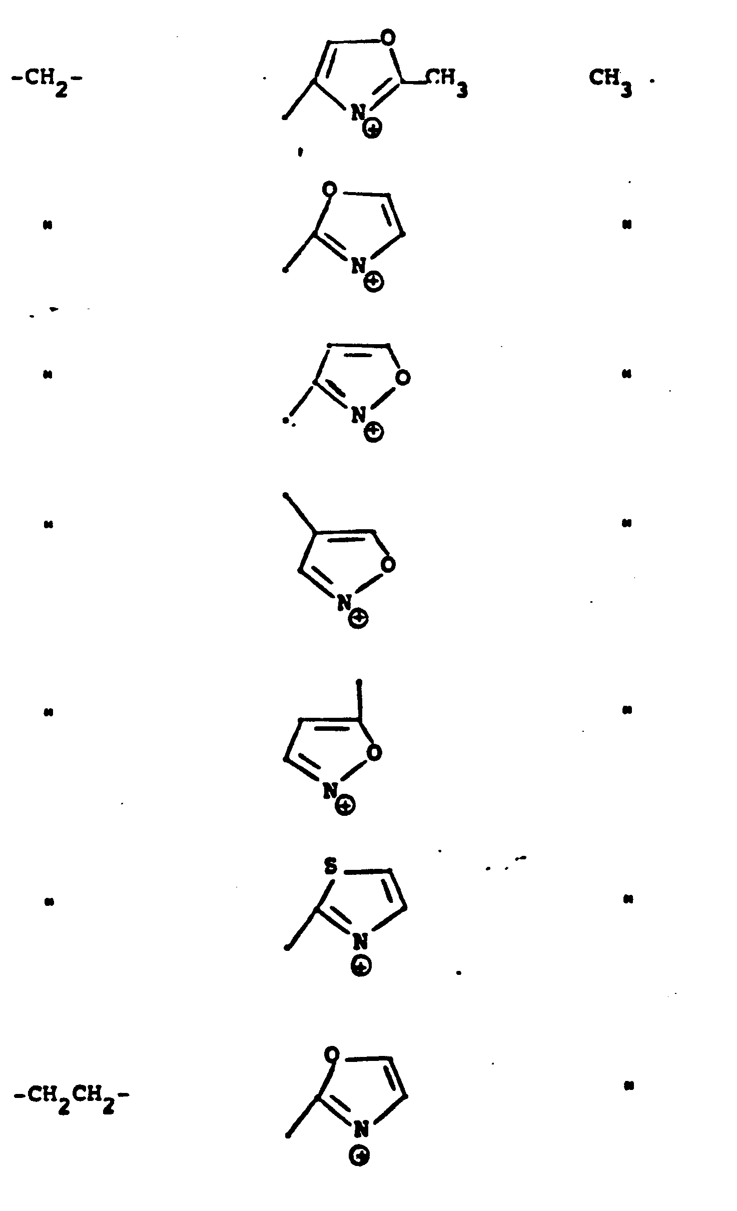

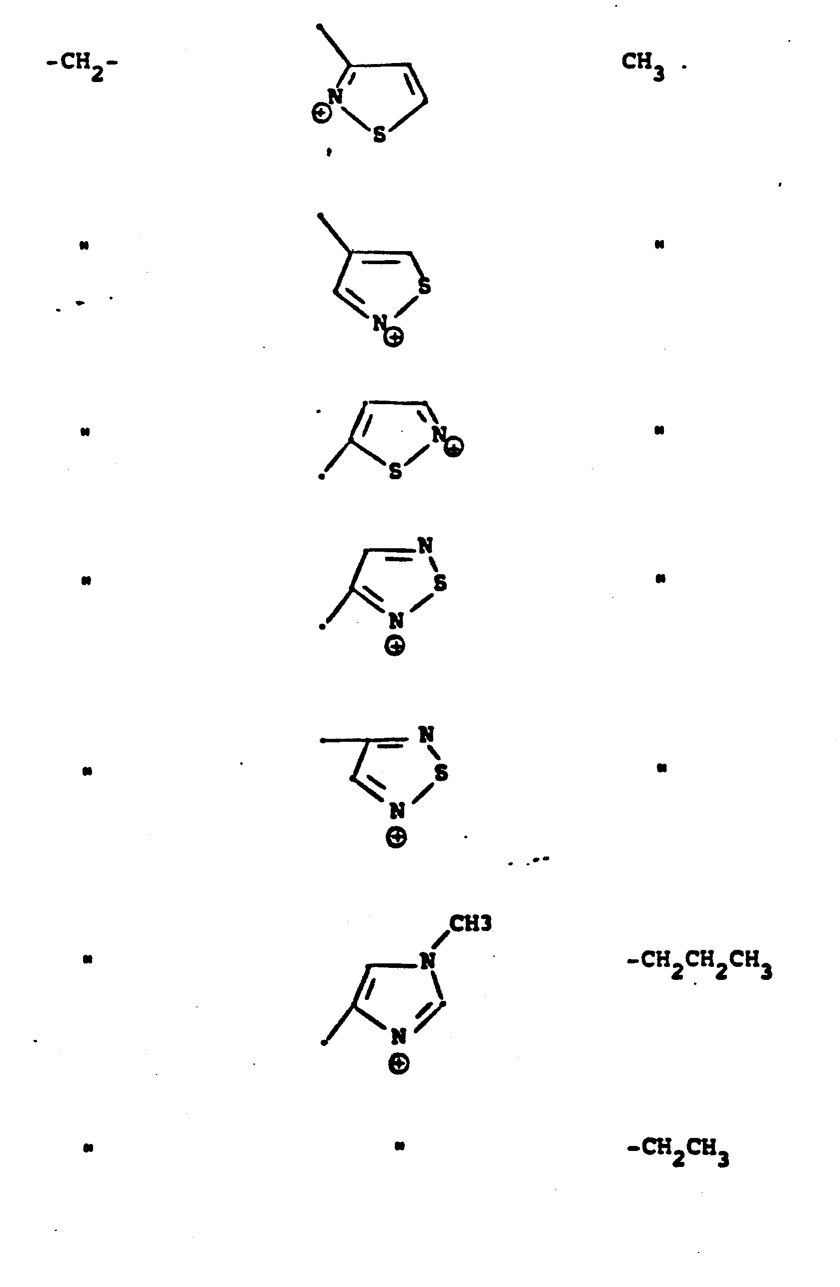

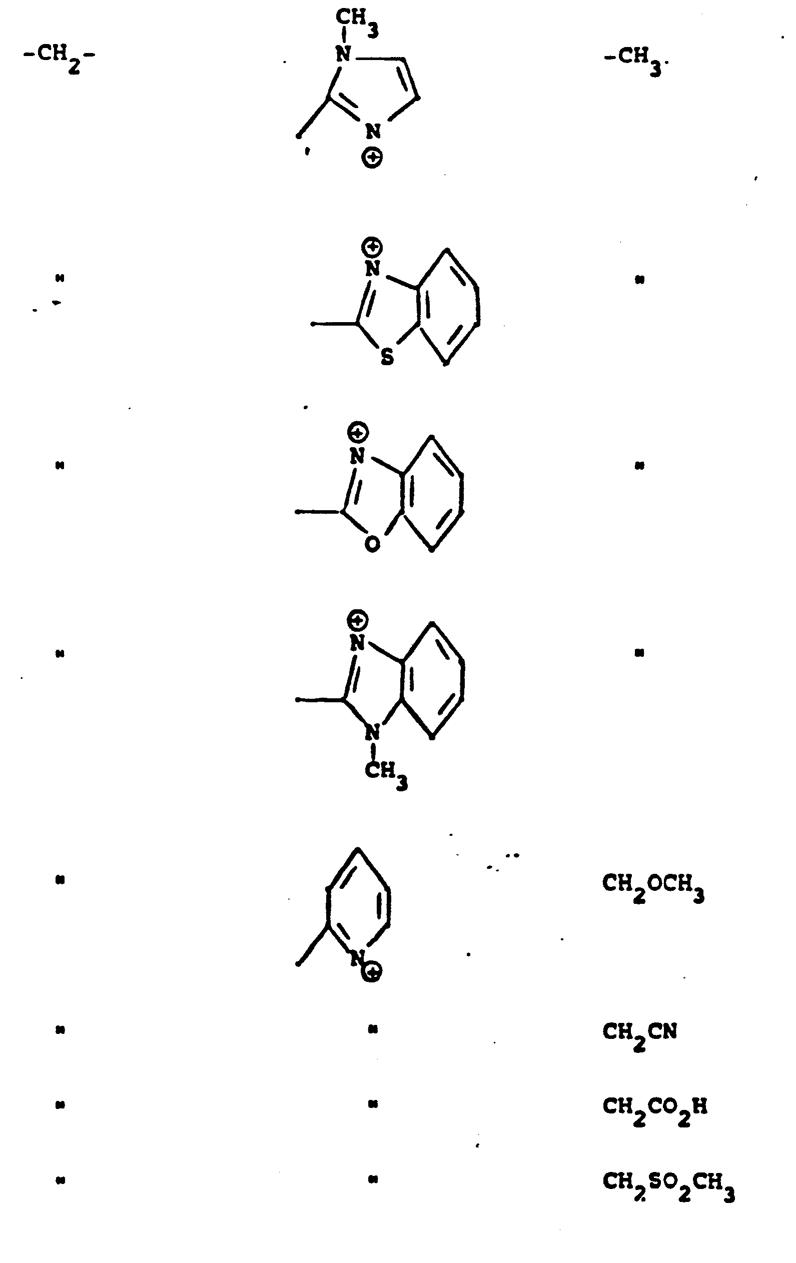

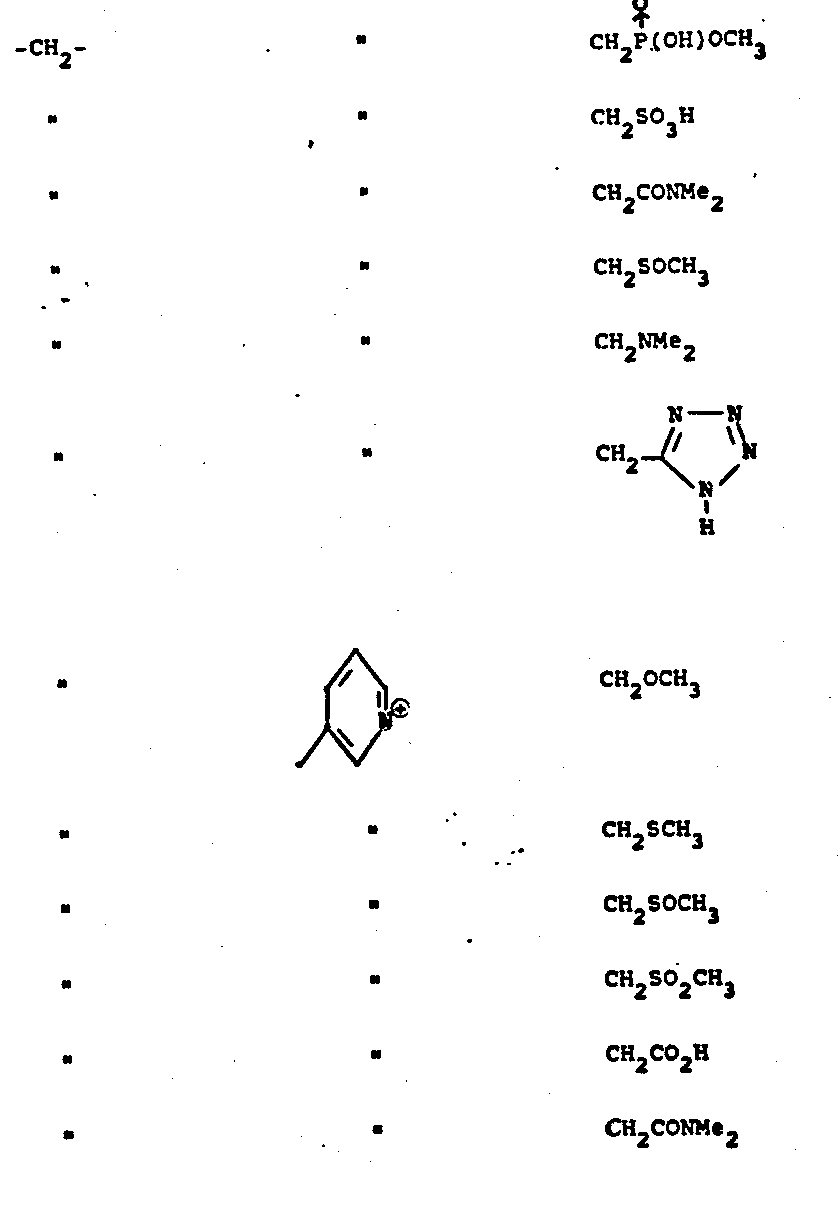

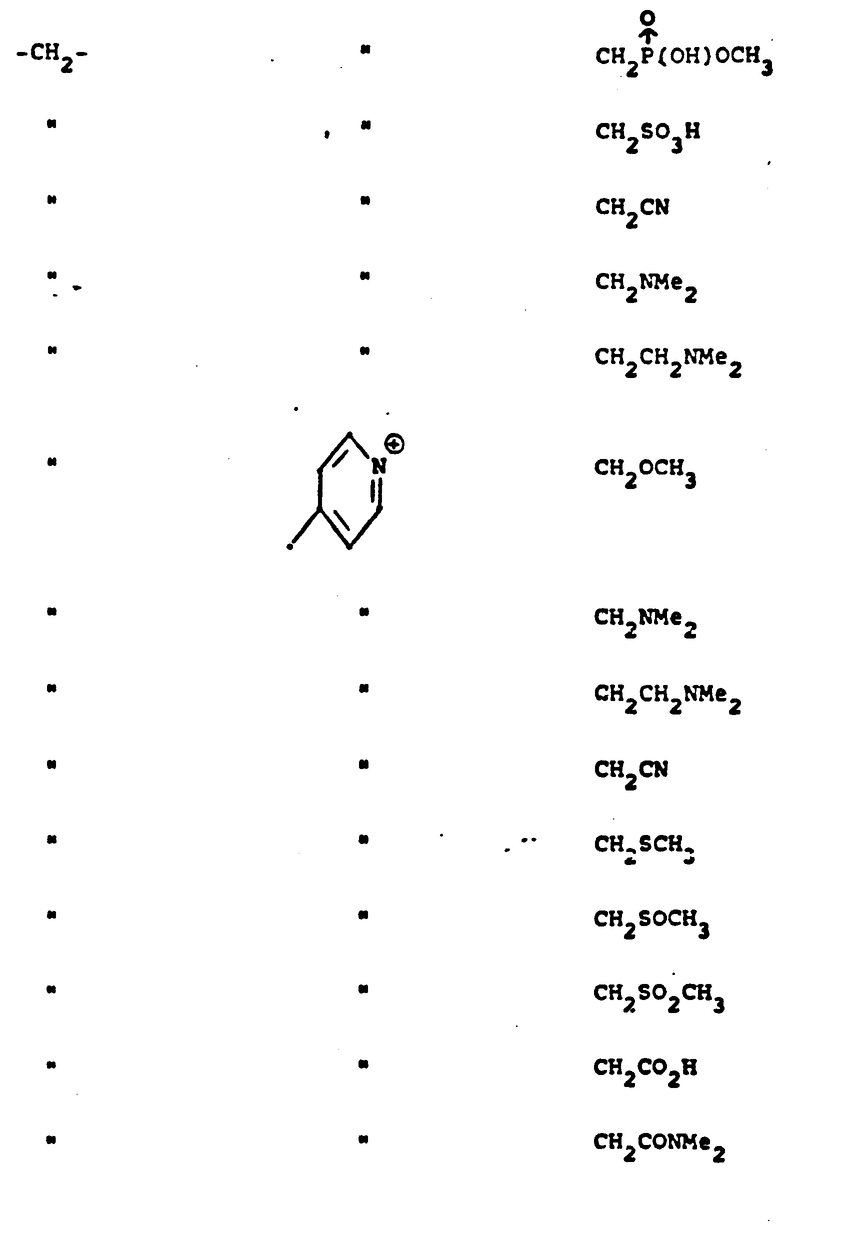

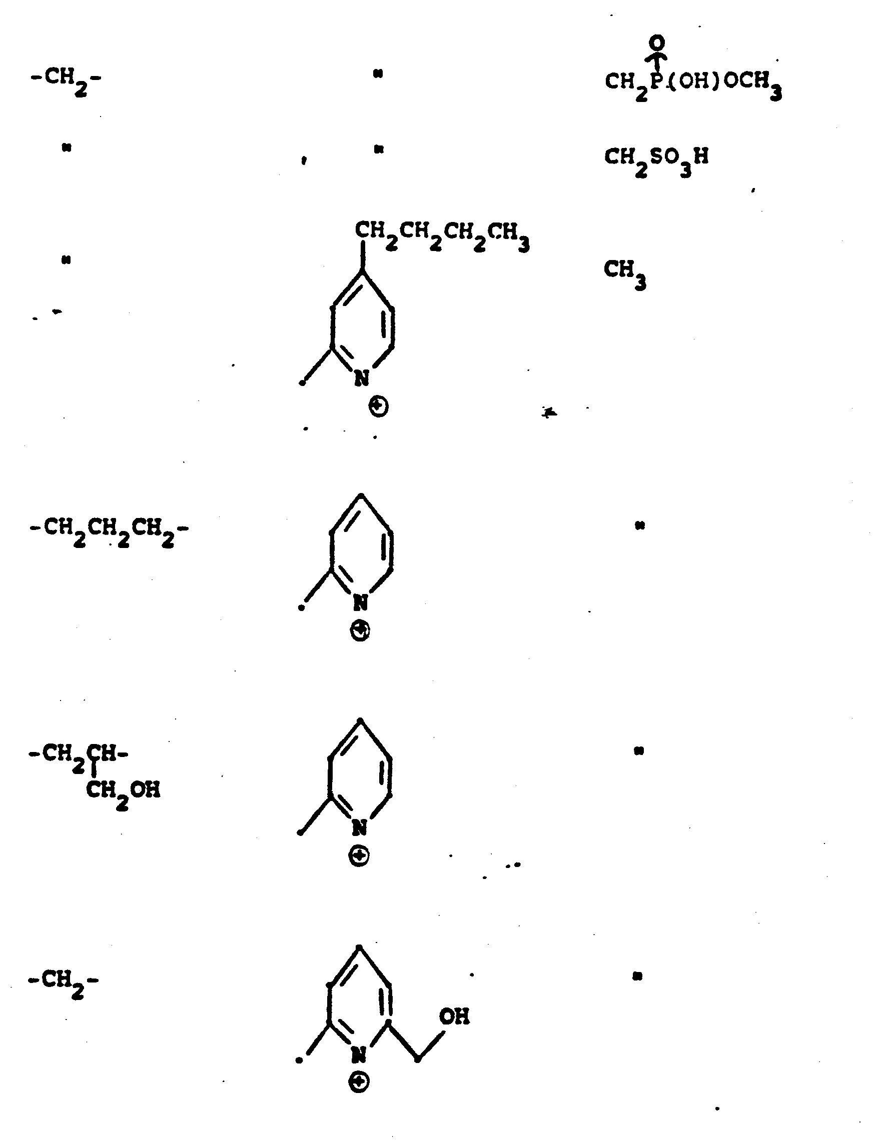

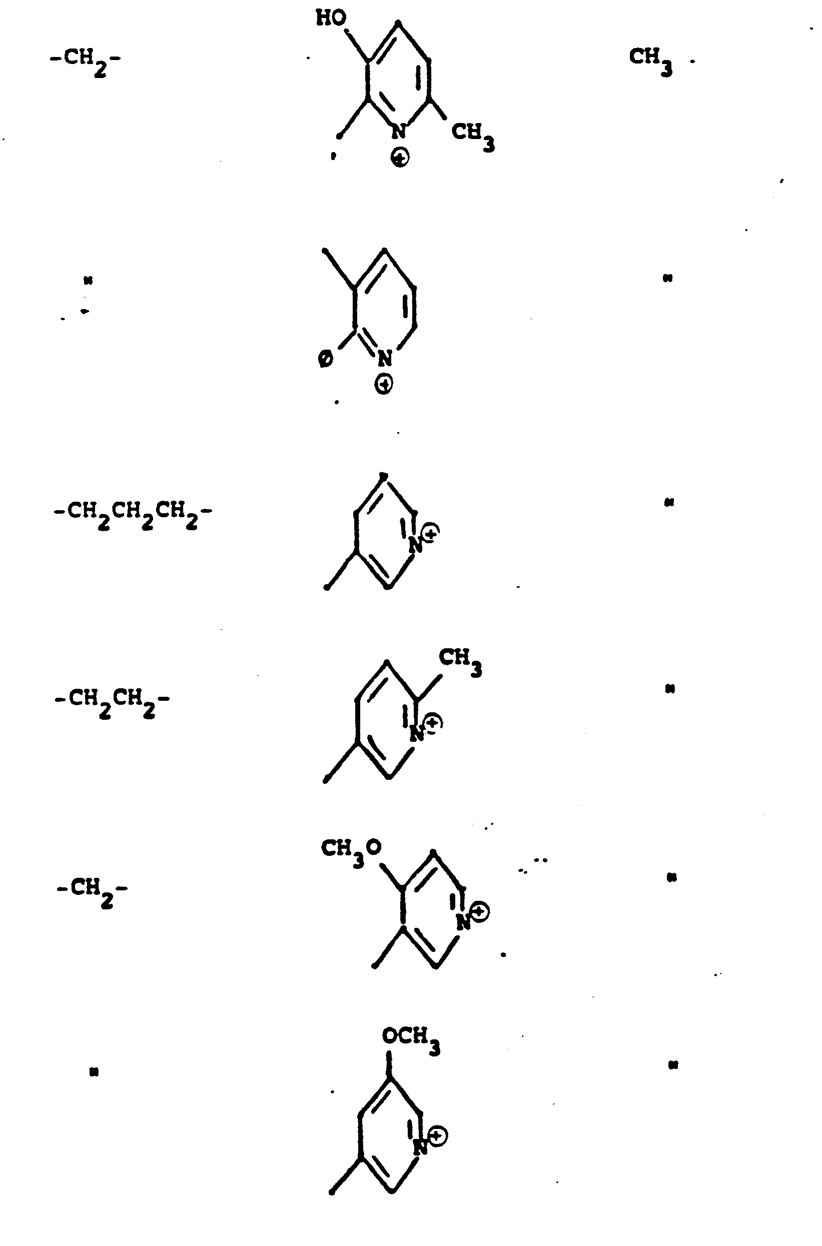

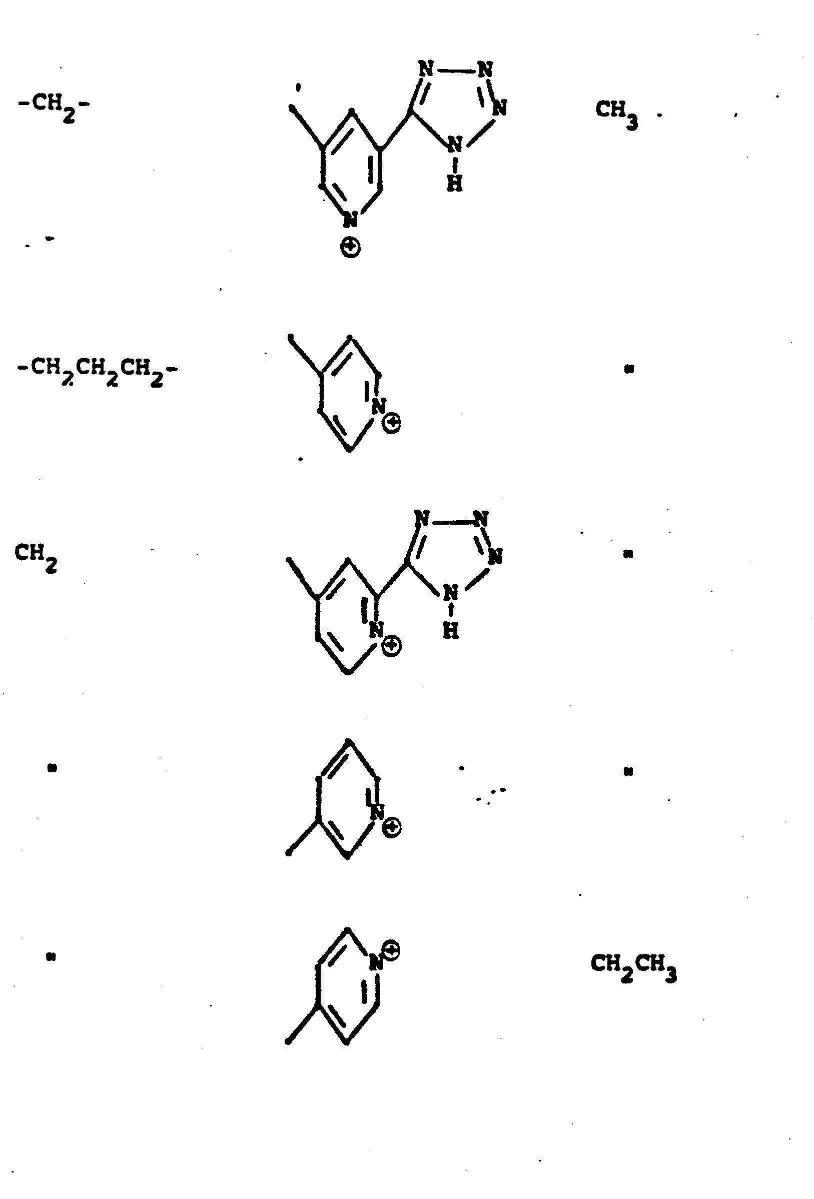

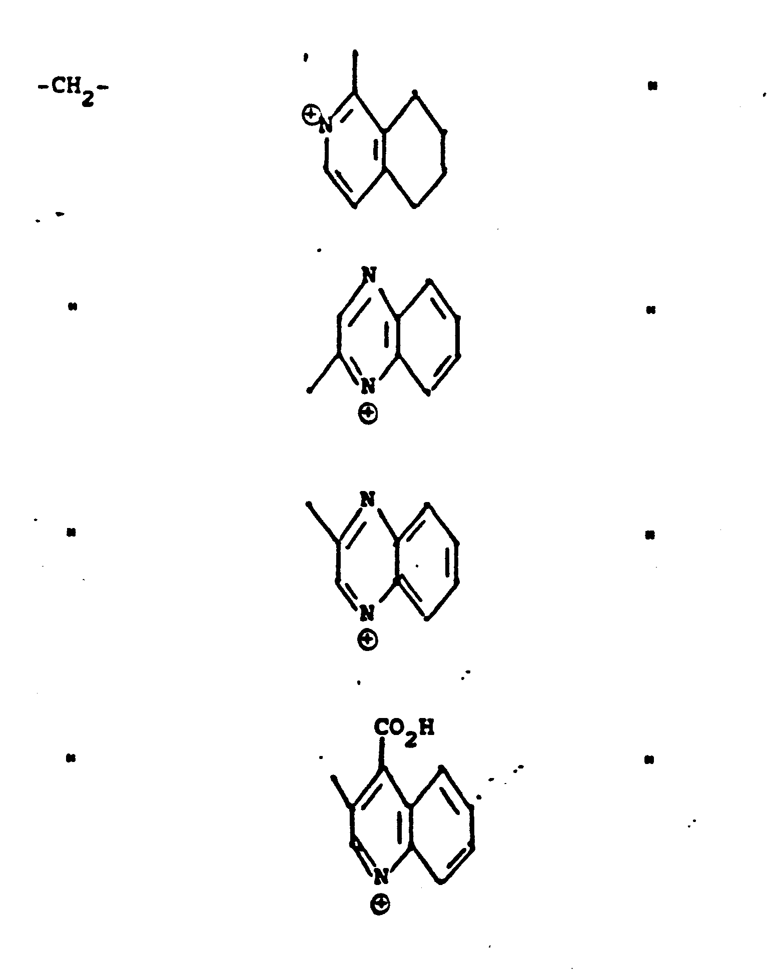

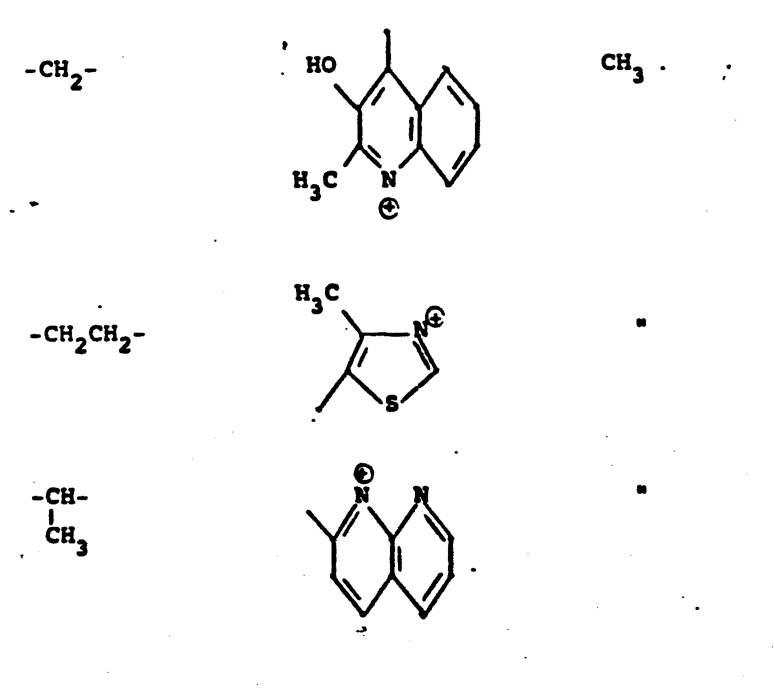

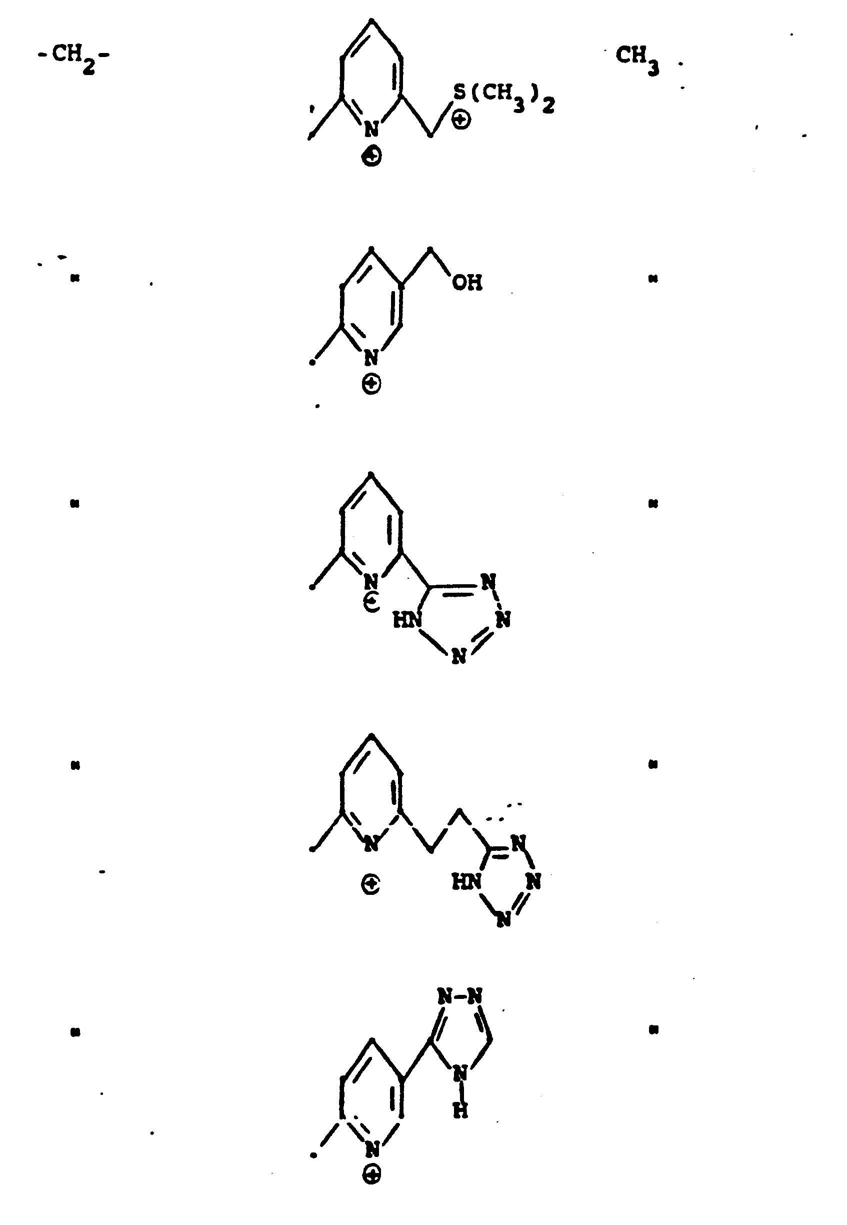

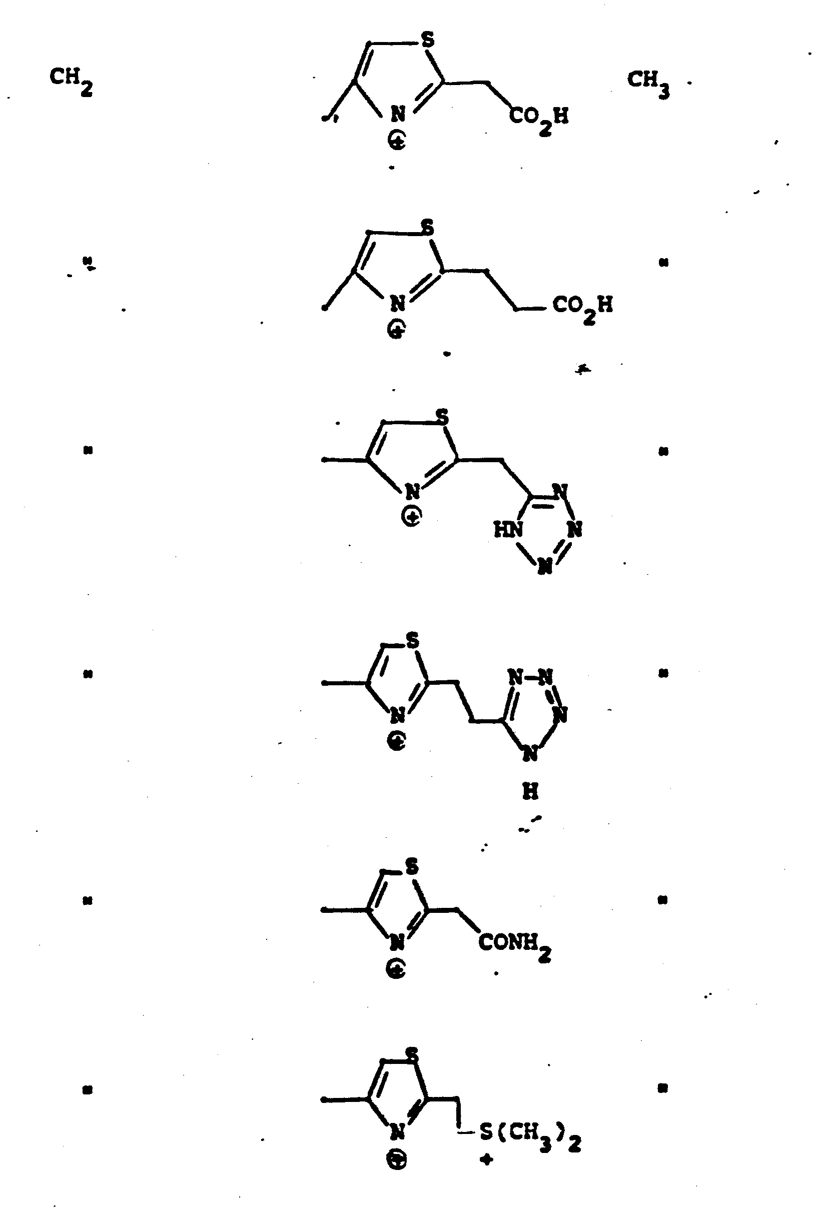

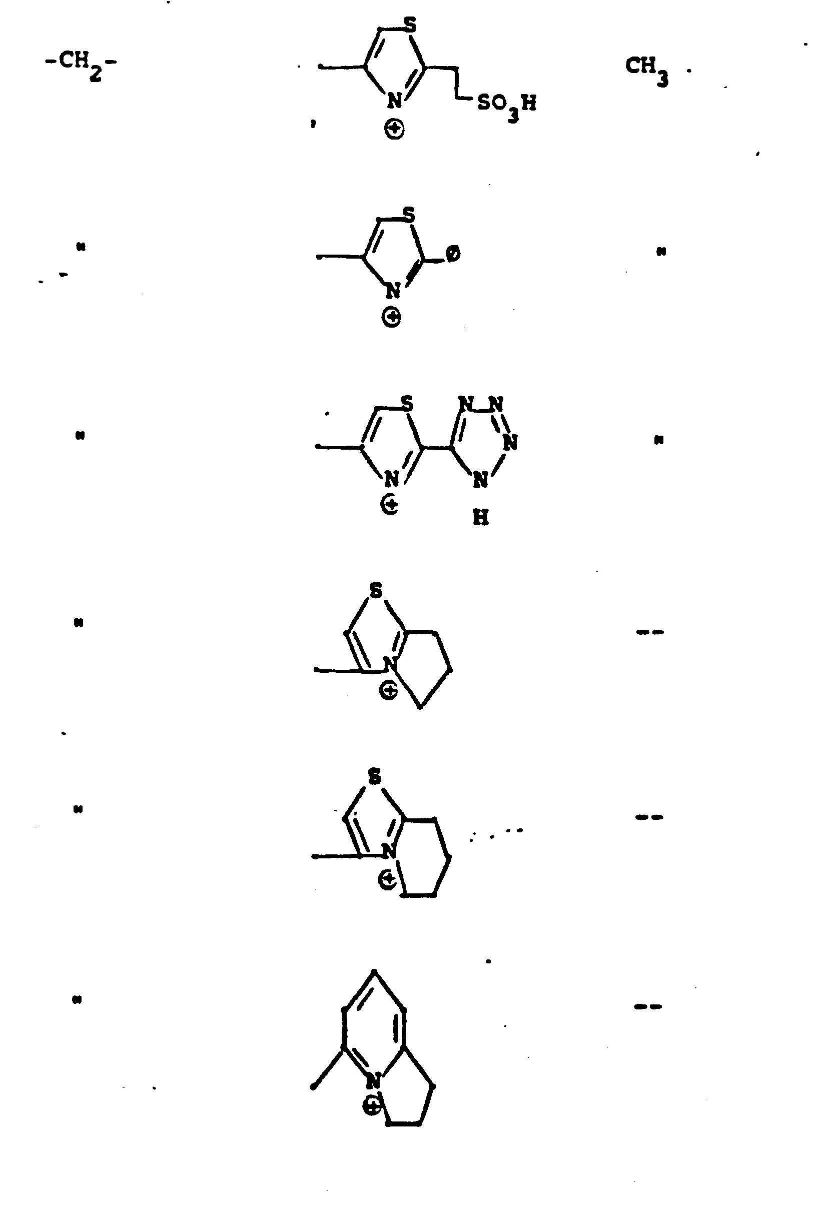

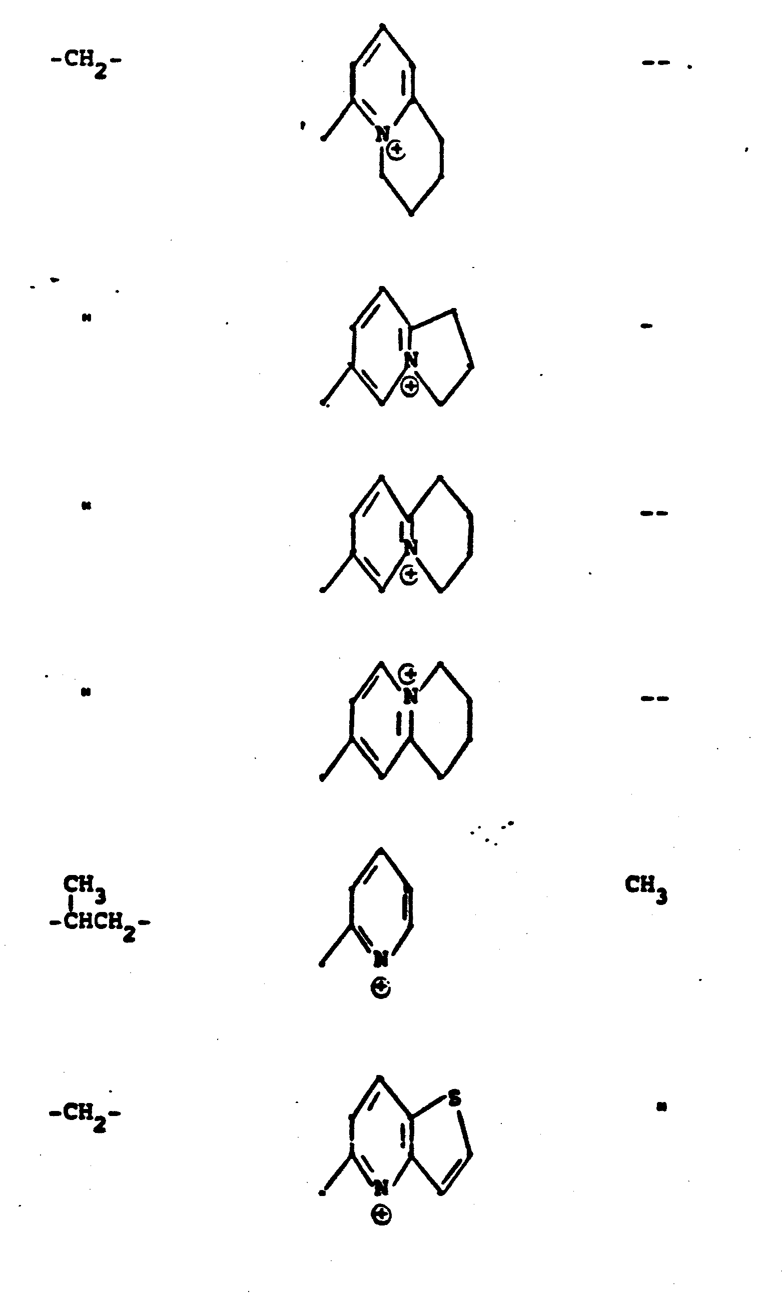

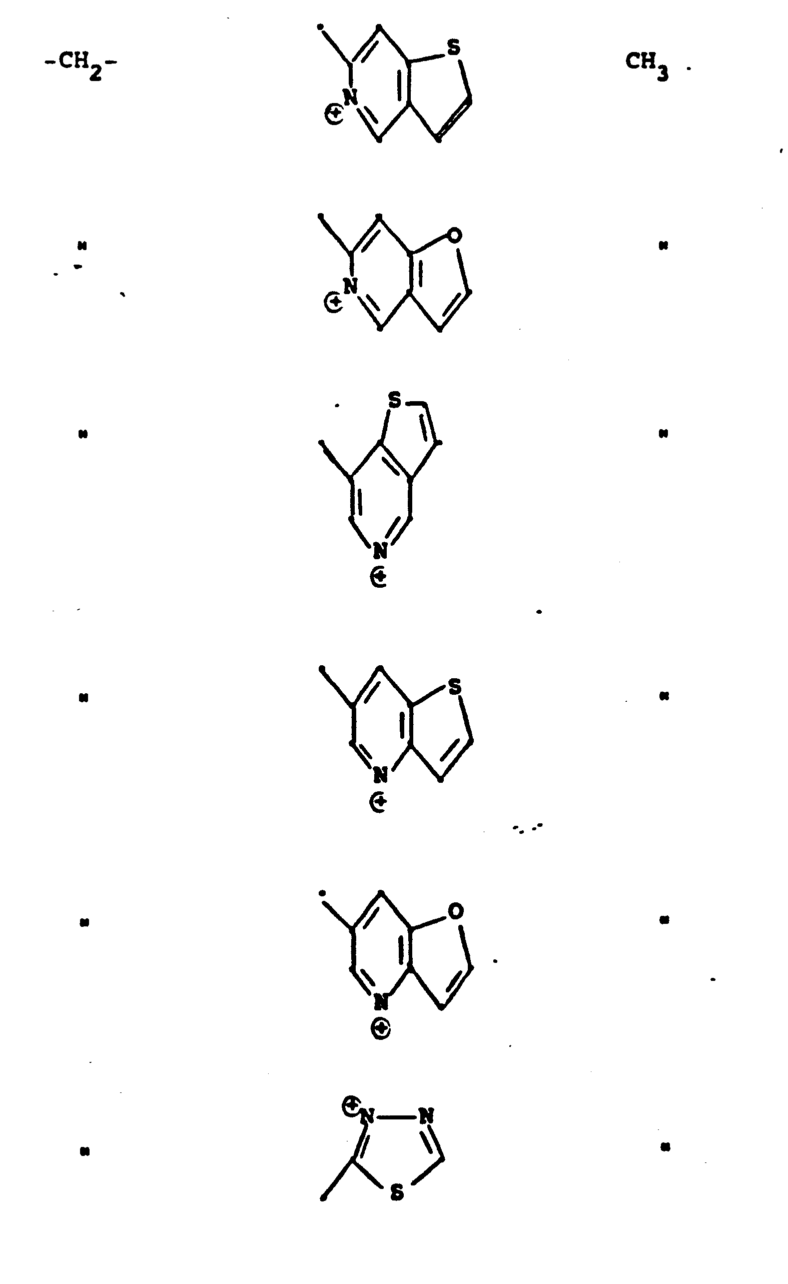

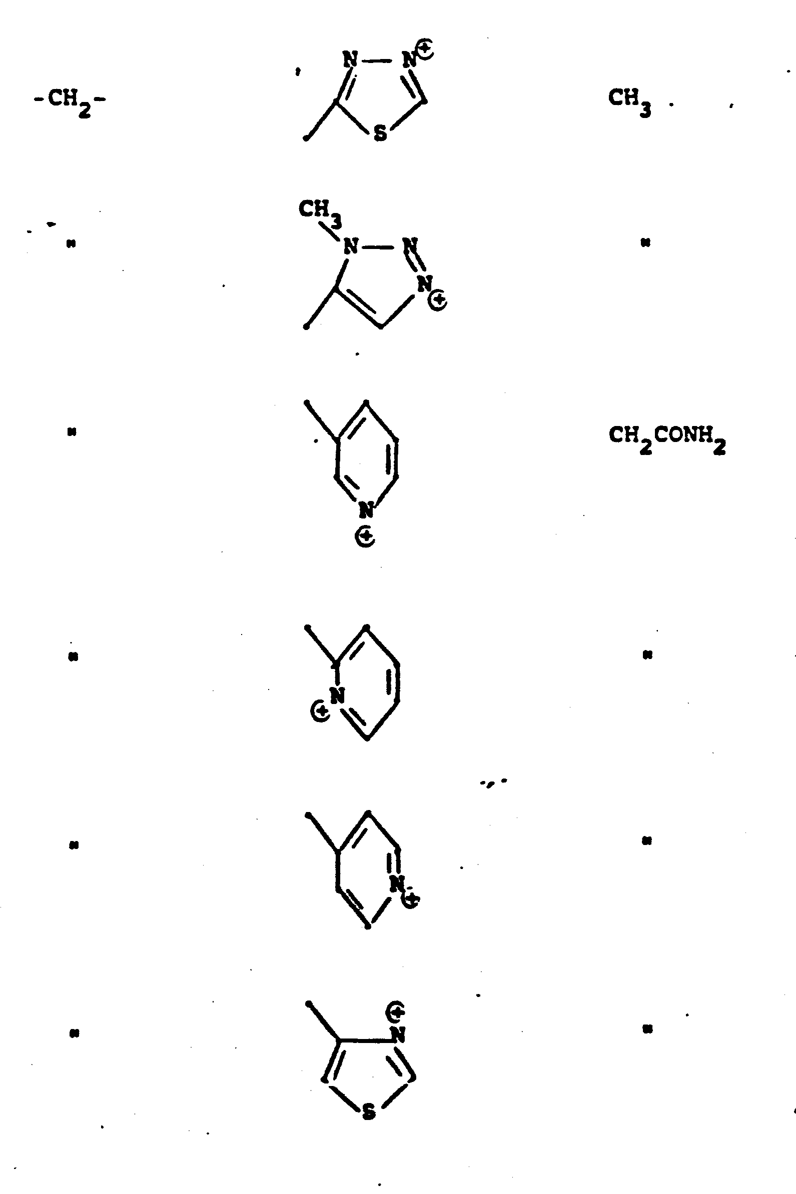

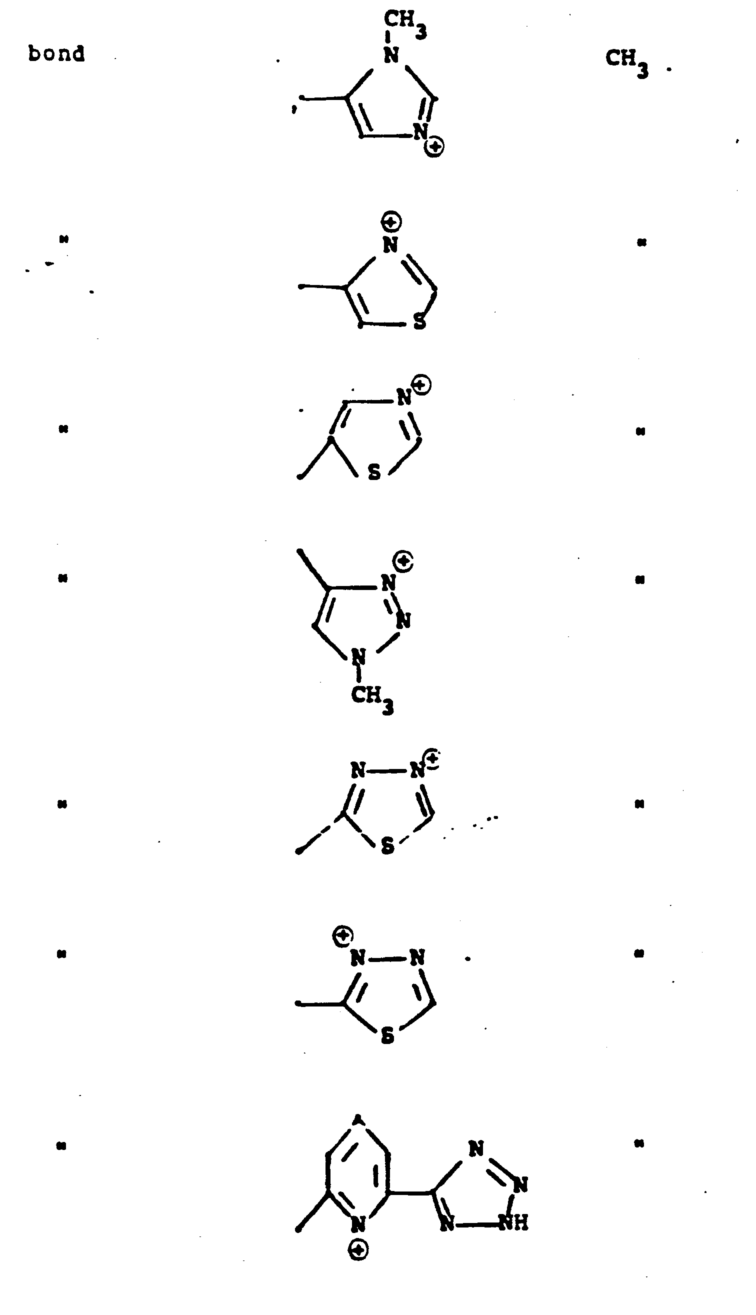

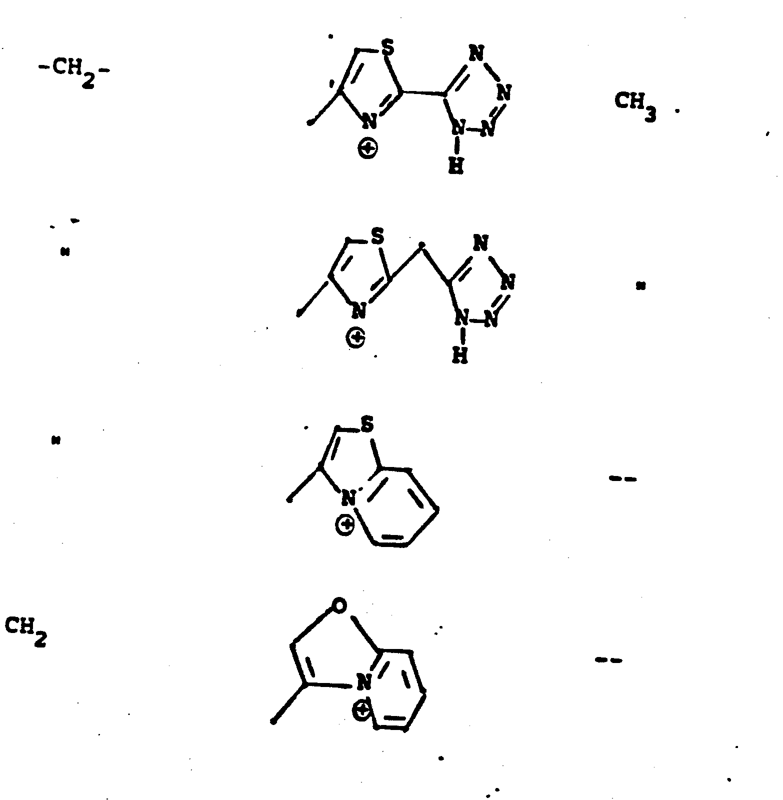

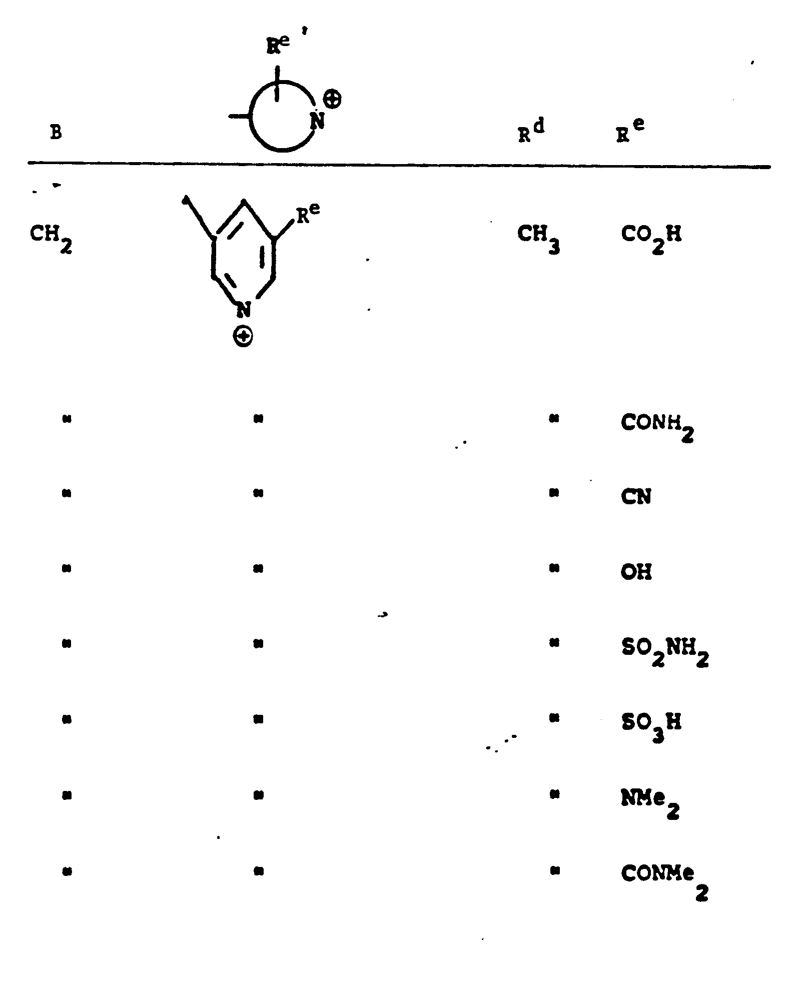

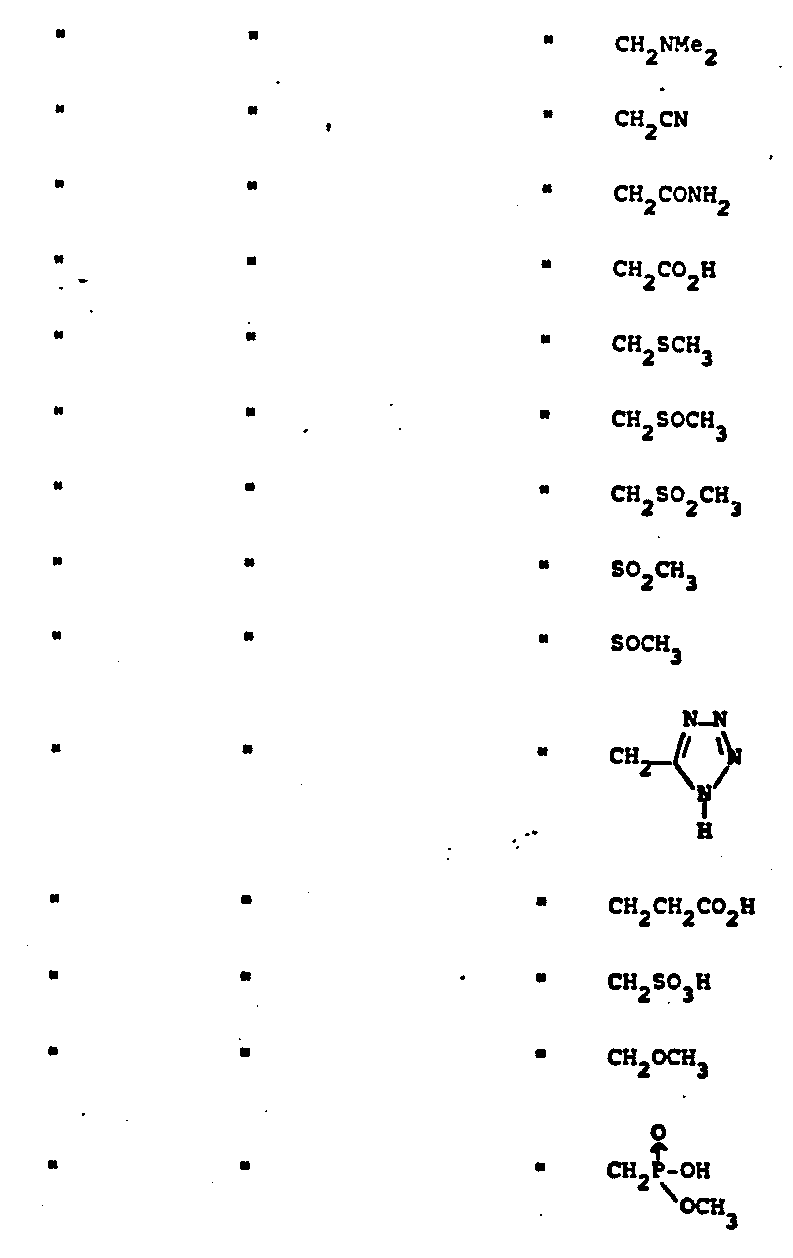

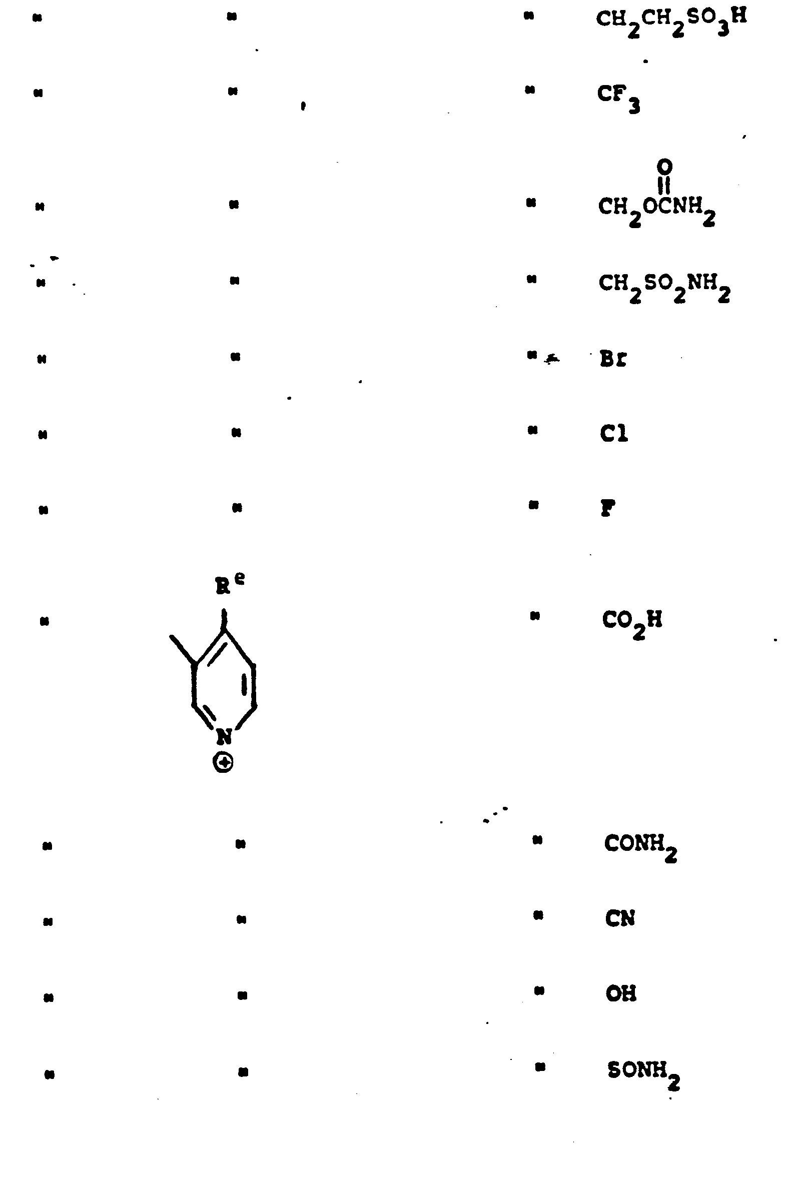

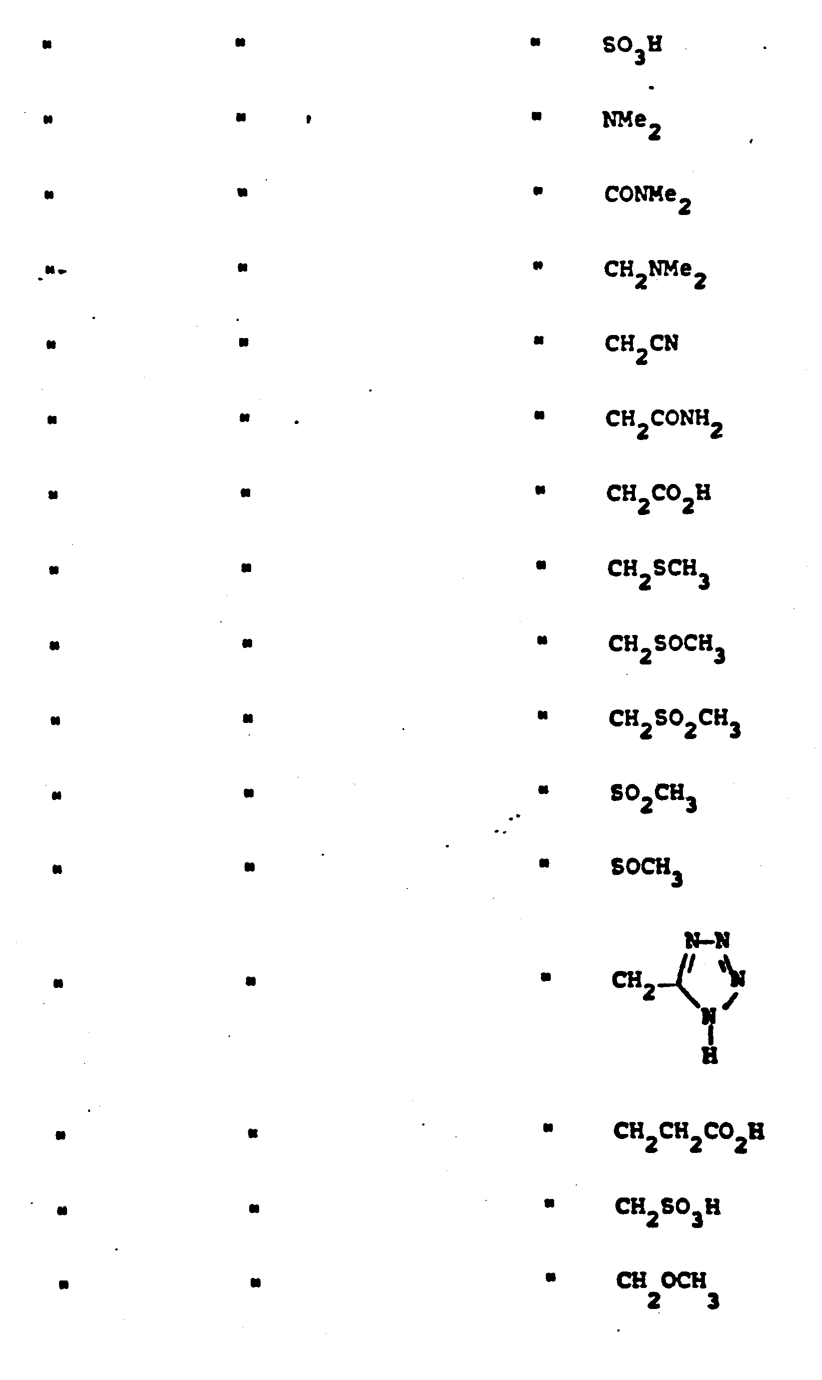

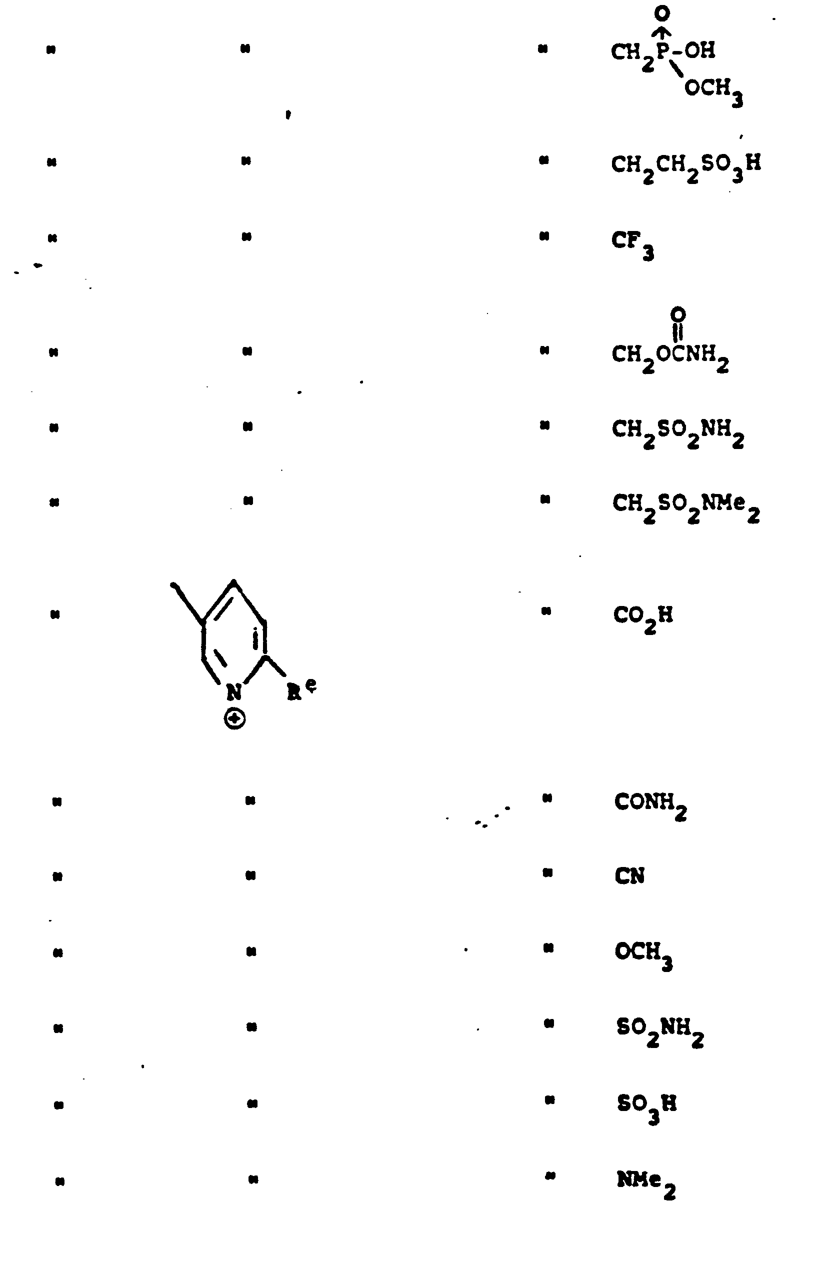

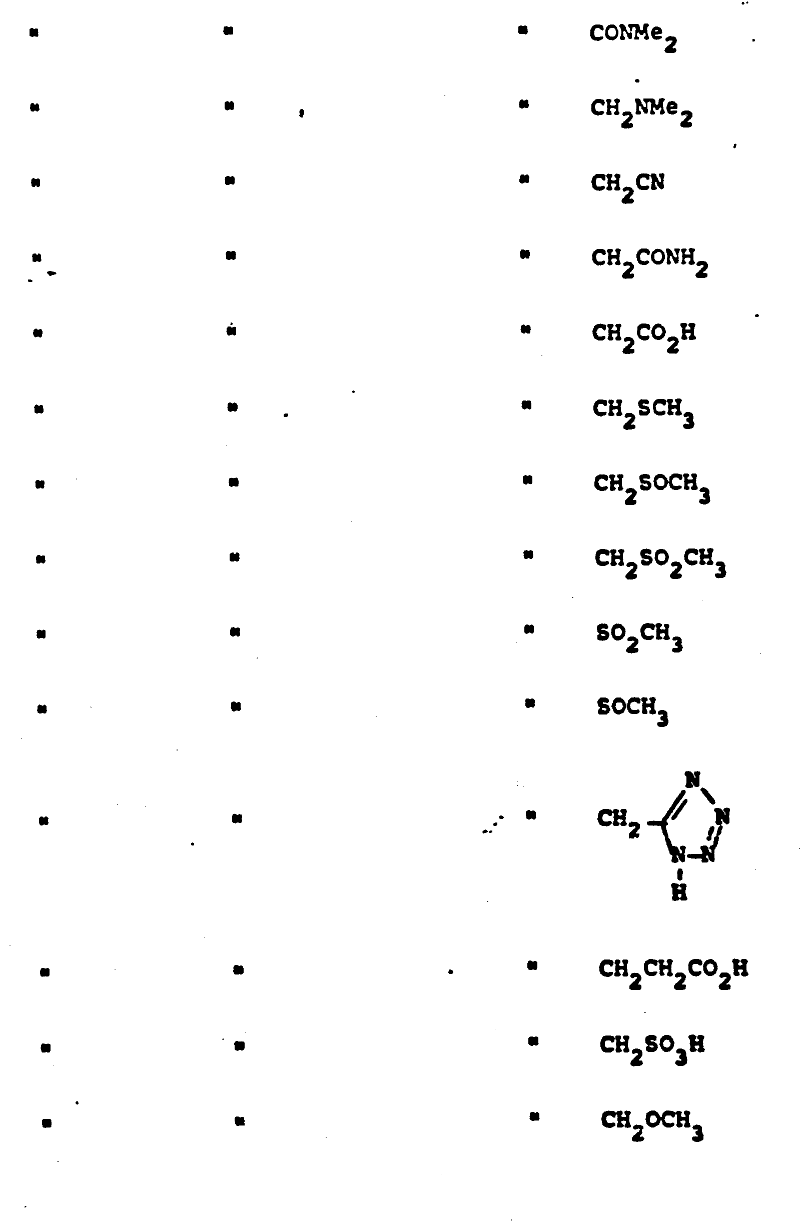

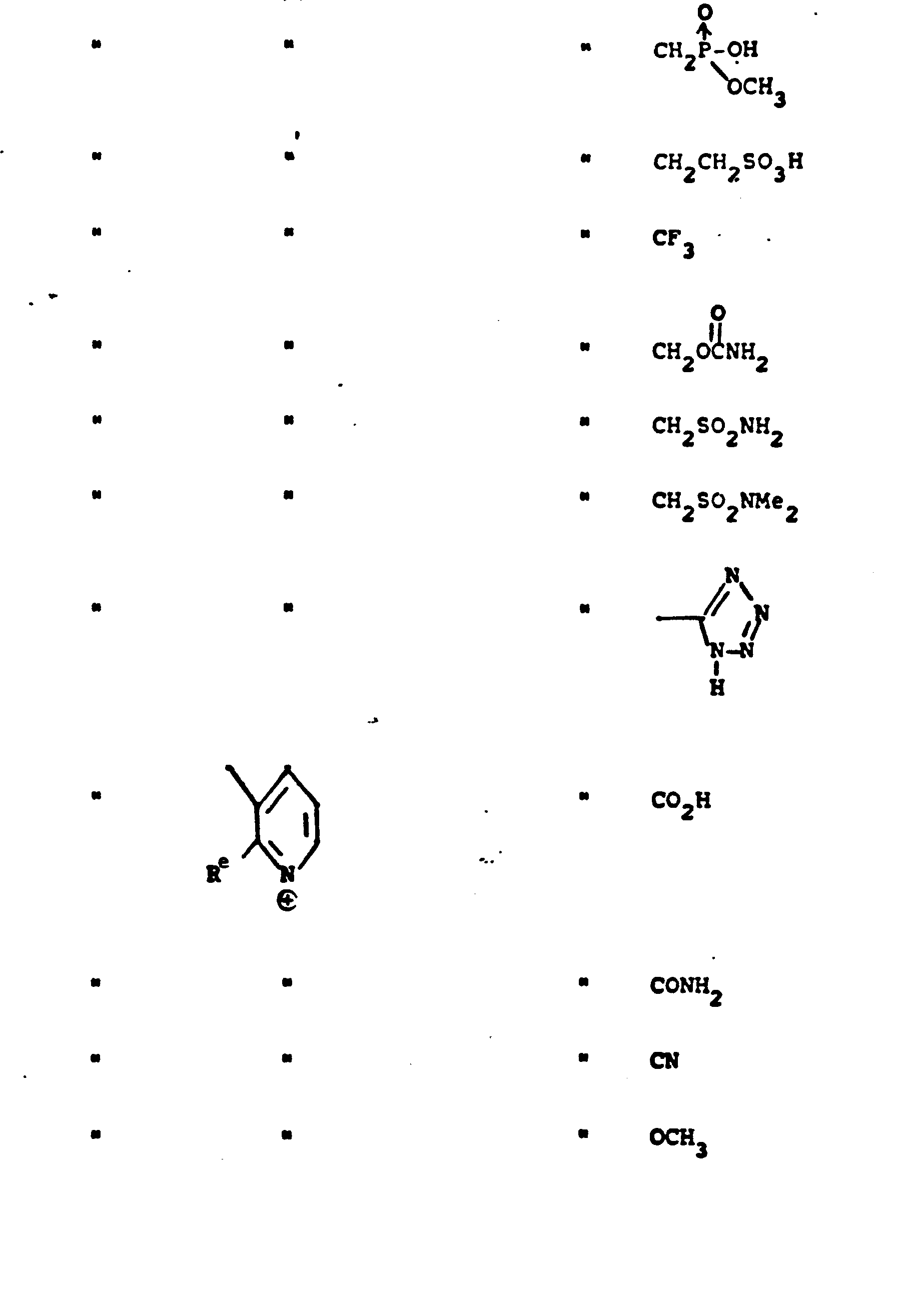

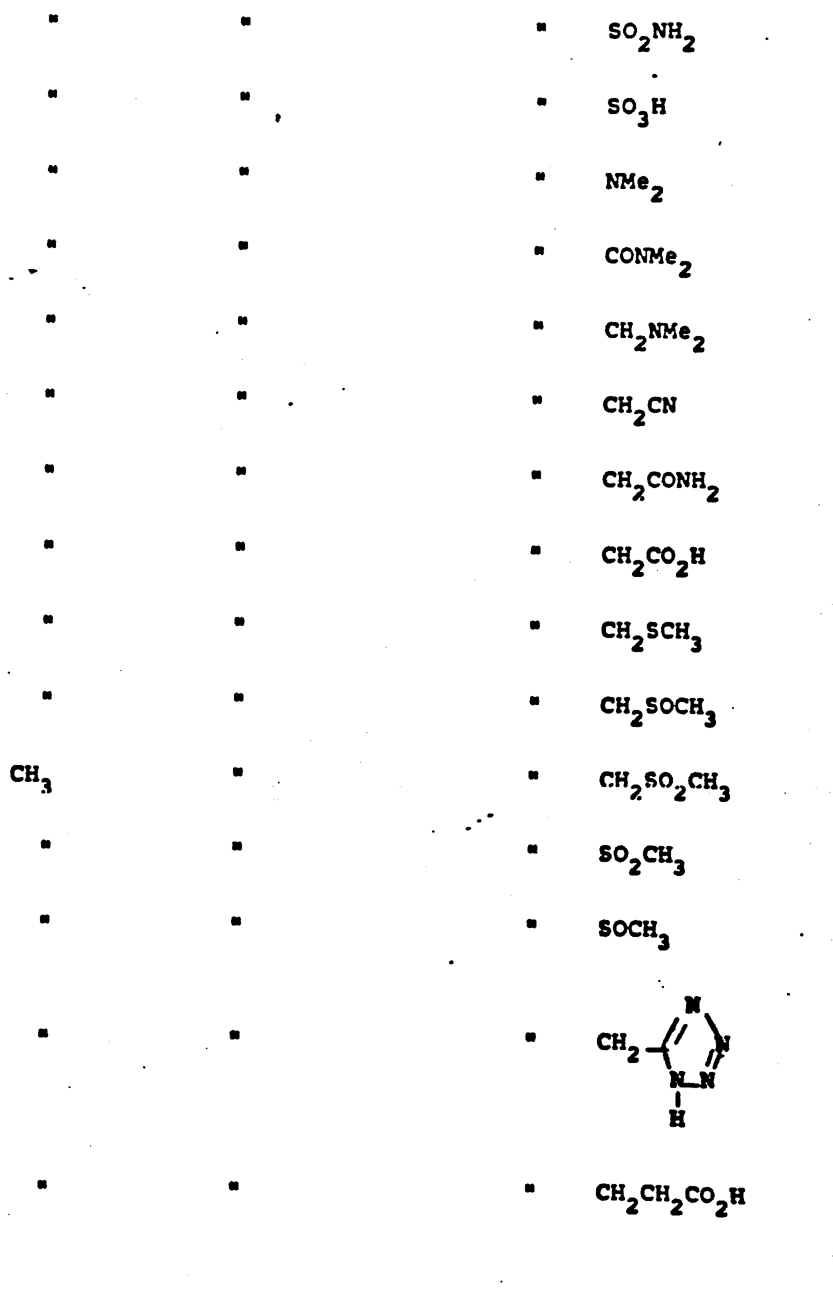

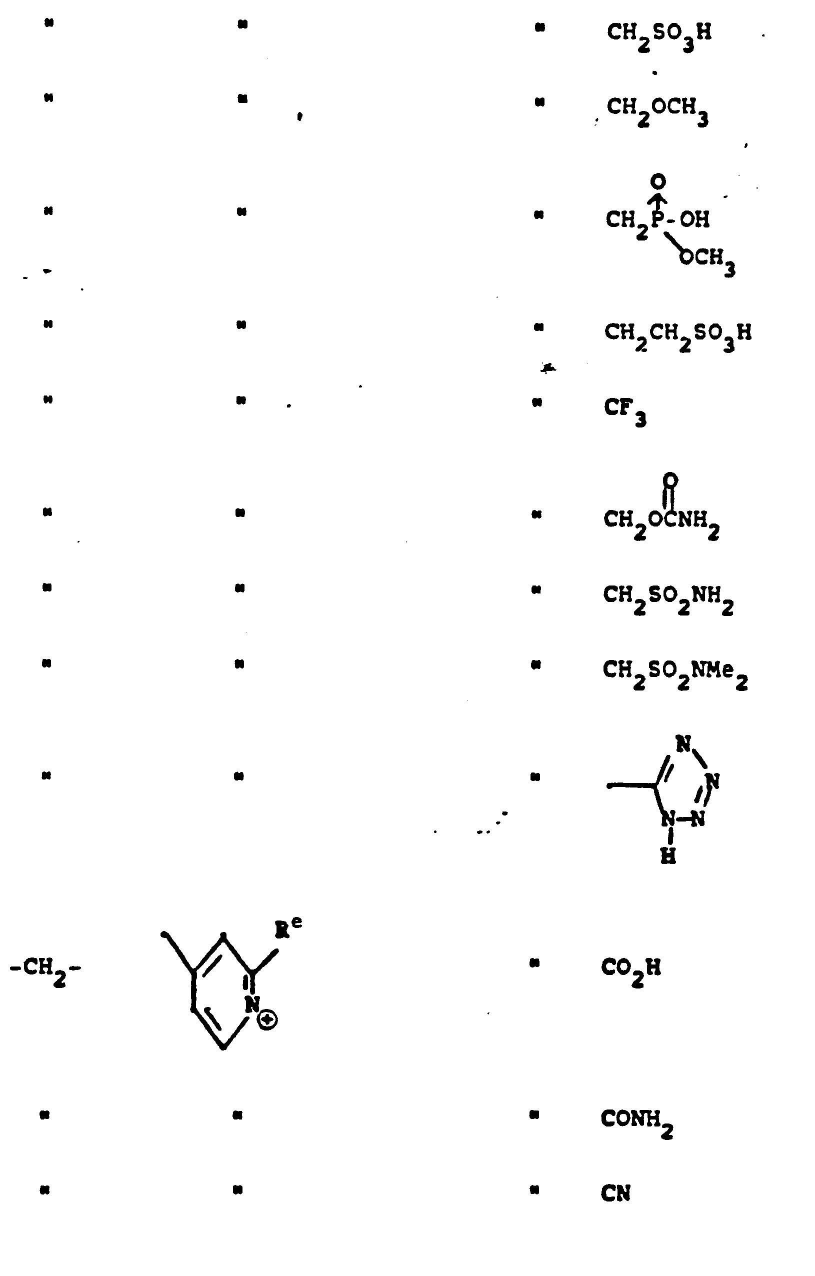

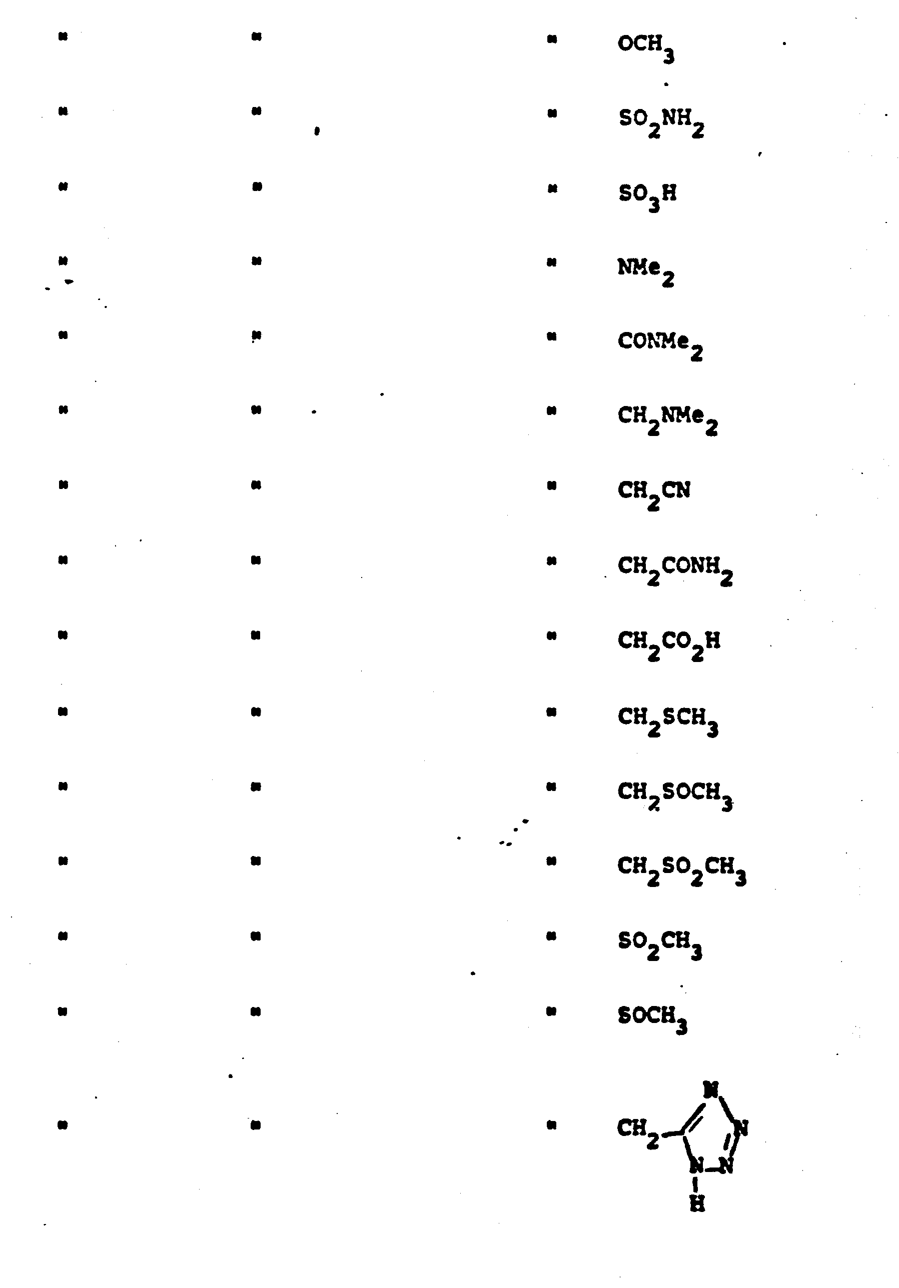

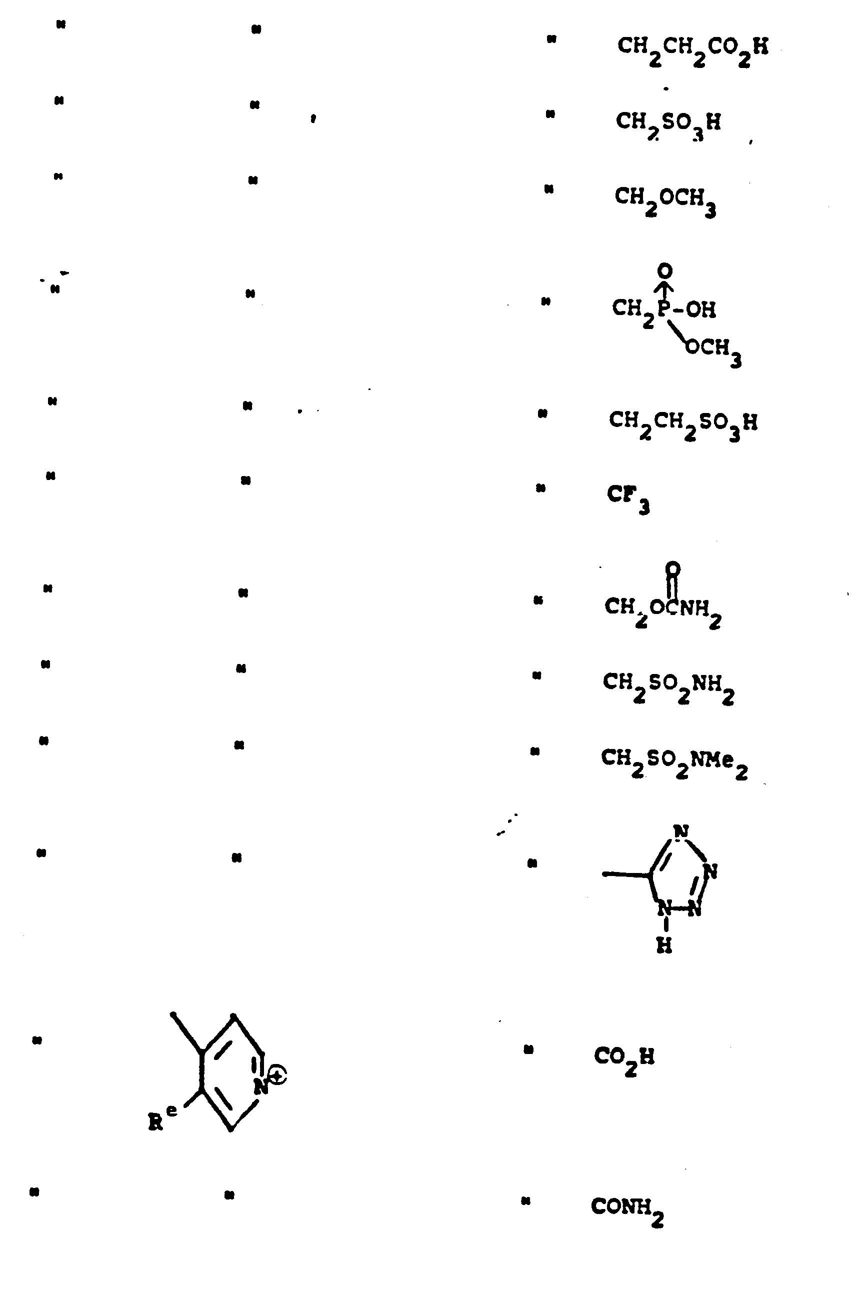

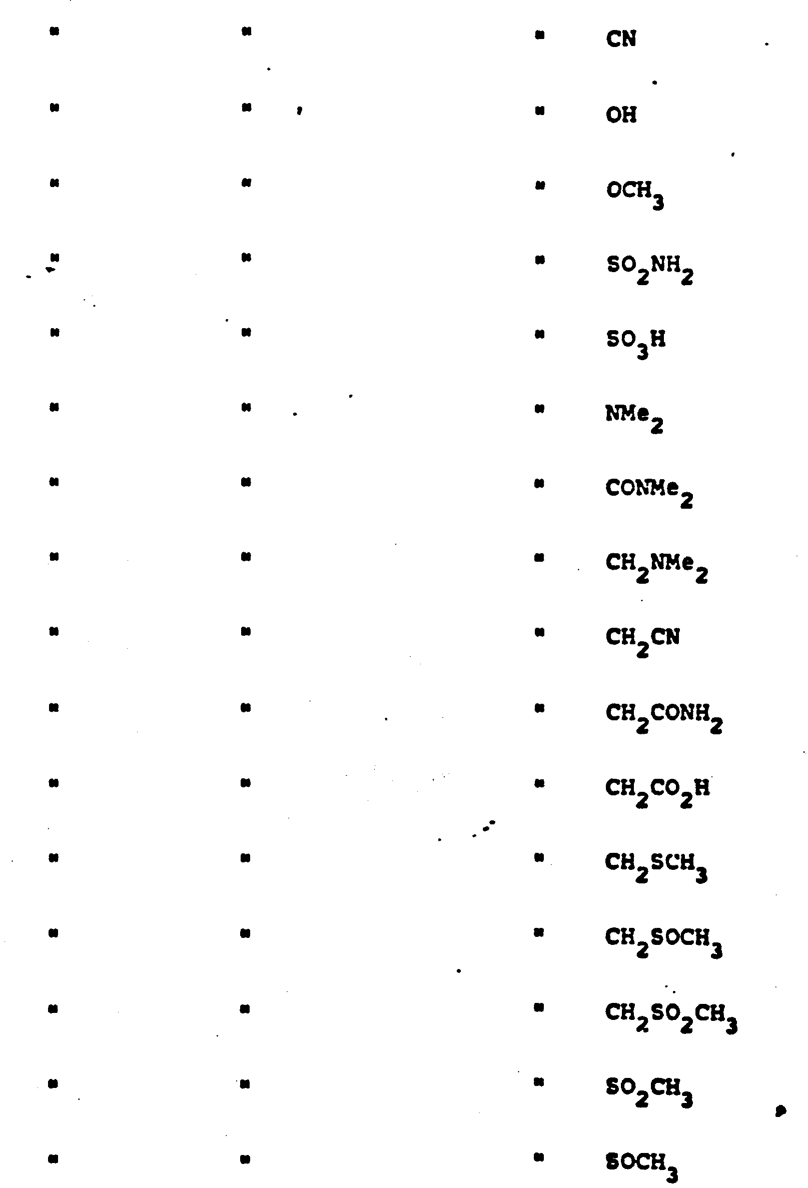

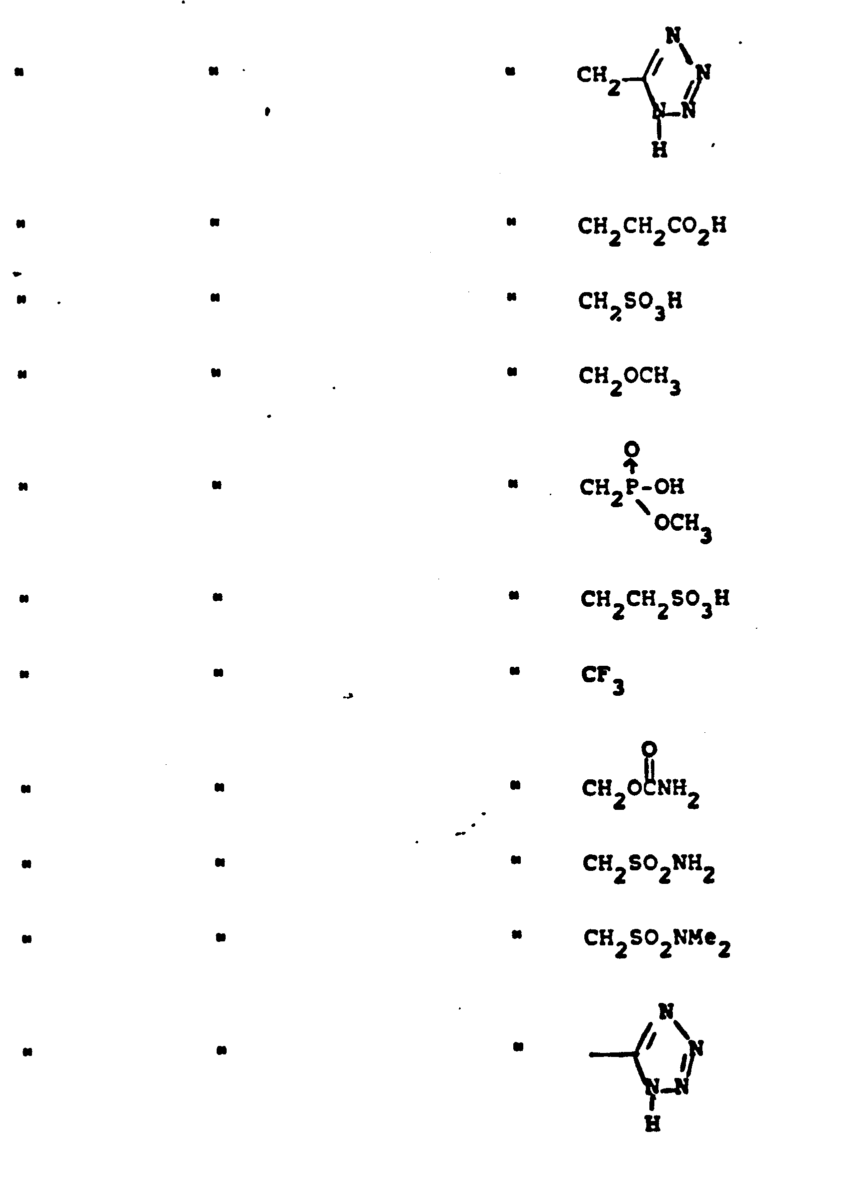

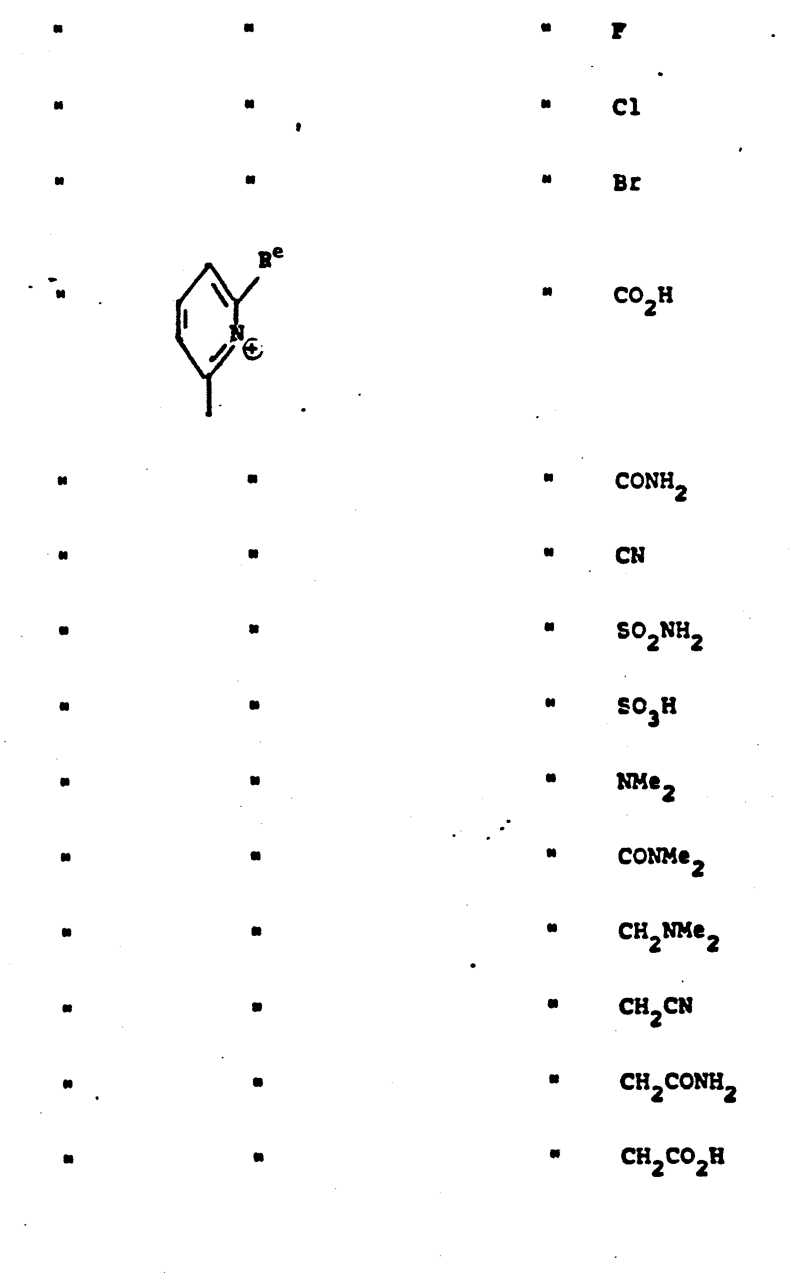

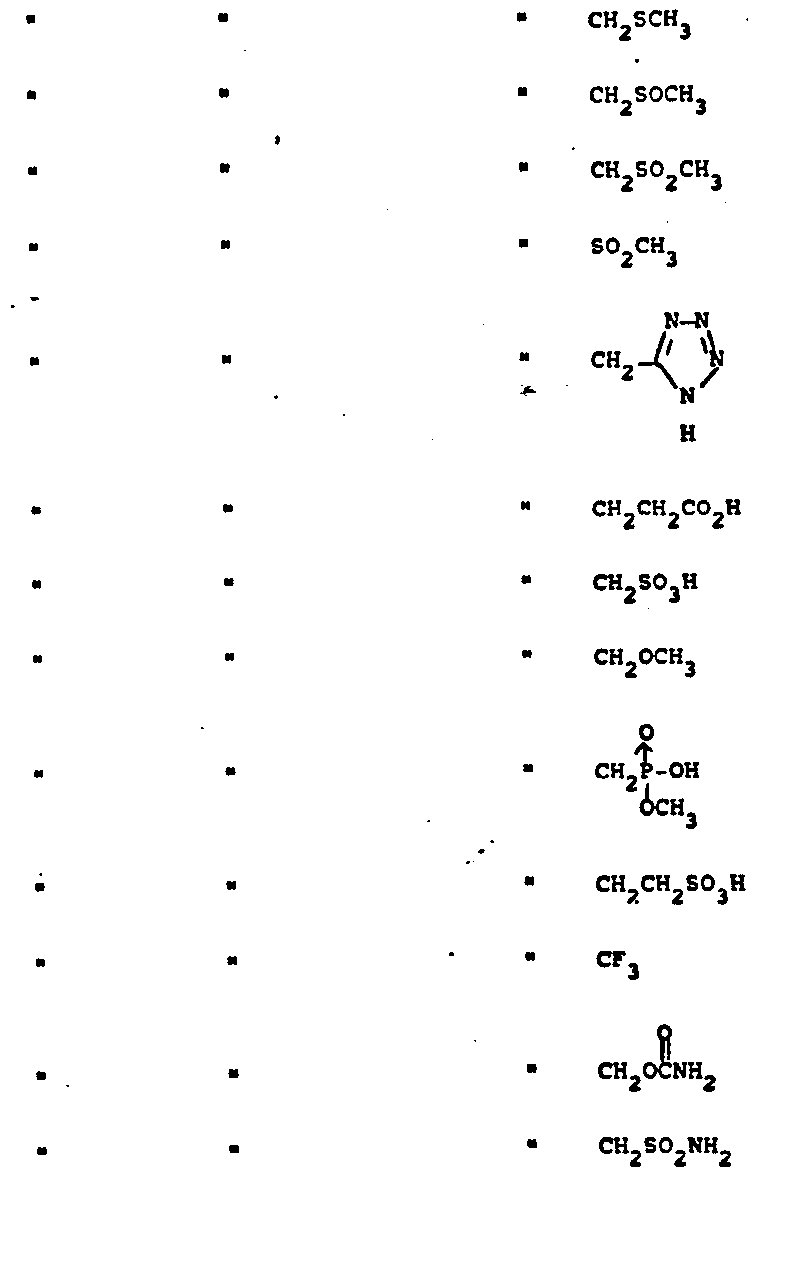

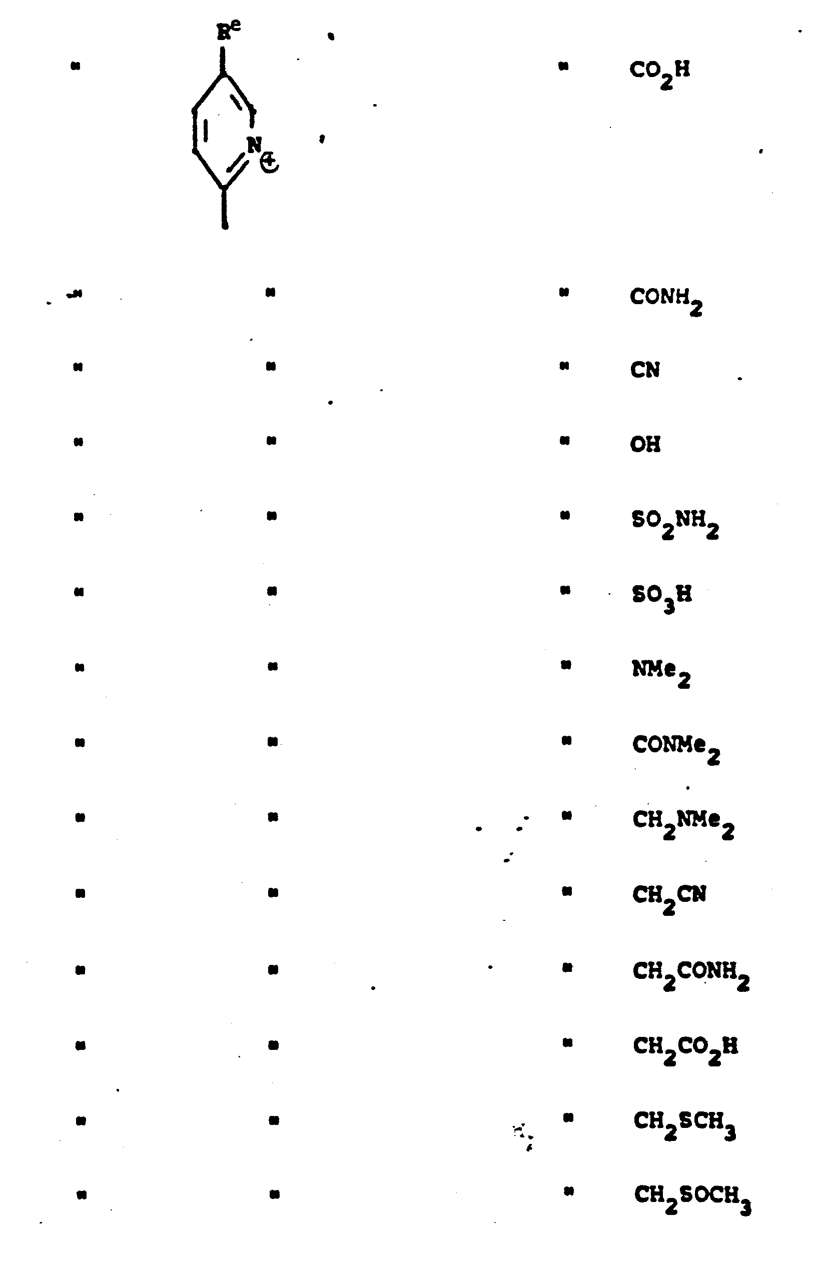

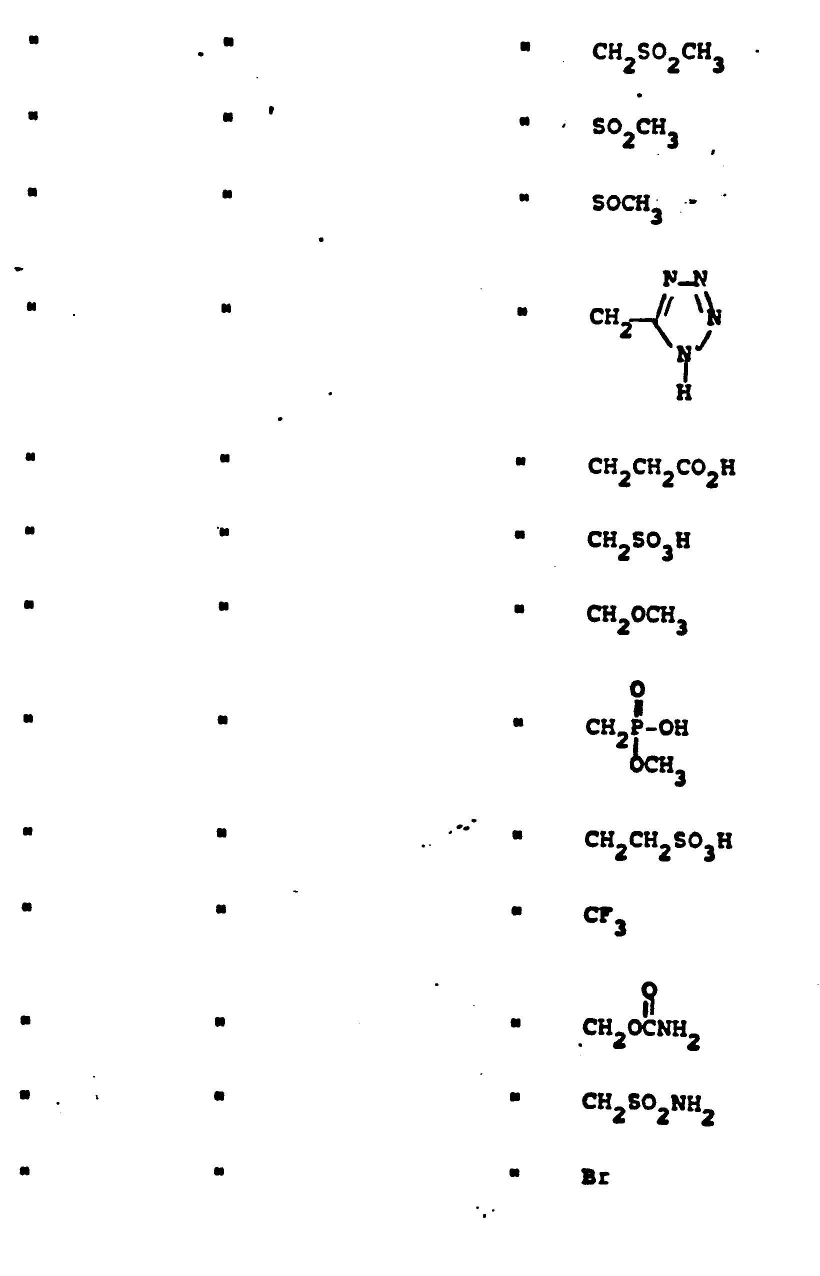

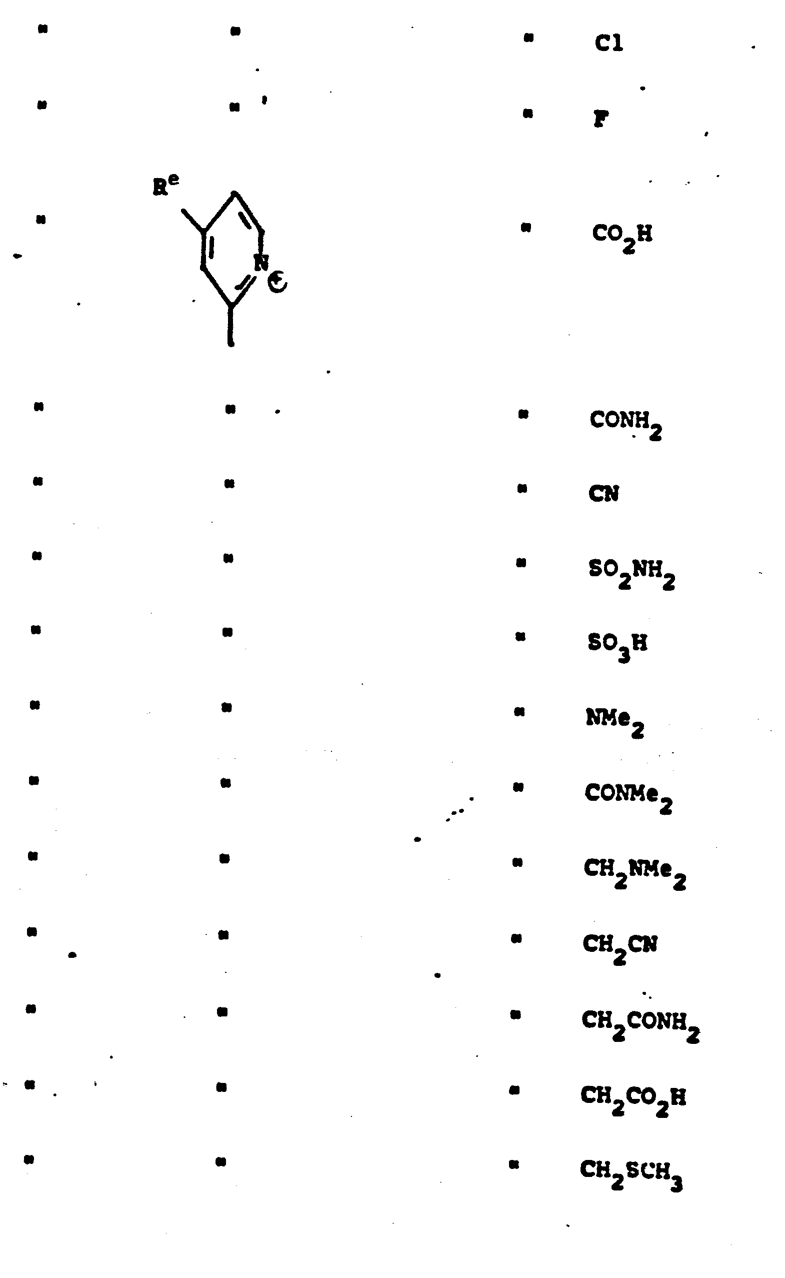

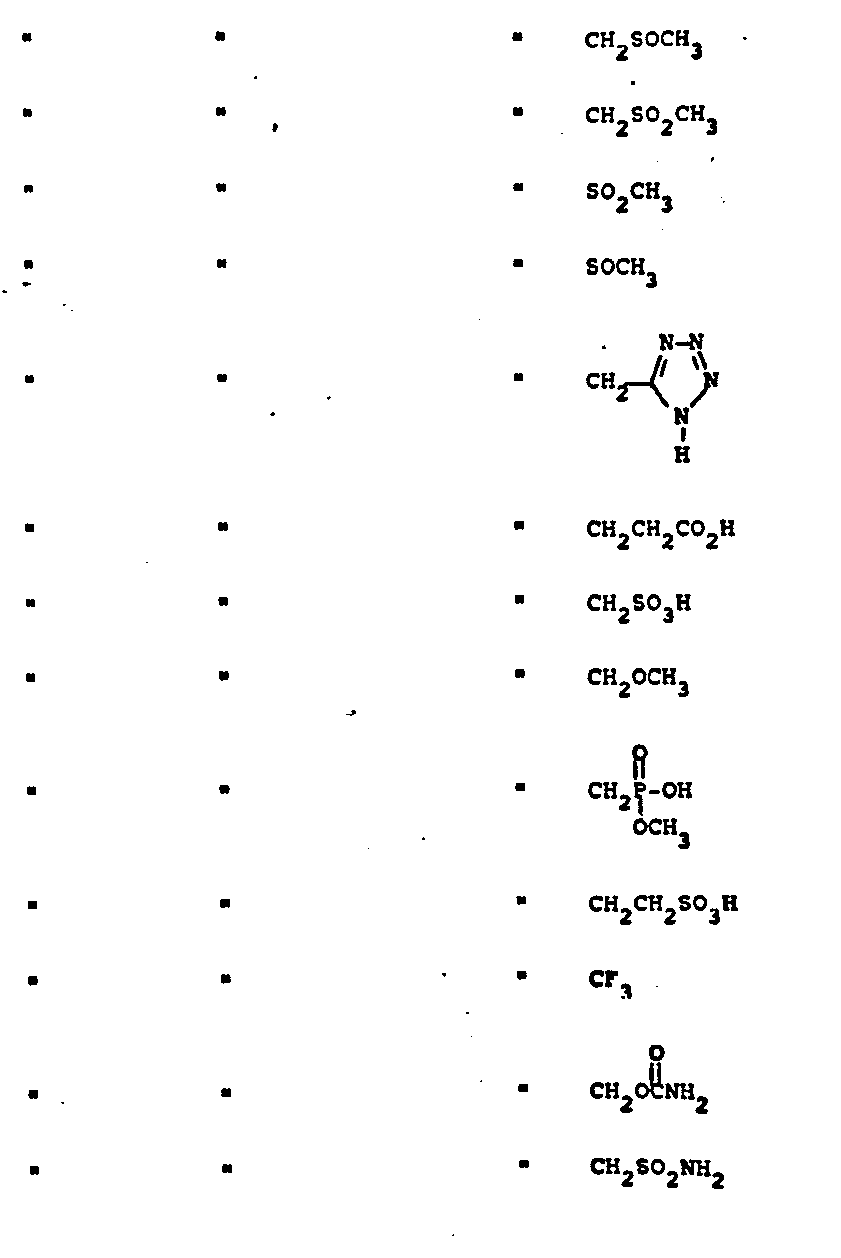

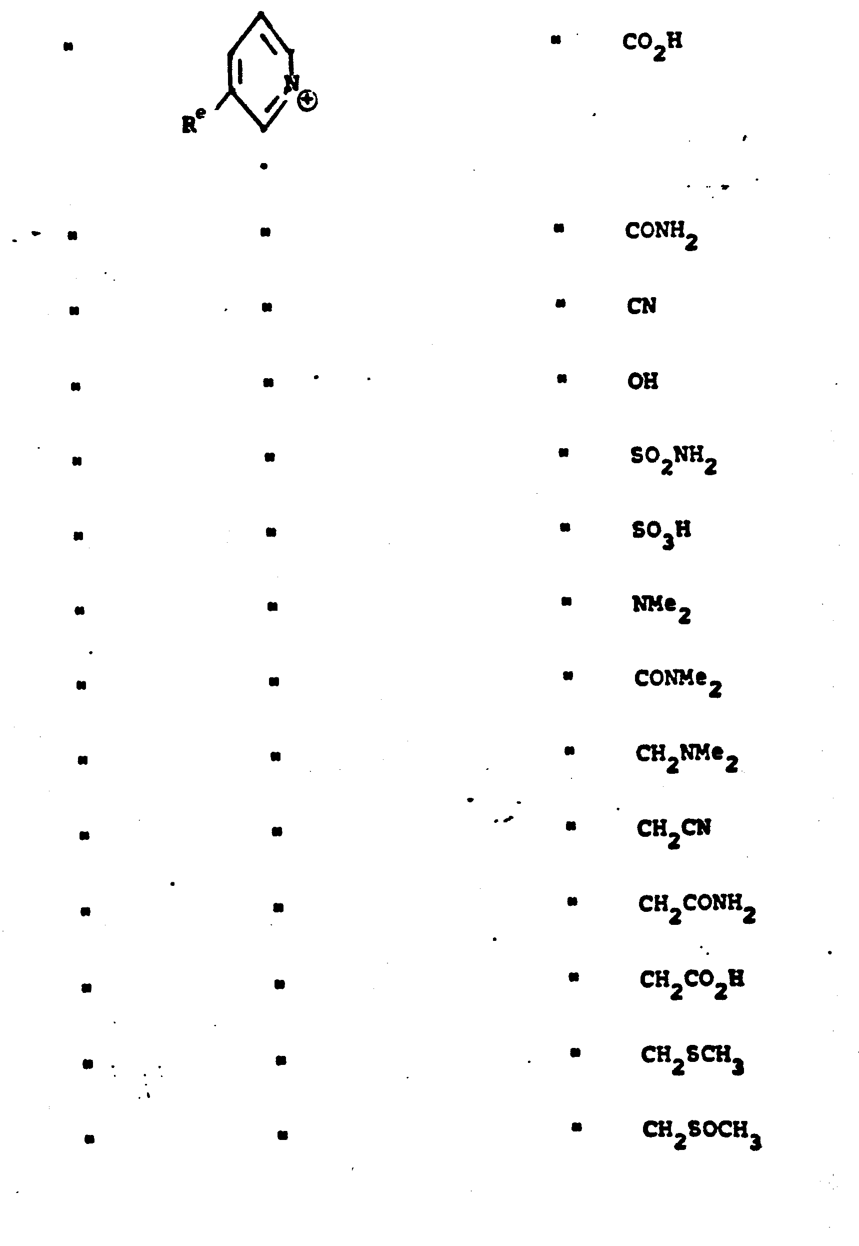

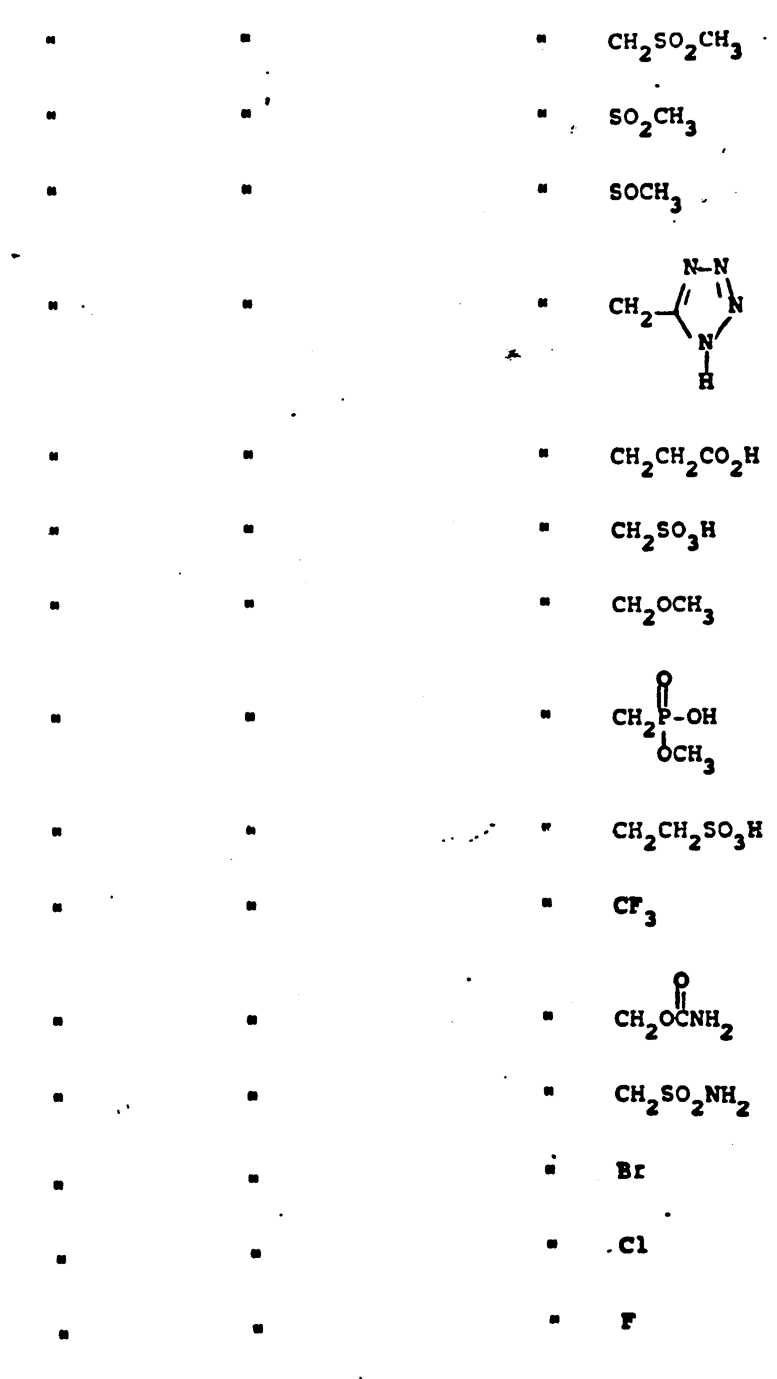

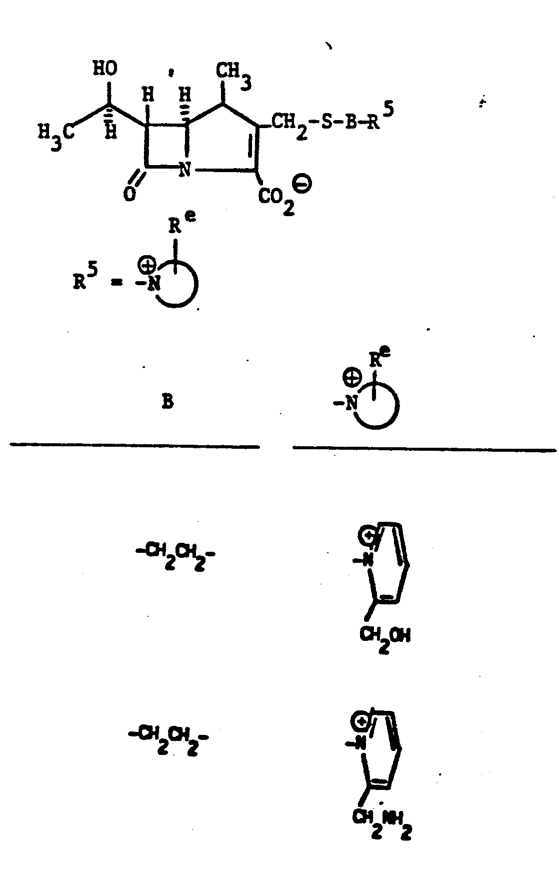

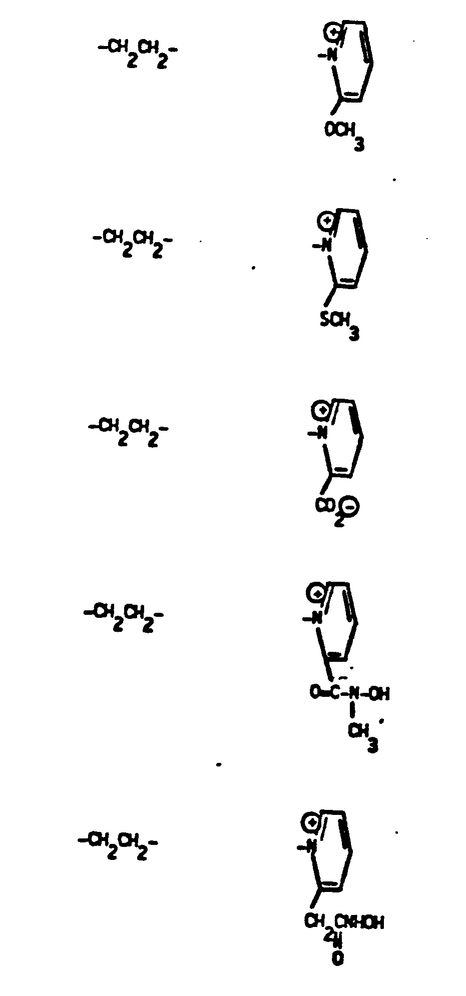

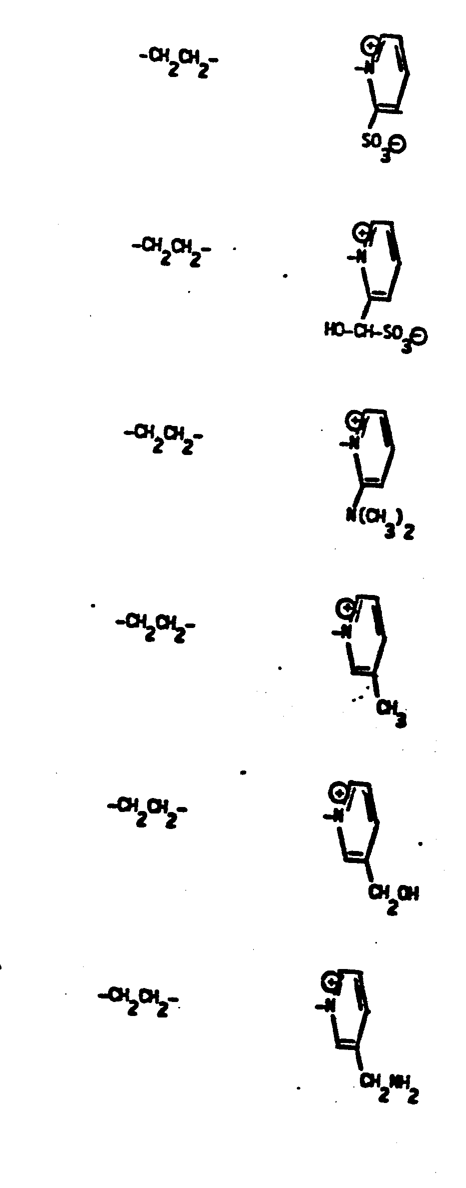

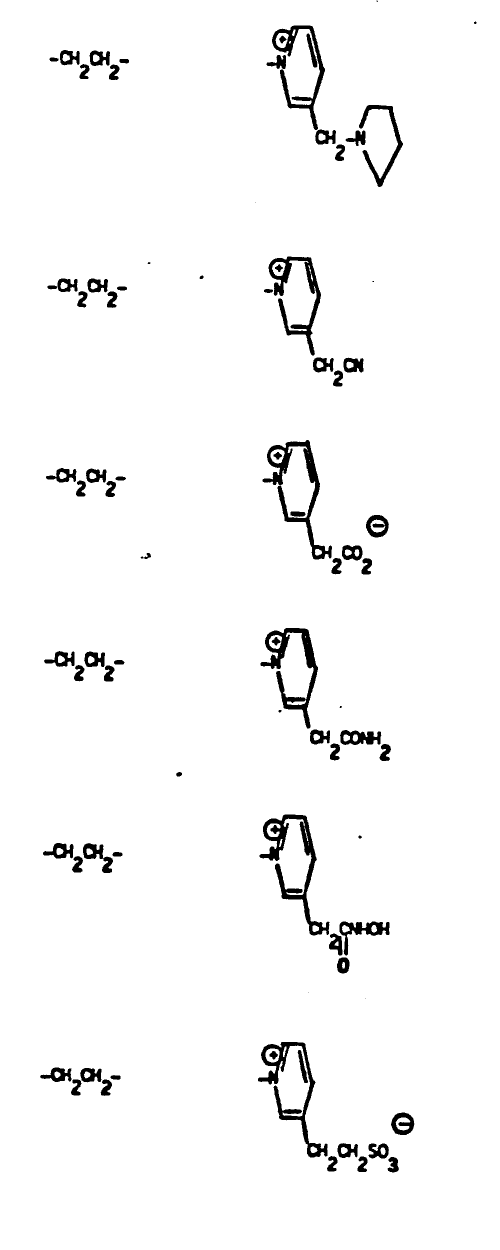

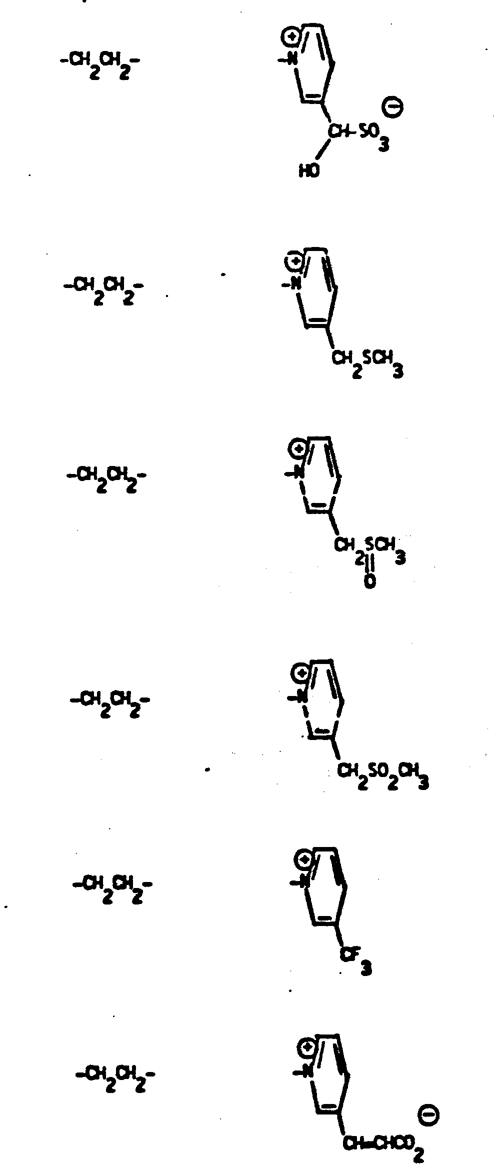

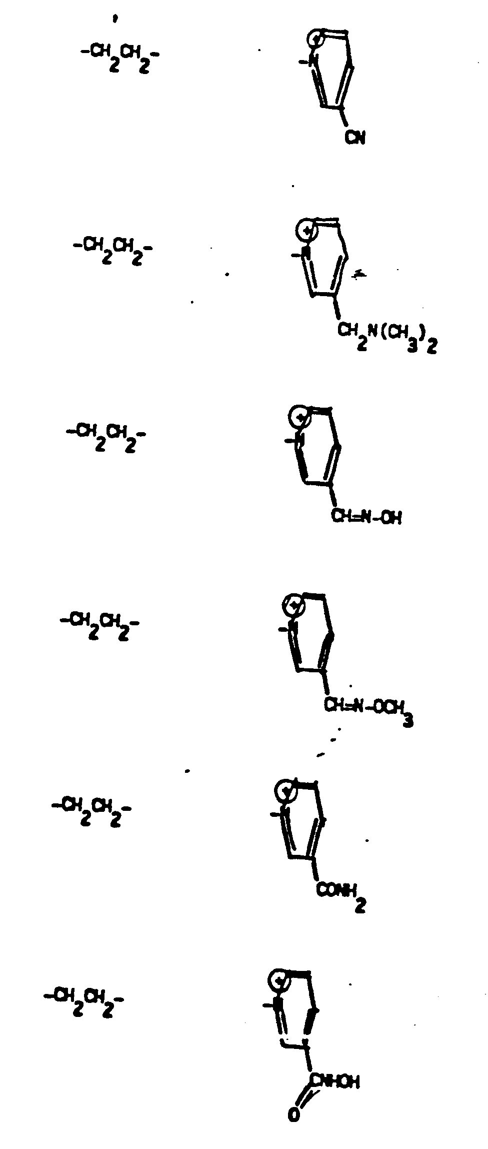

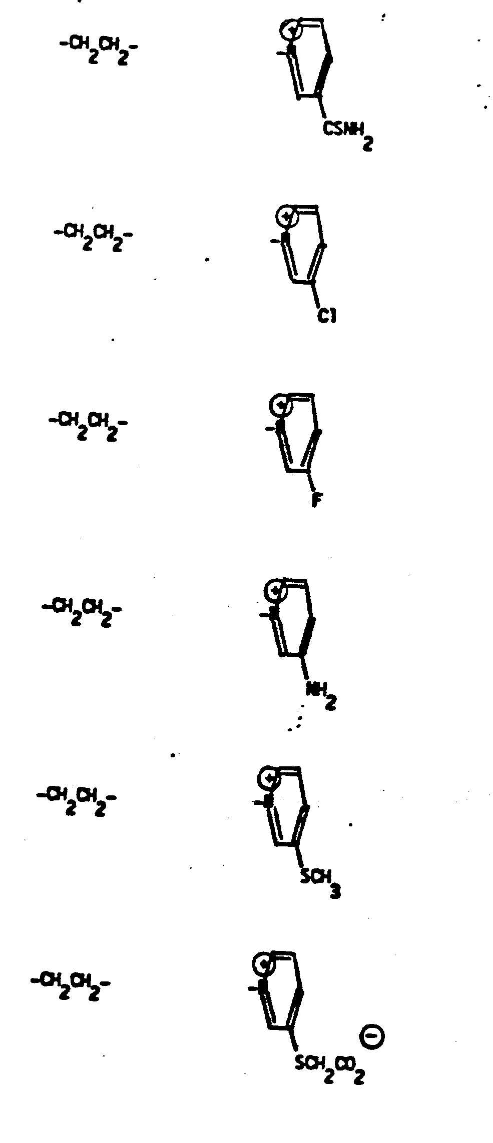

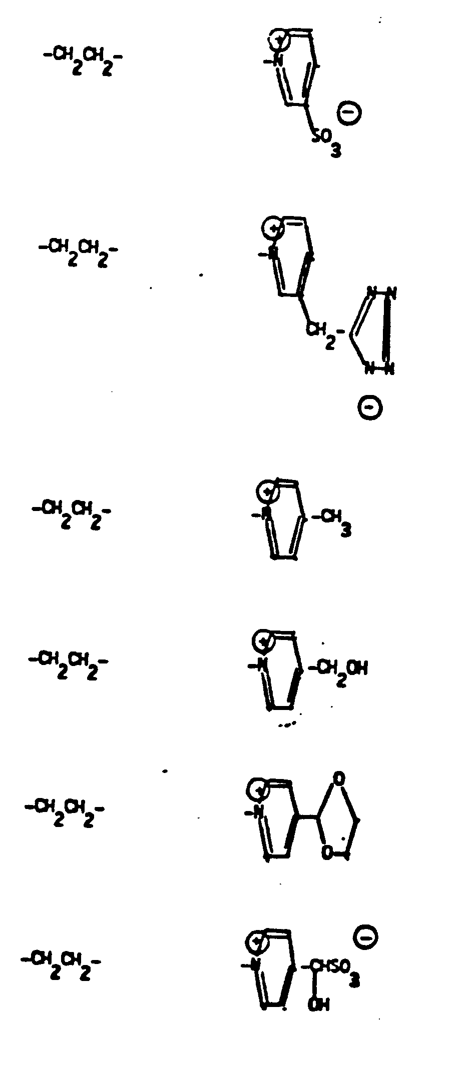

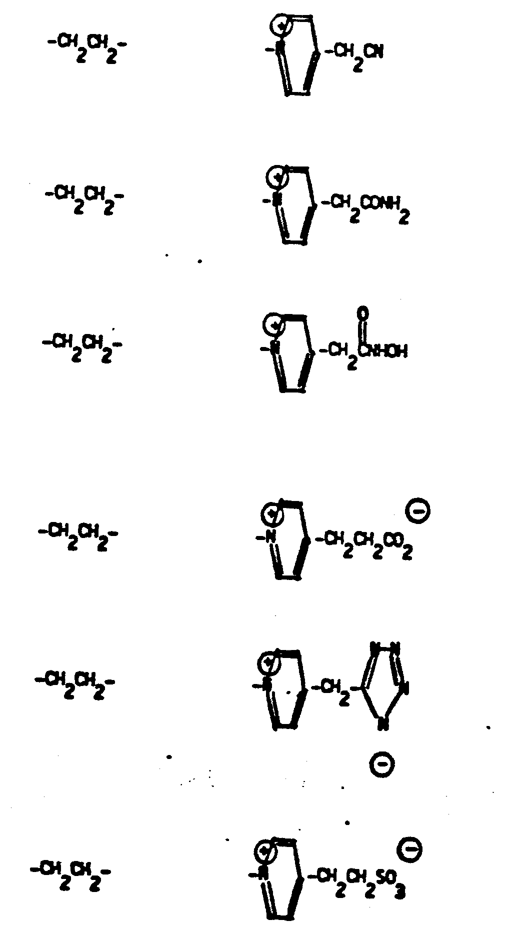

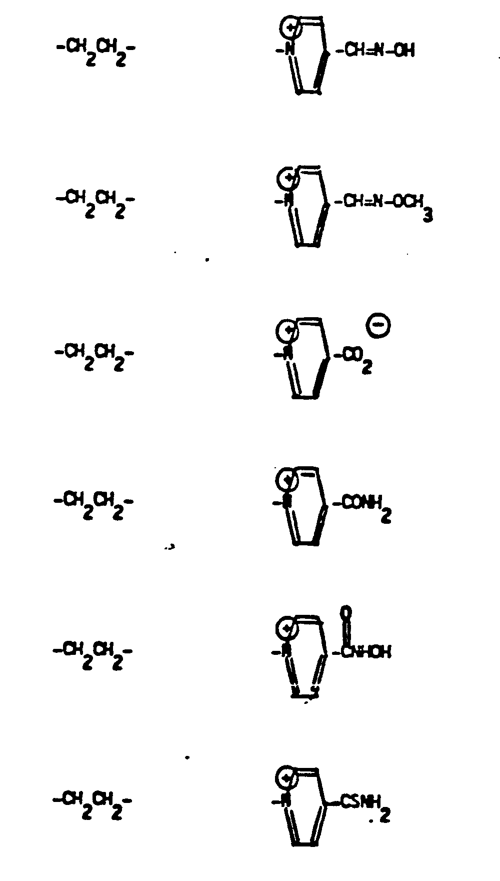

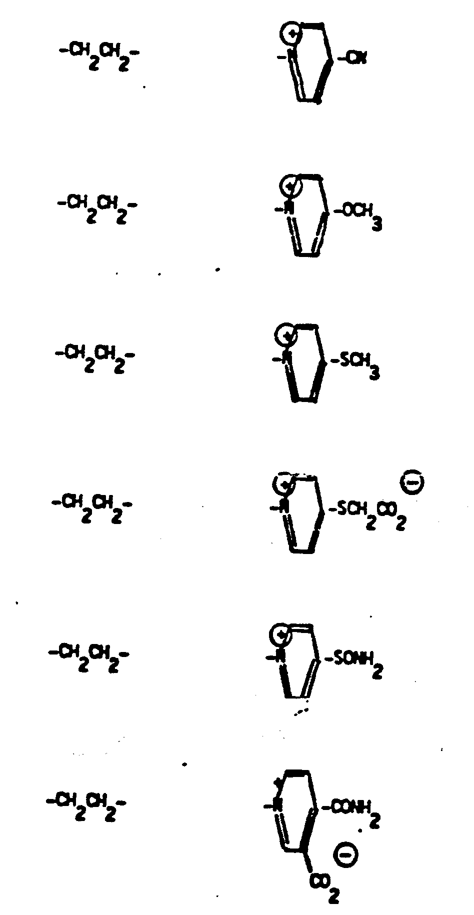

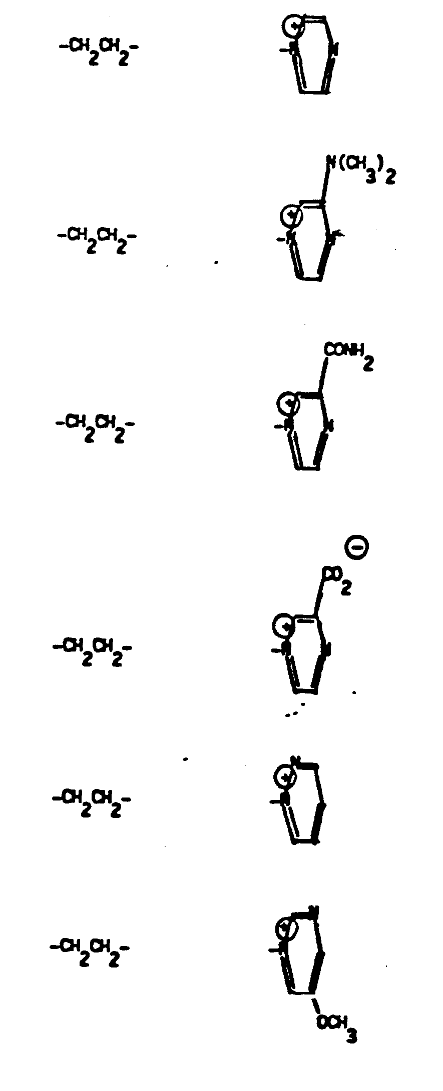

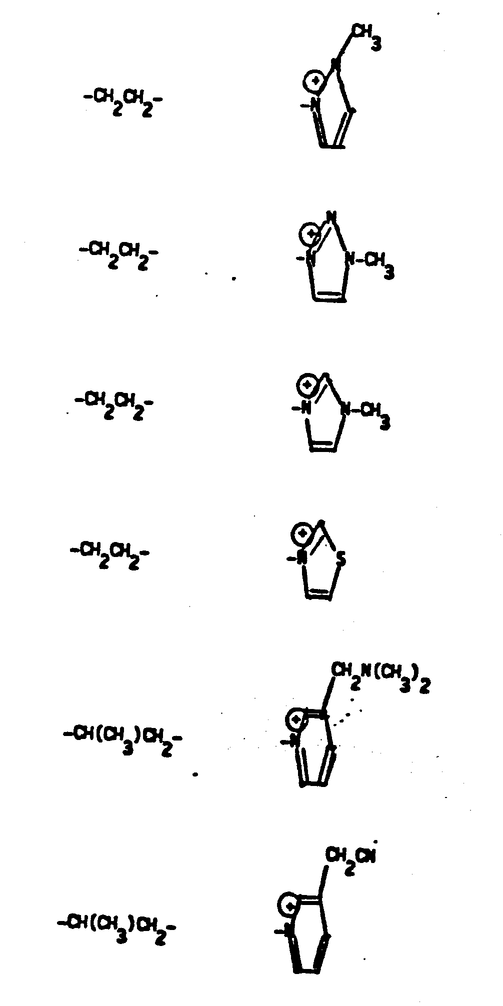

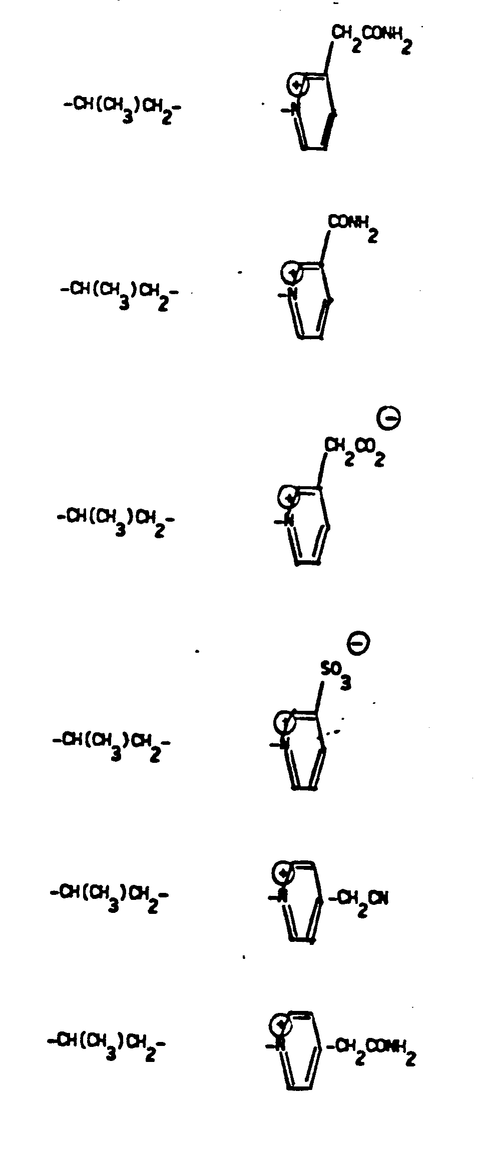

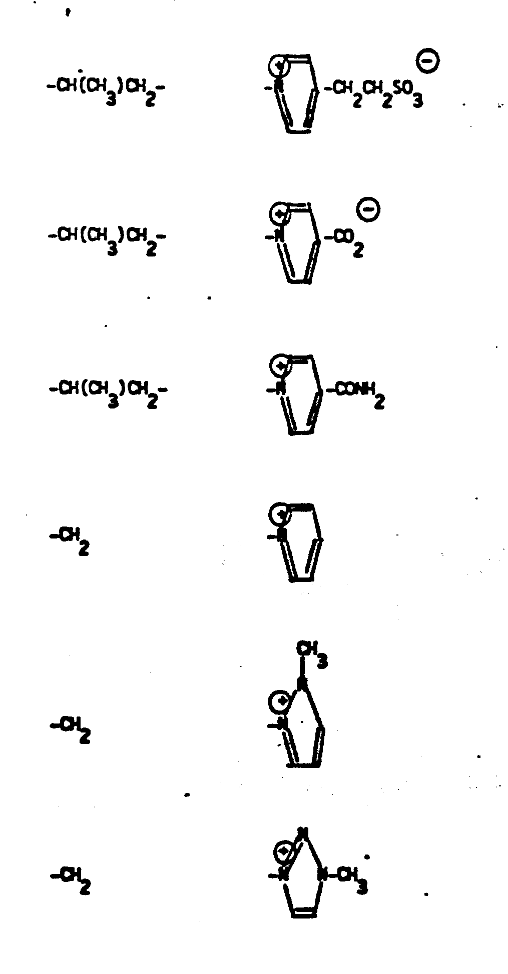

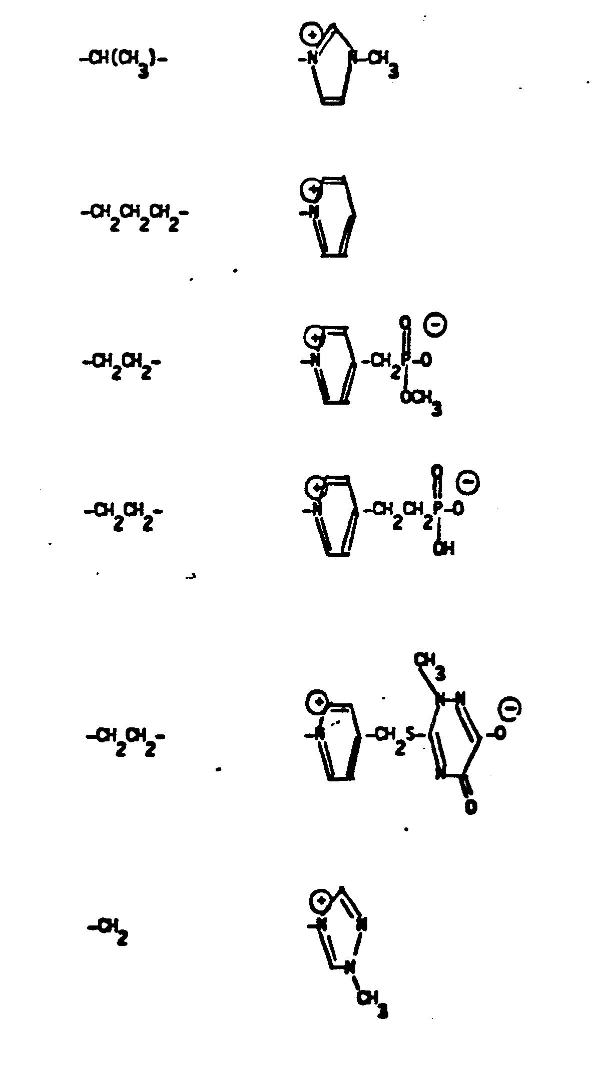

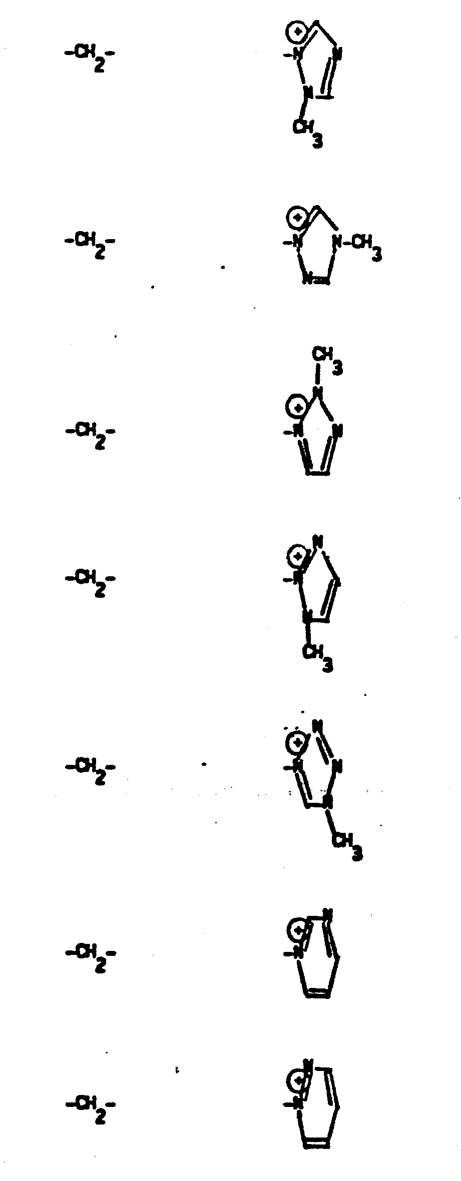

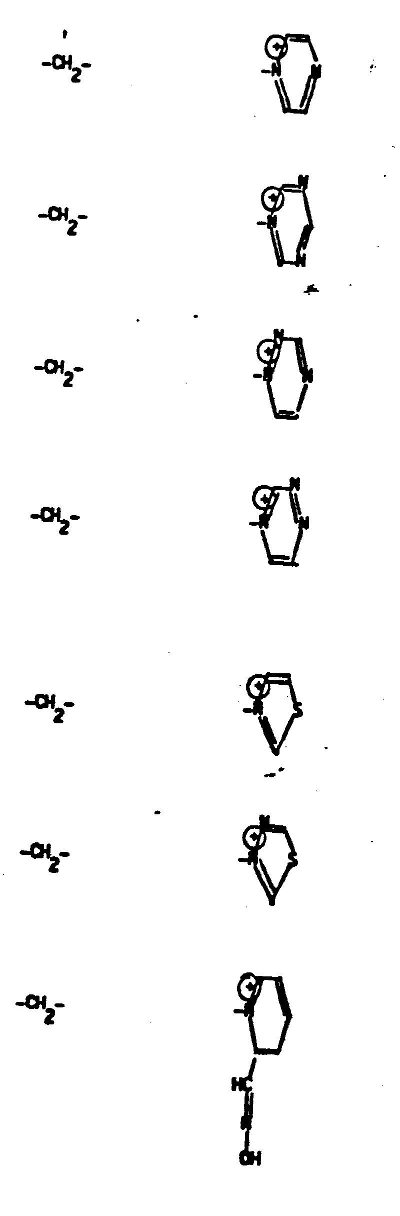

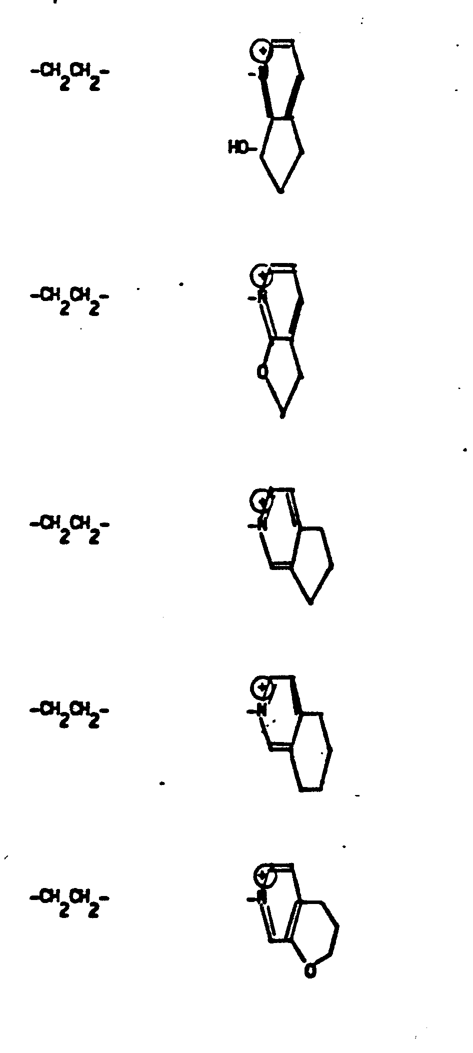

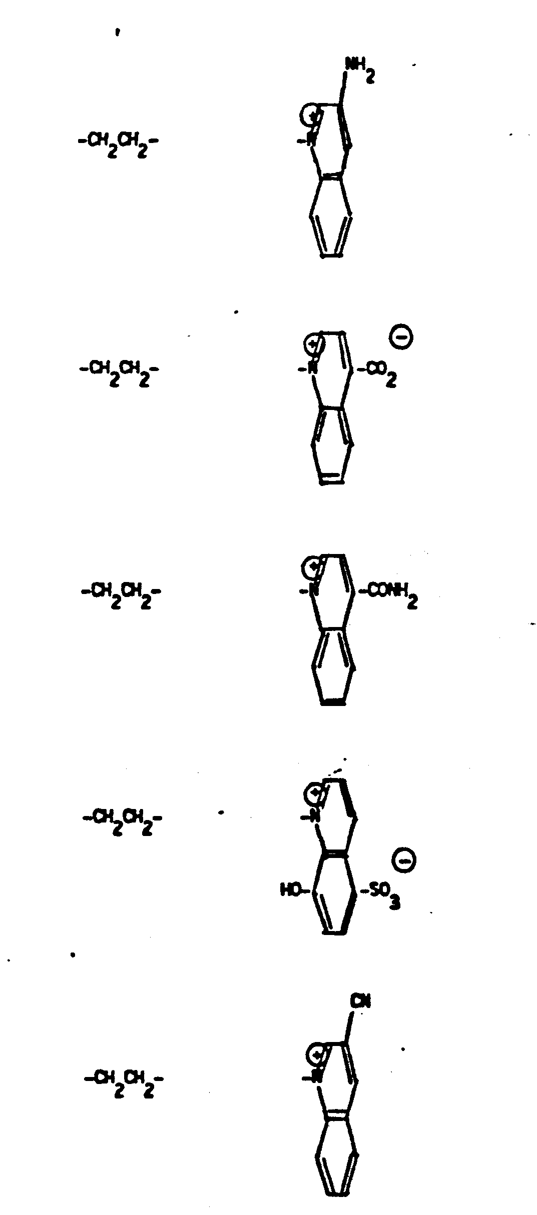

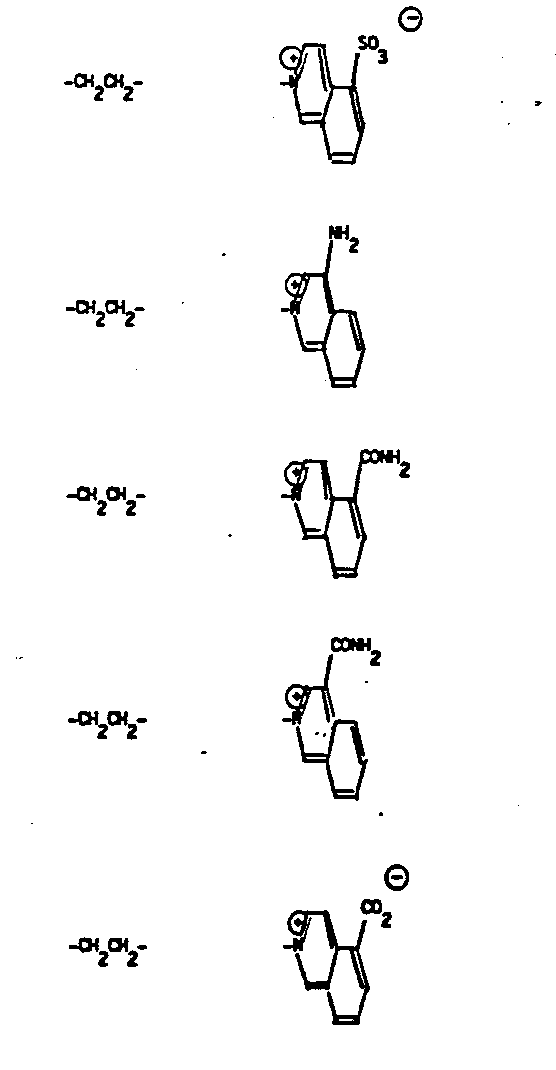

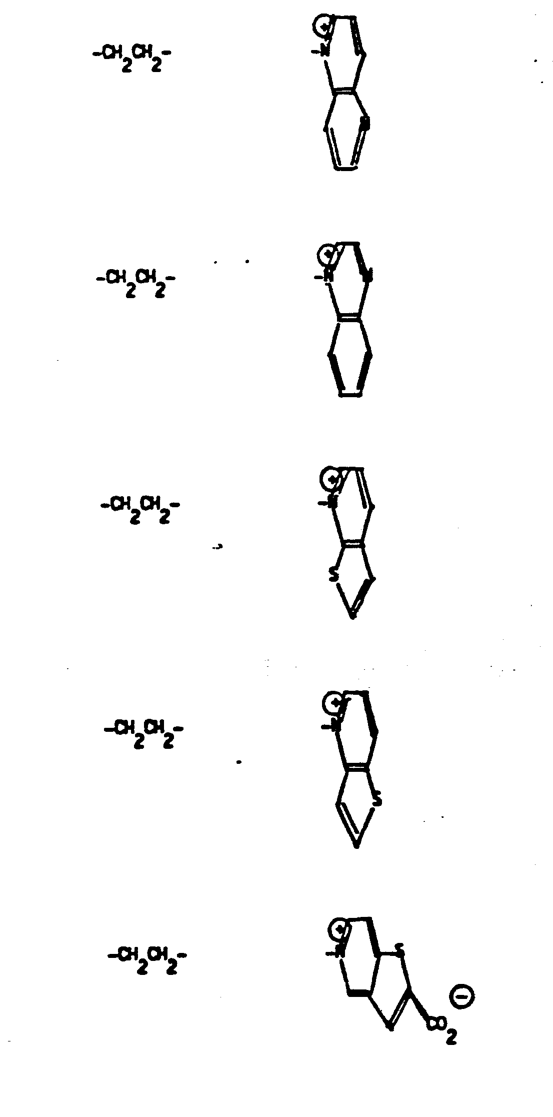

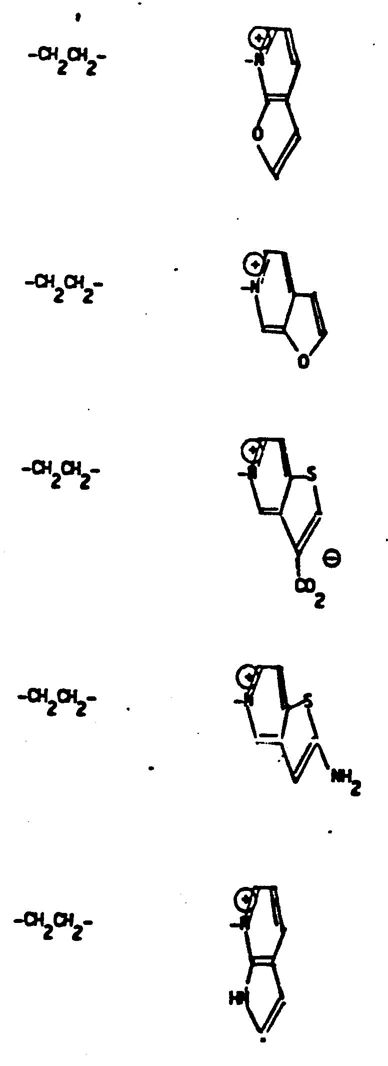

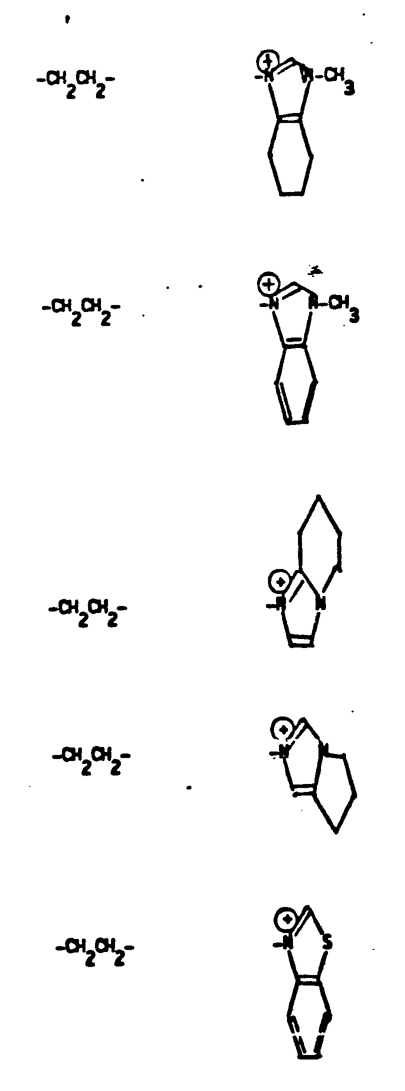

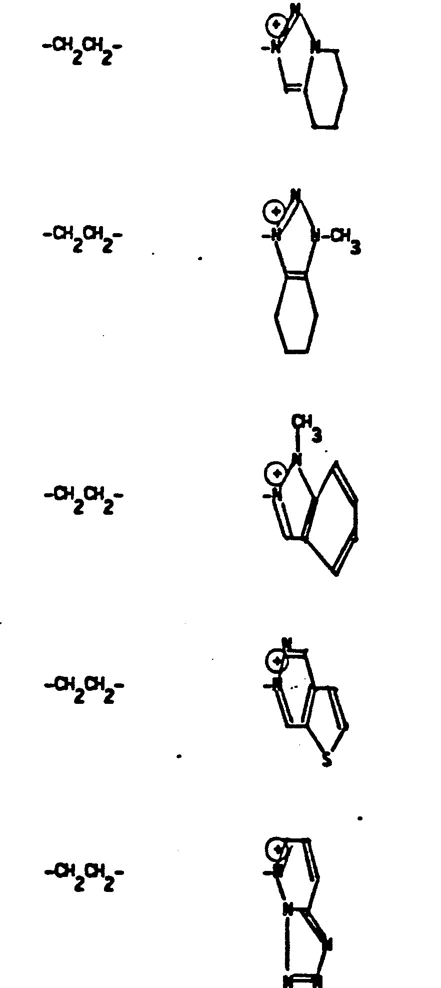

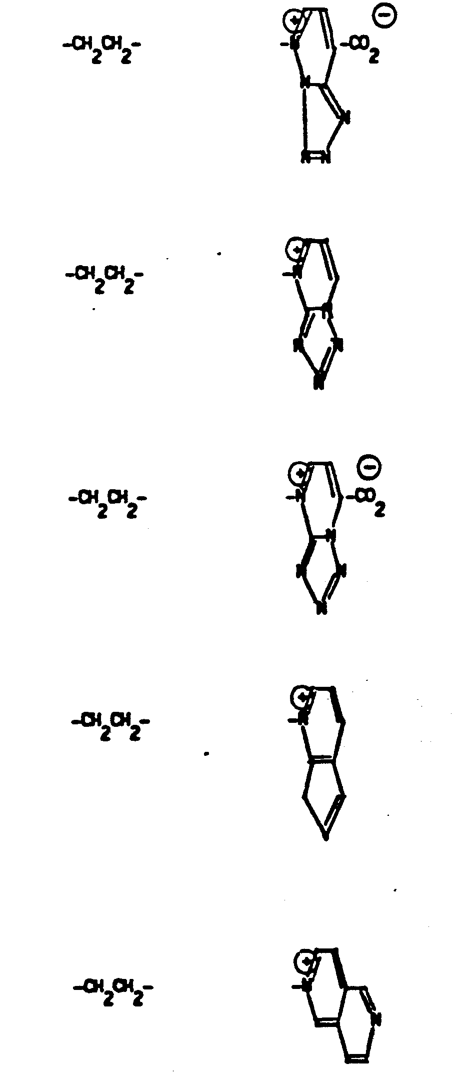

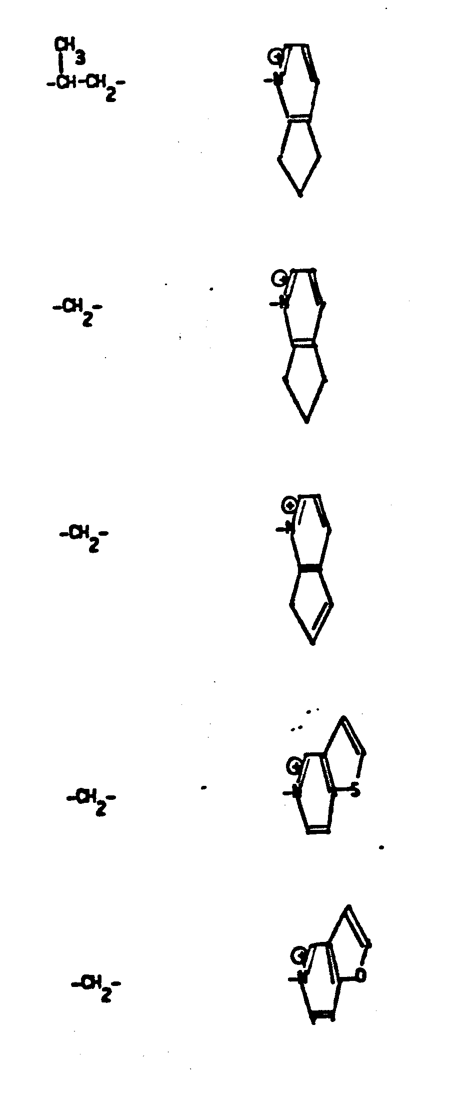

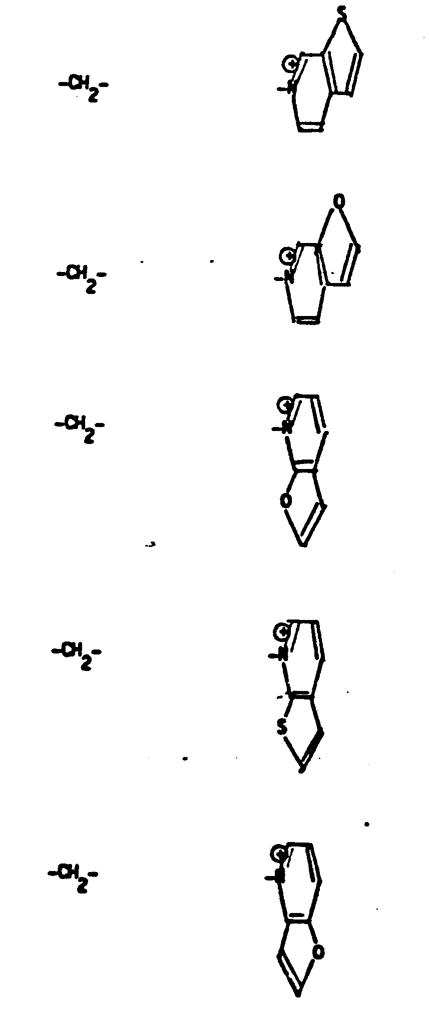

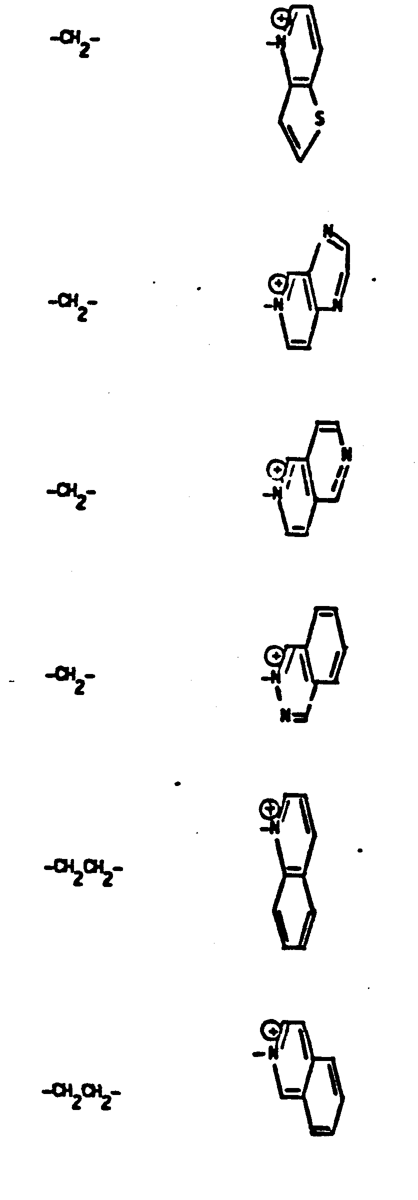

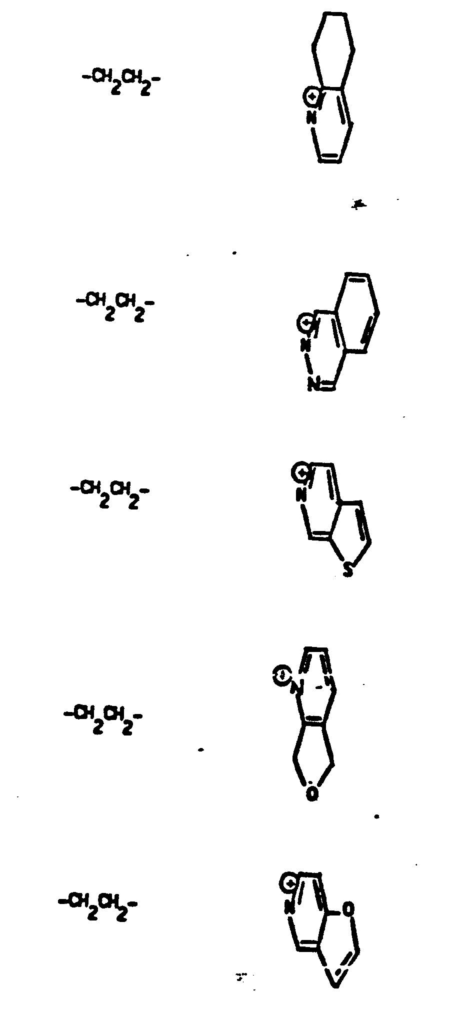

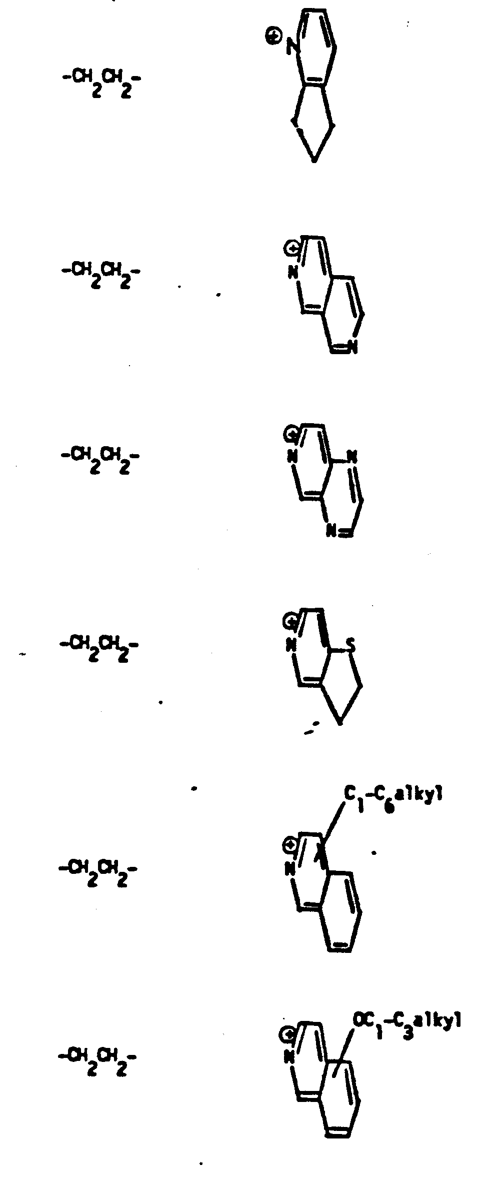

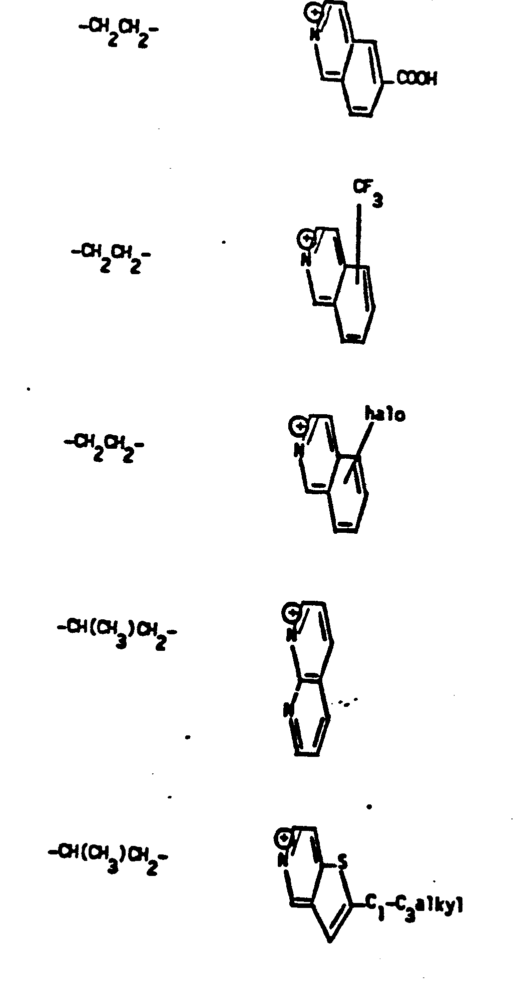

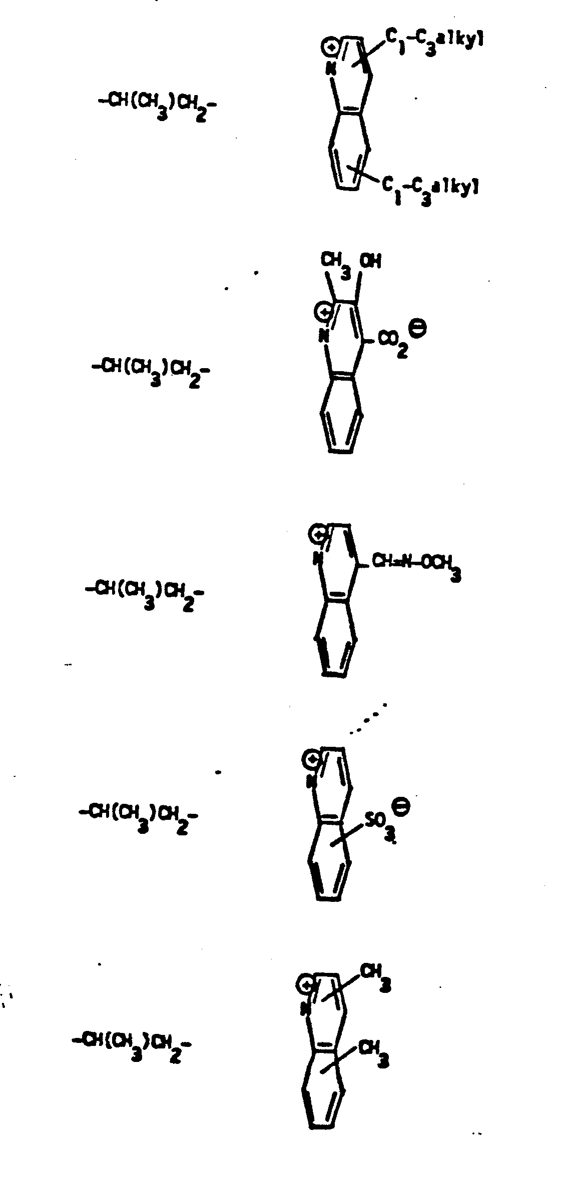

### 10. Amidino and Amidinium

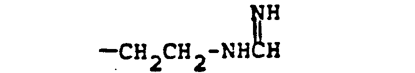

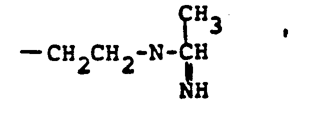

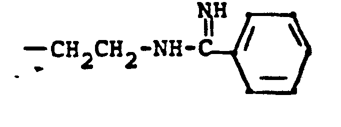

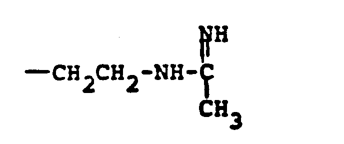

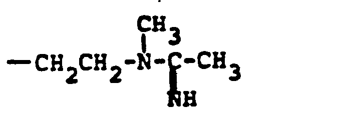

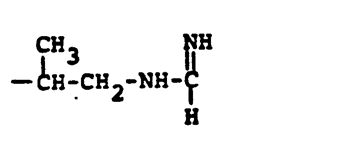

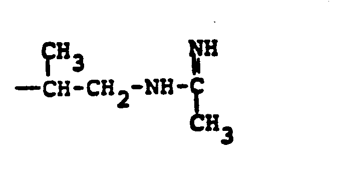

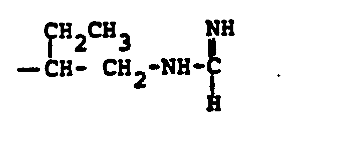

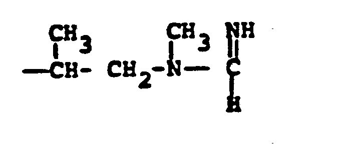

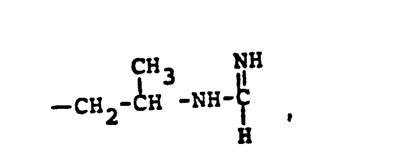

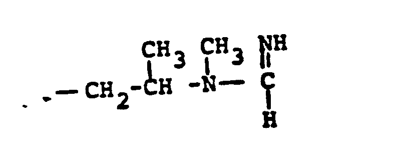

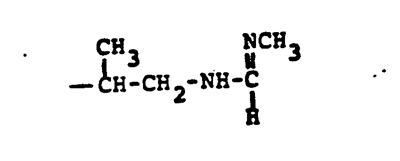

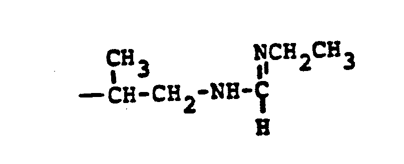

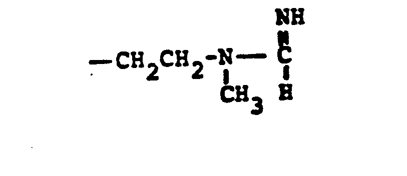

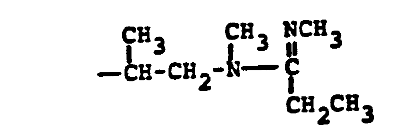

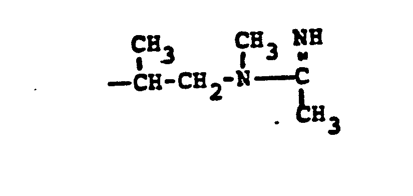

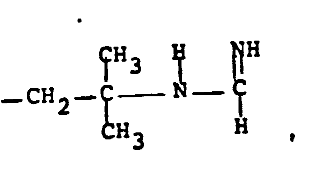

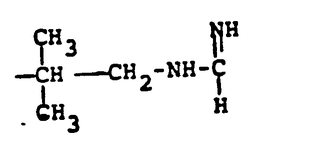

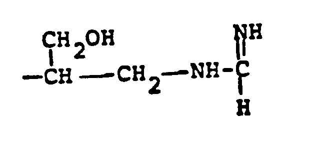

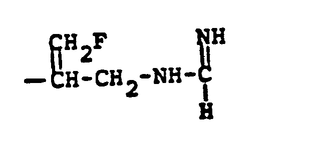

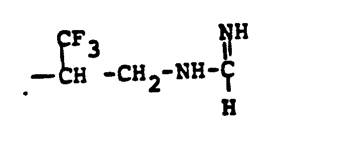

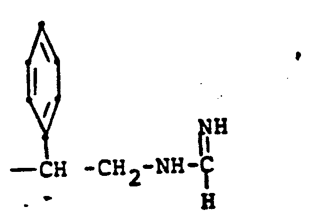

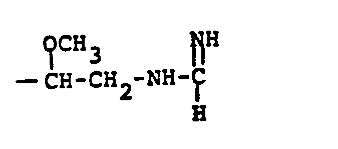

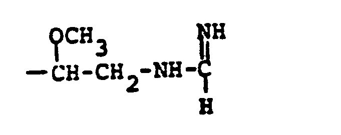

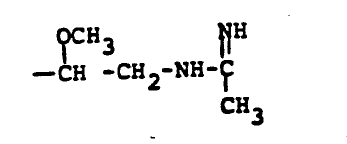

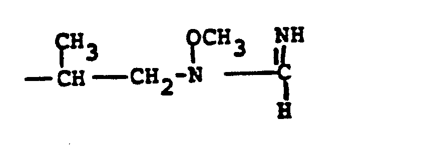

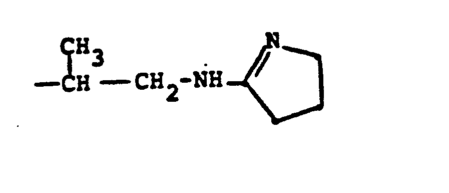

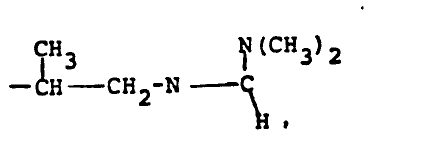

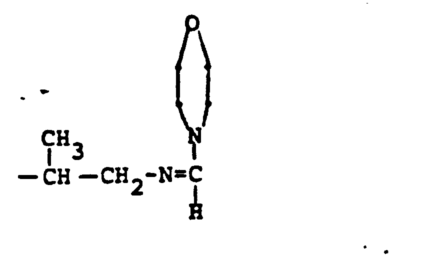

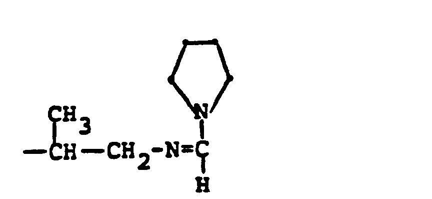

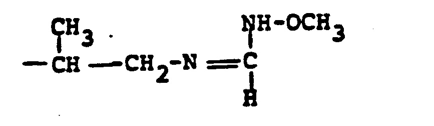

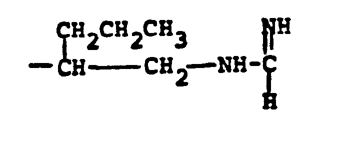

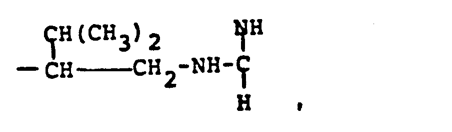

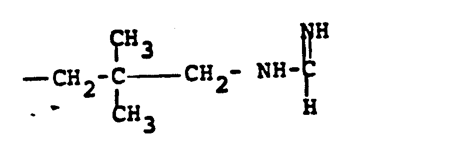

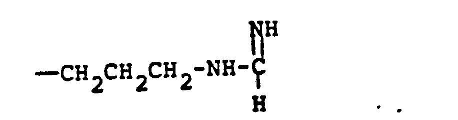

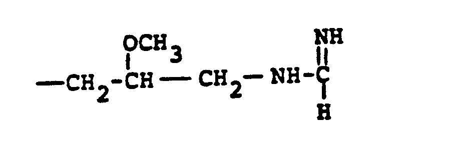

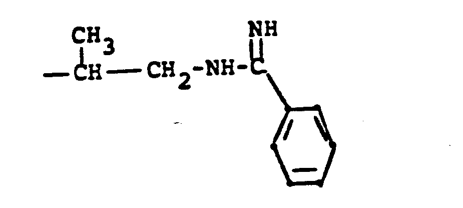

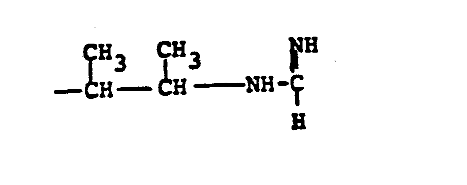

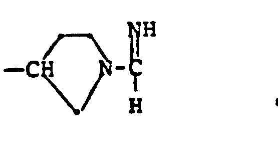

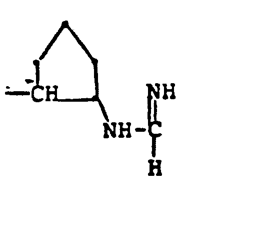

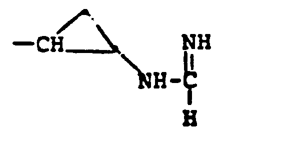

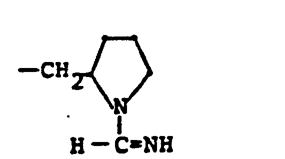

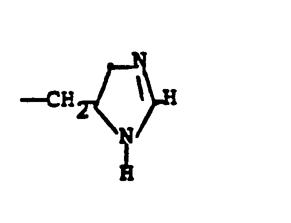

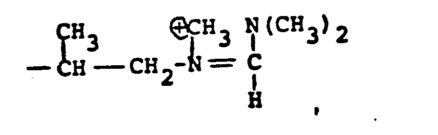

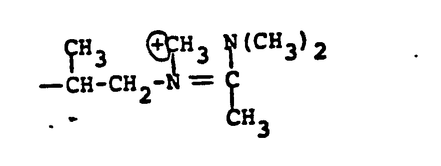

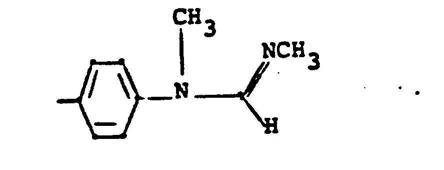

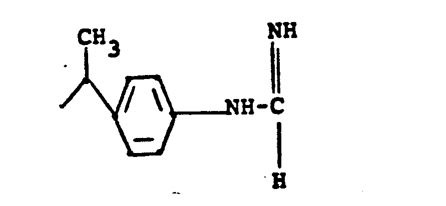

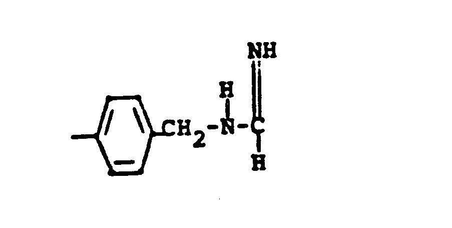

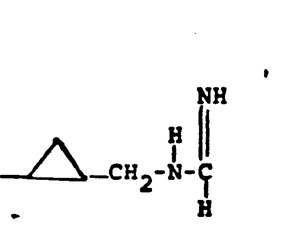

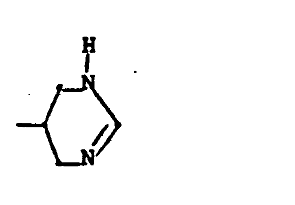

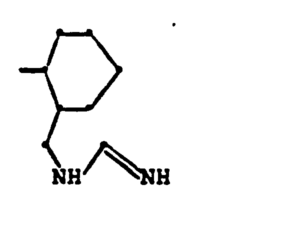

### 11. Guanidino and Guanidinium

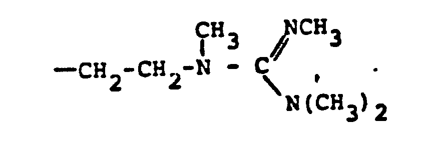

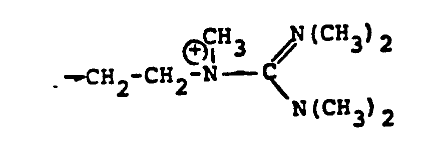

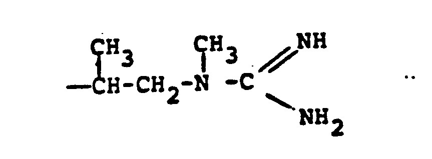

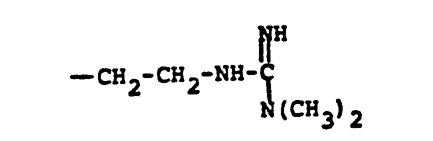

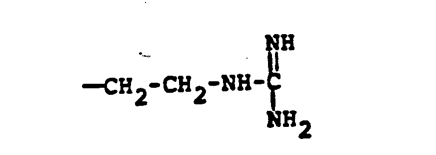

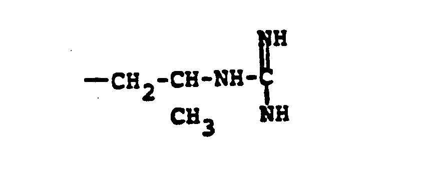

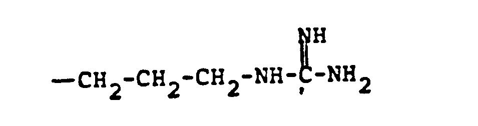

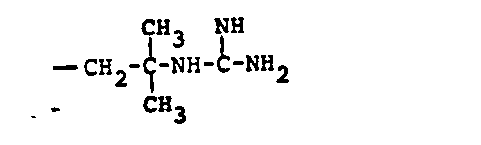

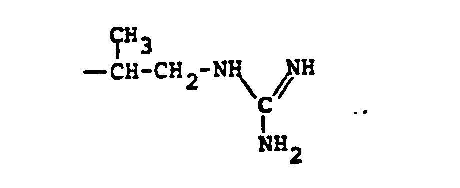

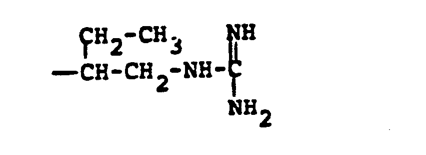

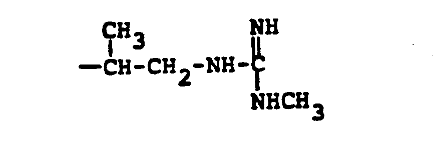

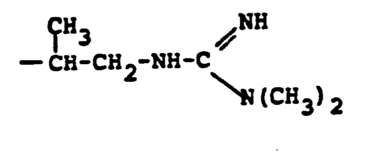

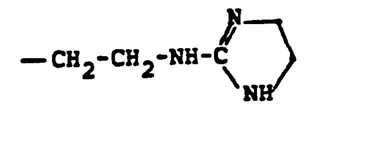

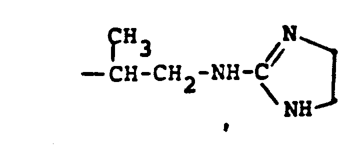

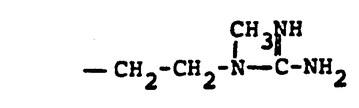

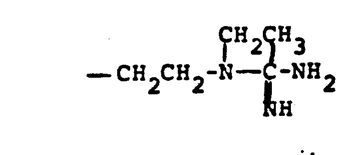

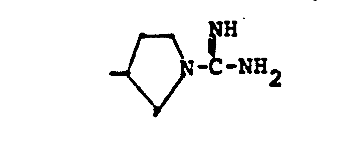

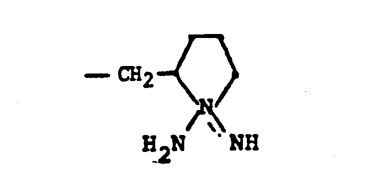

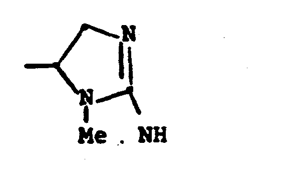

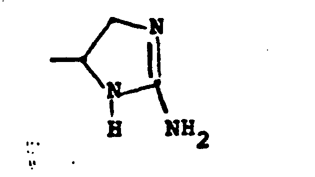

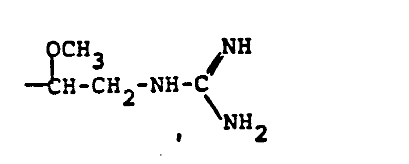

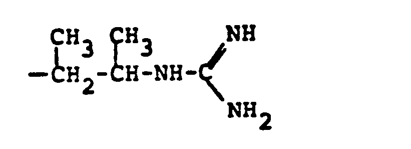

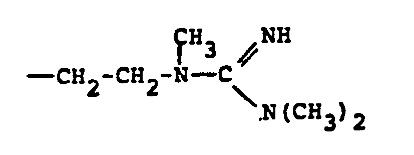

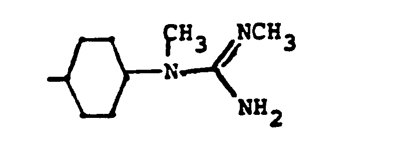

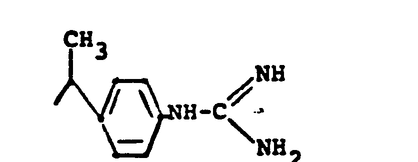

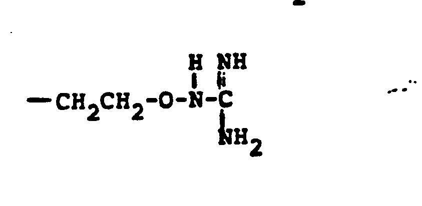

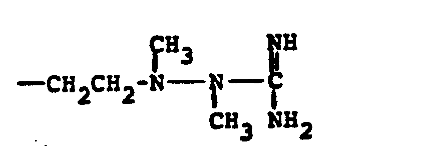

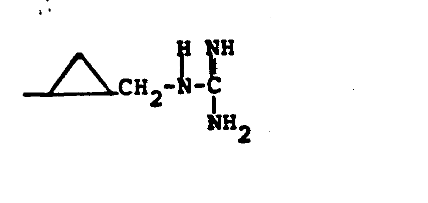

### 12. Carbamimidoyl

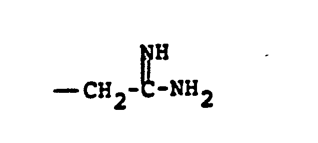

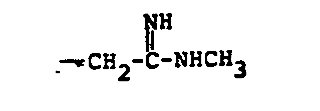

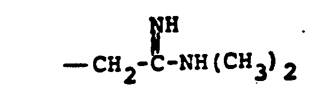

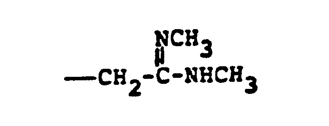

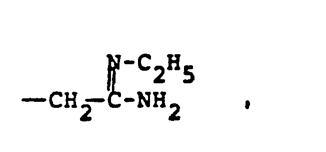

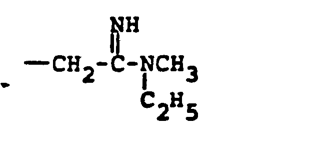

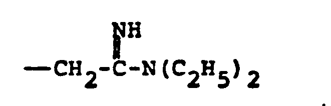

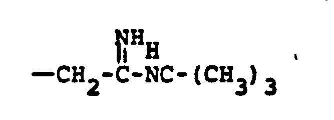

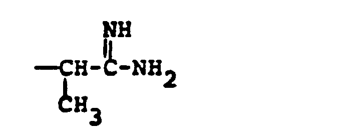

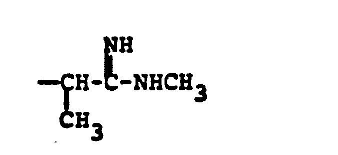

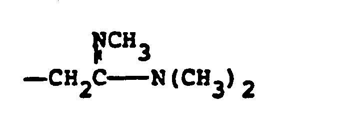

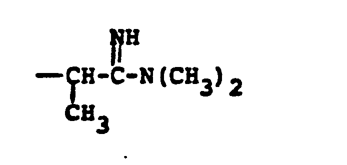

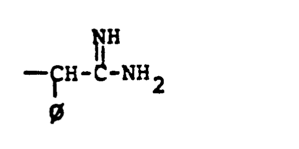

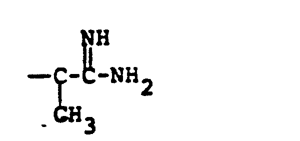

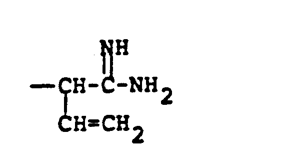

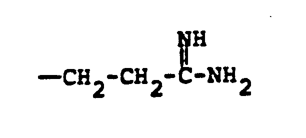

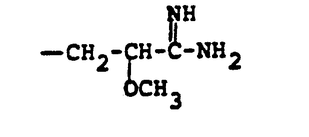

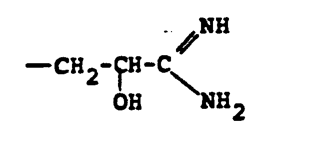

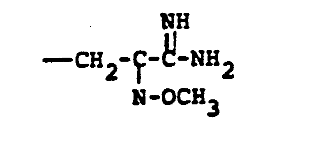

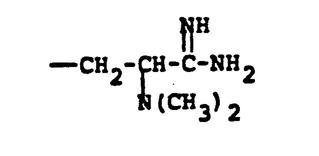

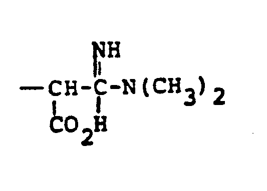

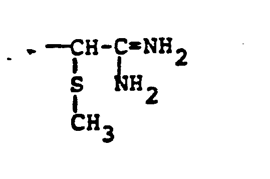

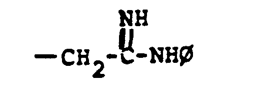

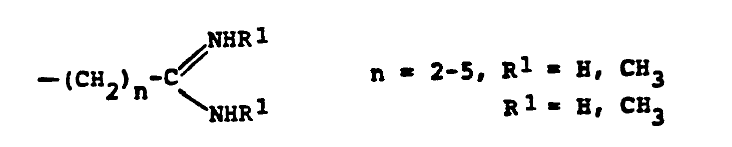

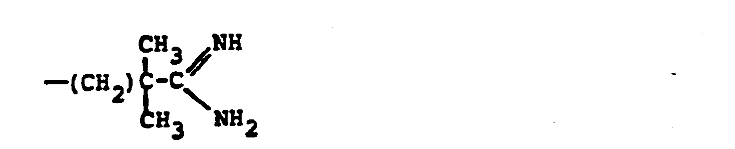

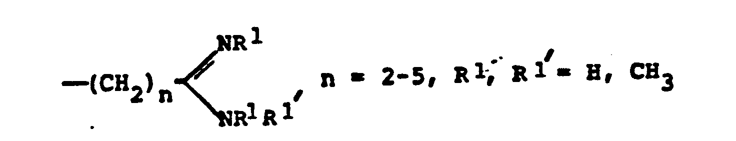

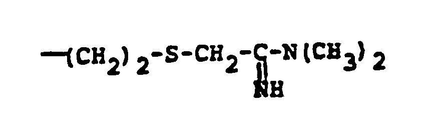

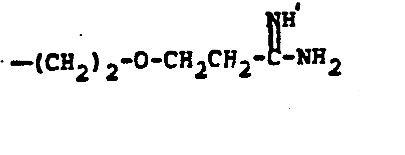

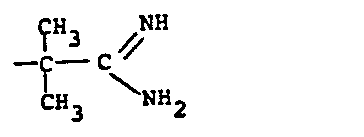

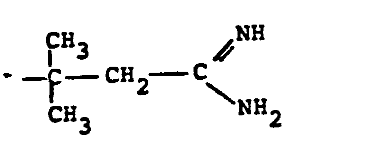

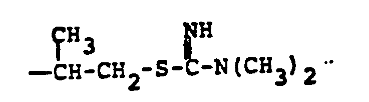

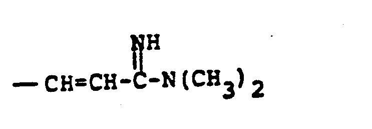

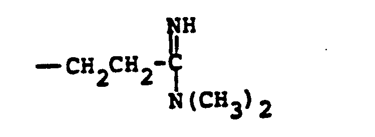

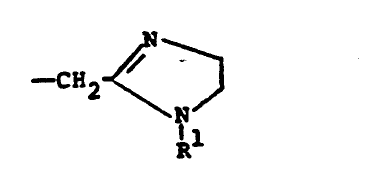

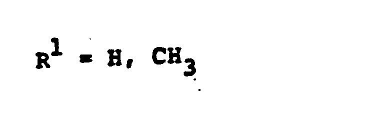

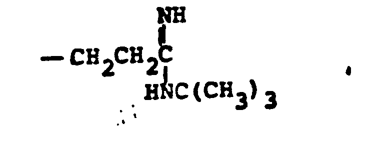

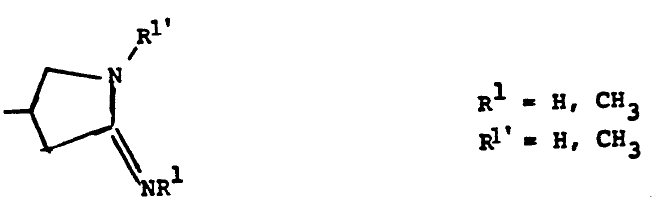

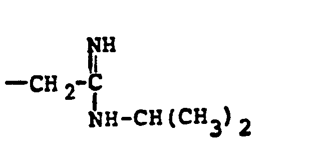

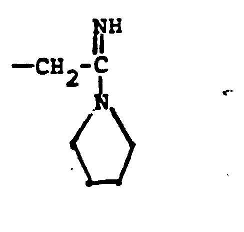

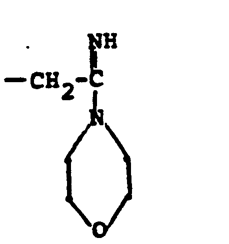

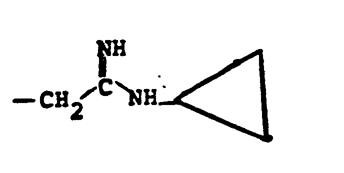

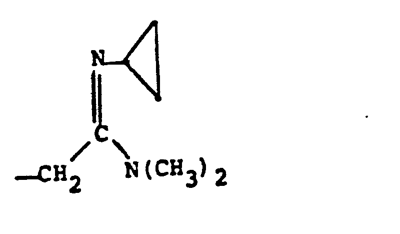

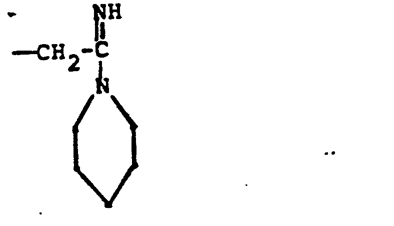

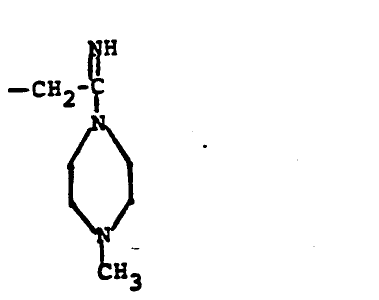

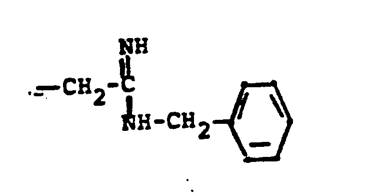

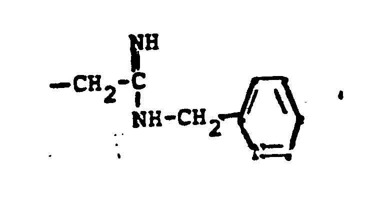

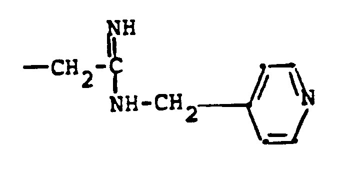

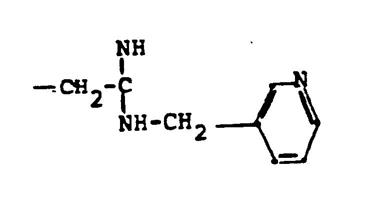

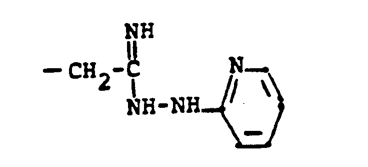

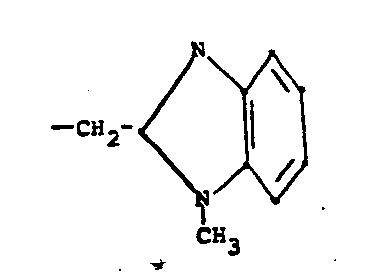

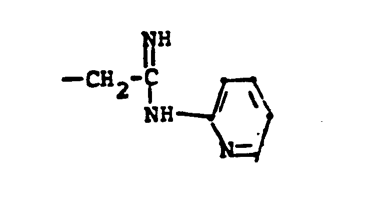

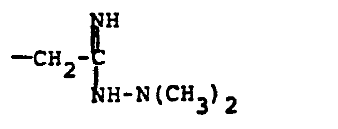

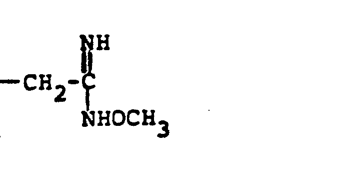

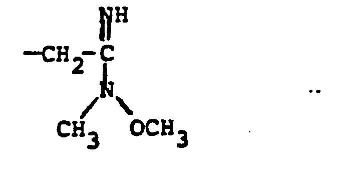

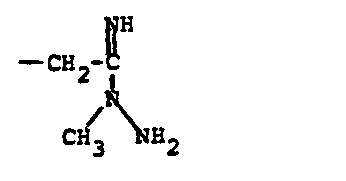

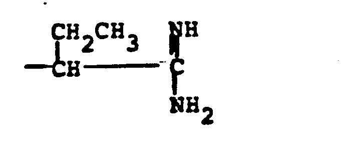

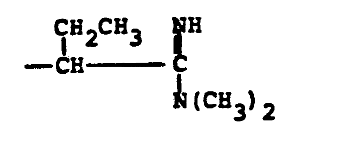

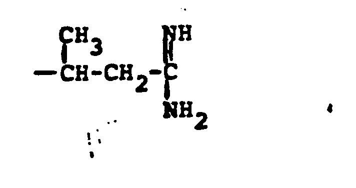

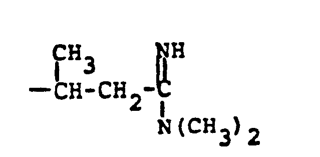

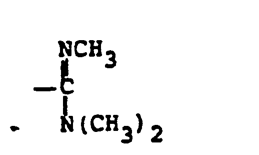

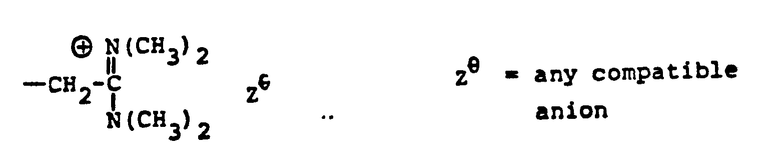

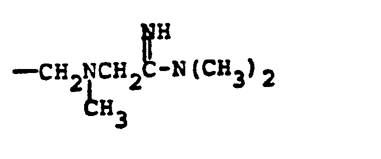

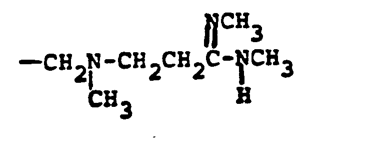

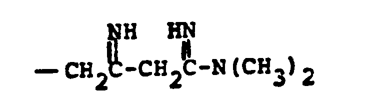

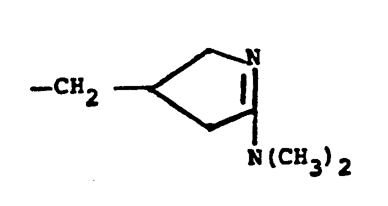

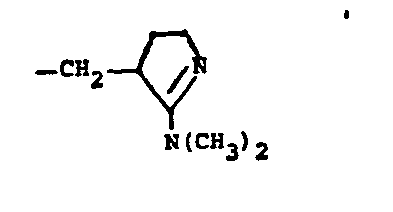

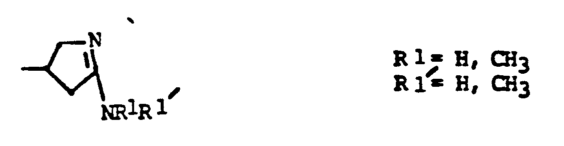

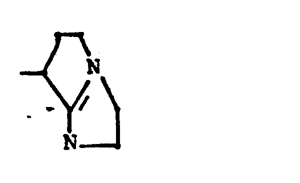

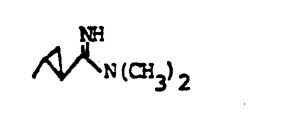

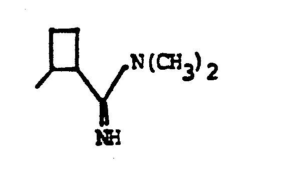

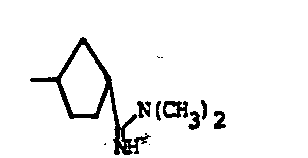

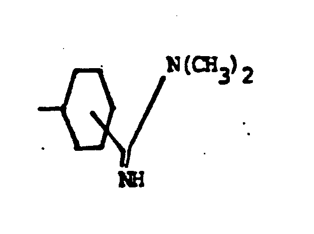

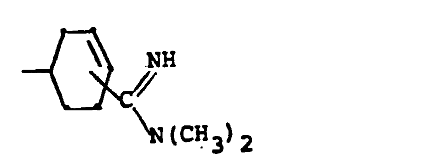

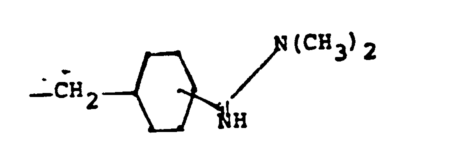

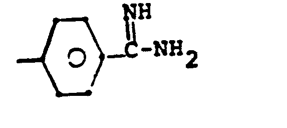

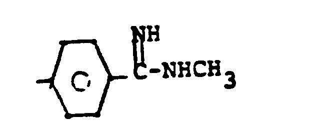

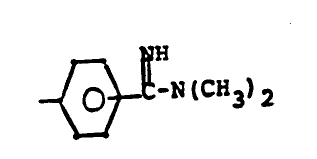

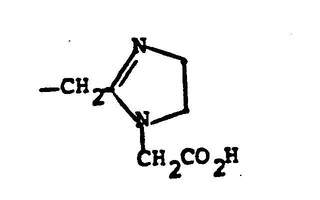

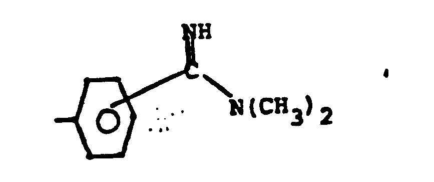

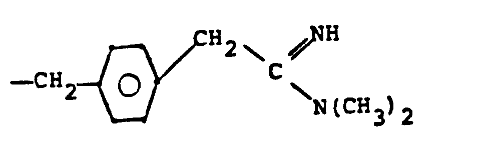

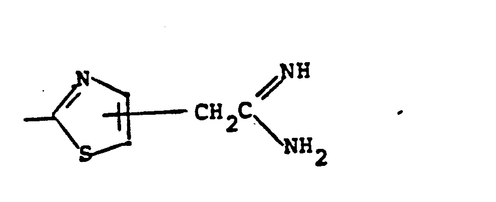

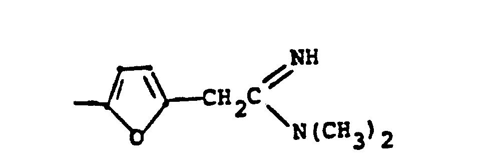

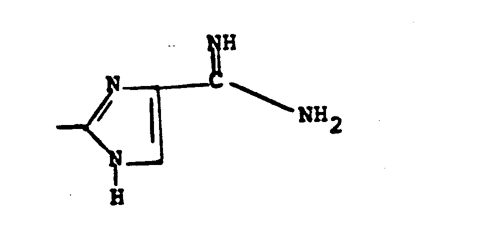

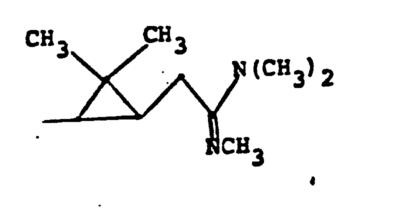

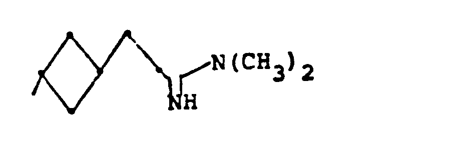

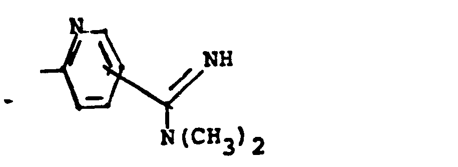

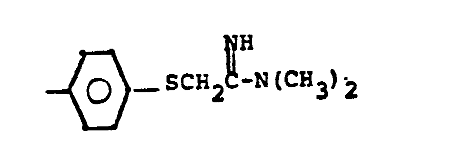

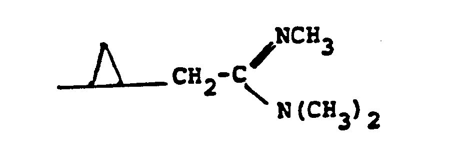

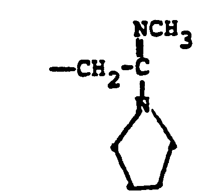

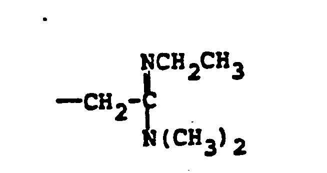

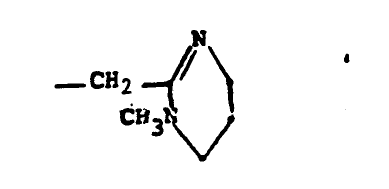

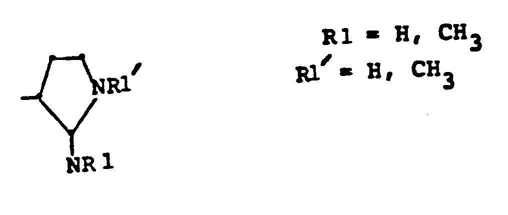

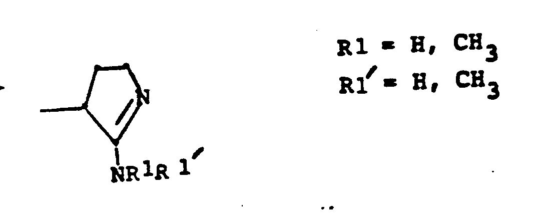

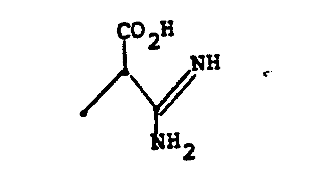

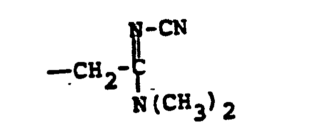

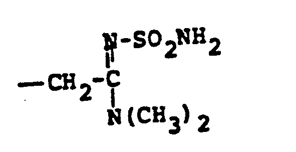

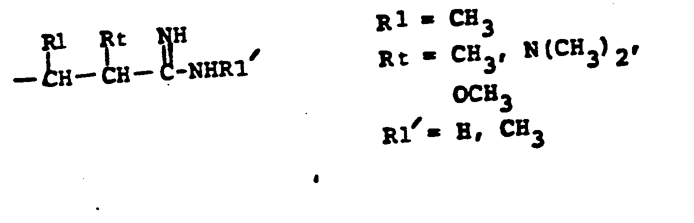

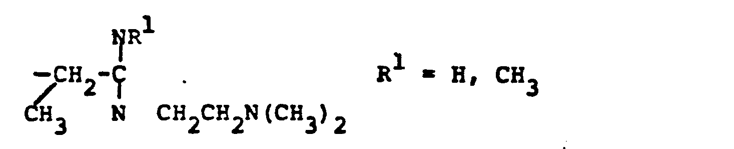

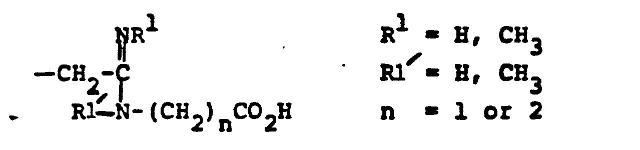

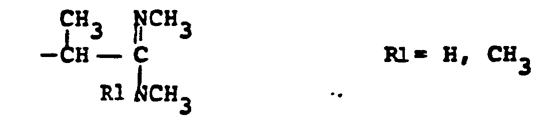

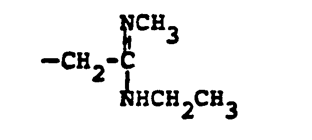

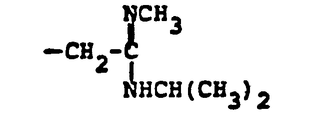

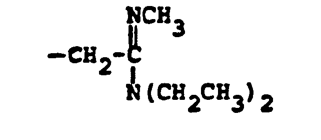

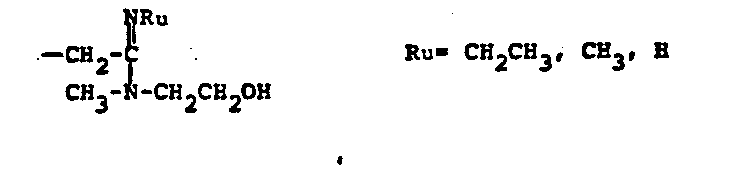

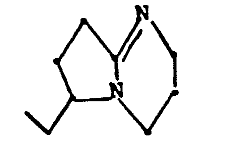

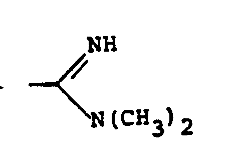

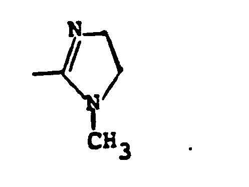

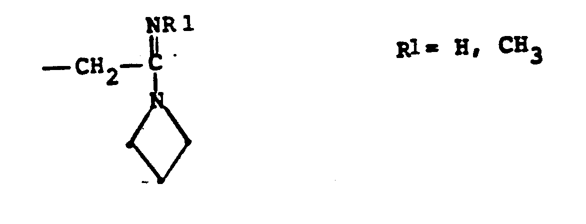

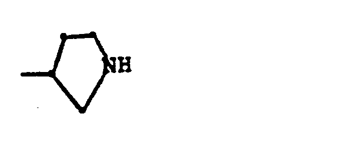

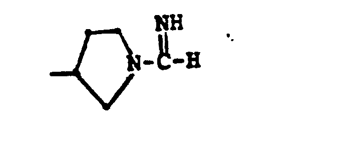

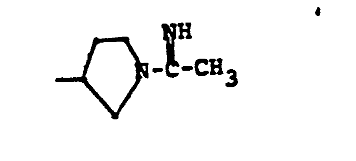

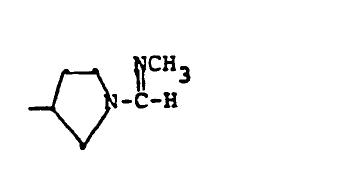

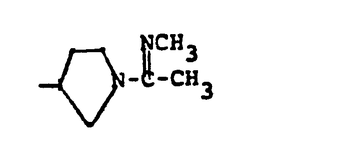

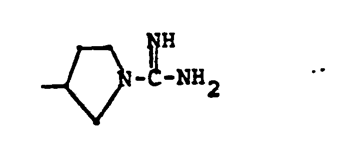

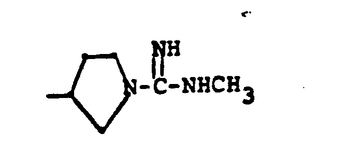

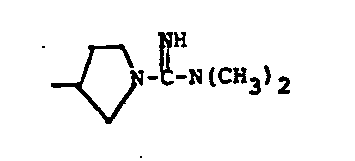

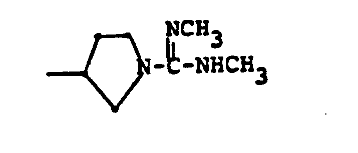

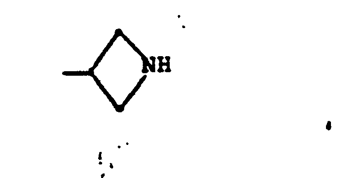

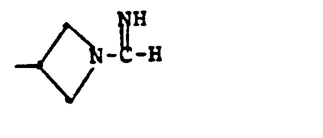

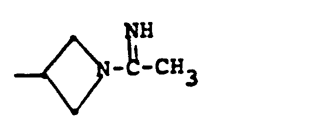

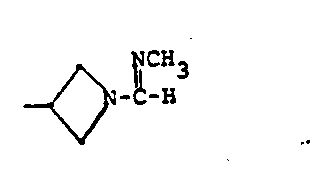

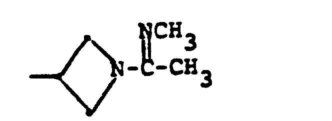

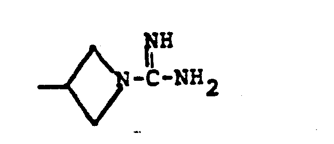

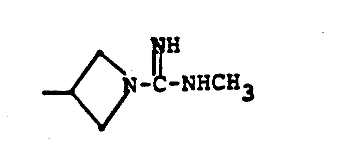

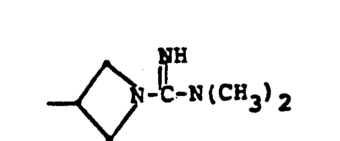

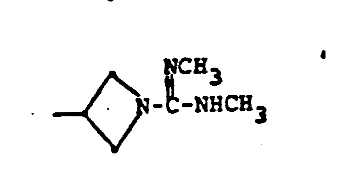

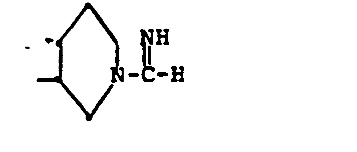

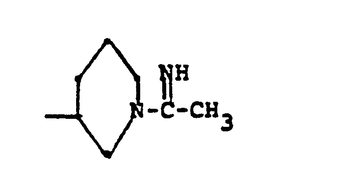

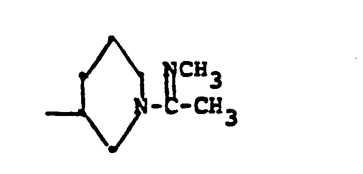

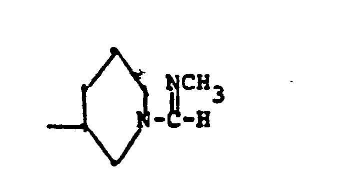

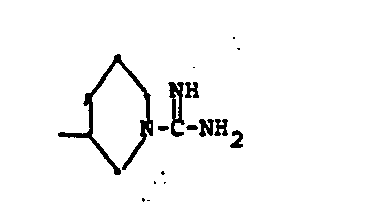

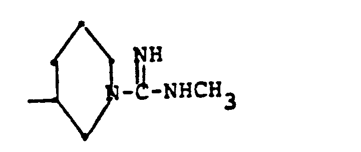

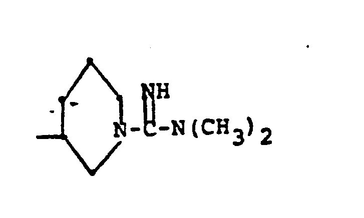

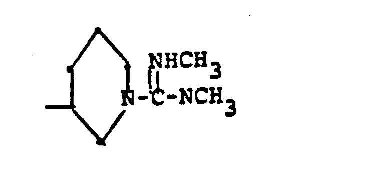

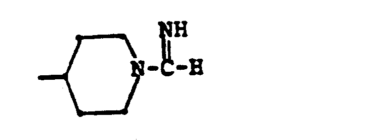

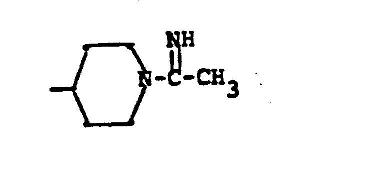

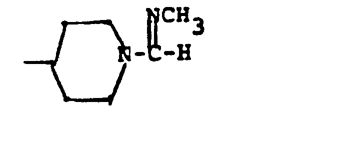

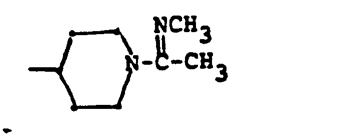

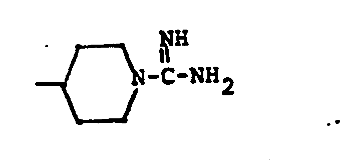

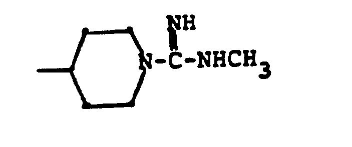

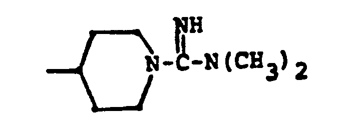

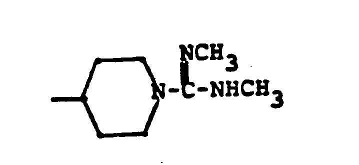

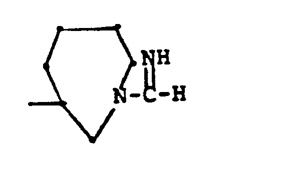

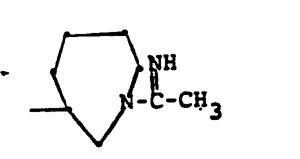

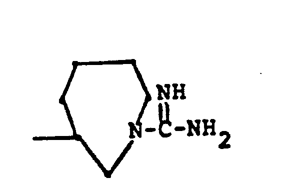

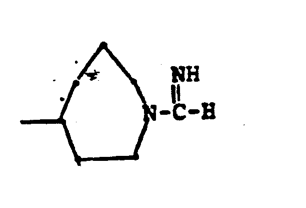

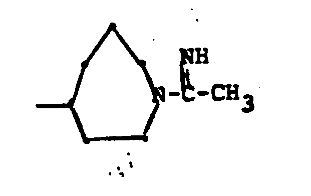

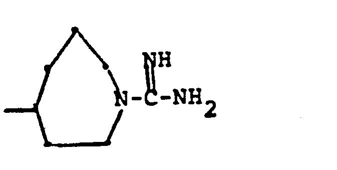

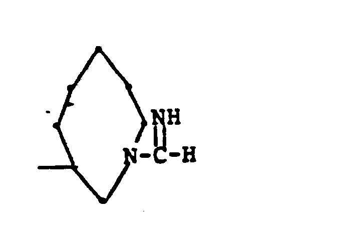

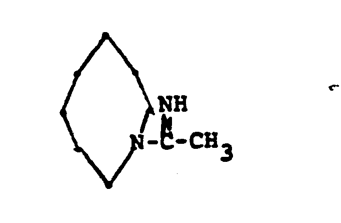

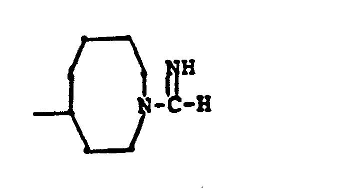

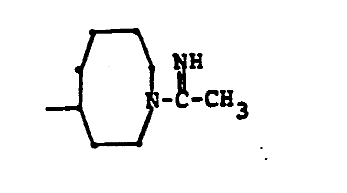

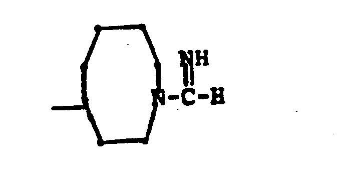

The compounds of Formula I include inner (Zwitterion) salts when W is COO e.g. 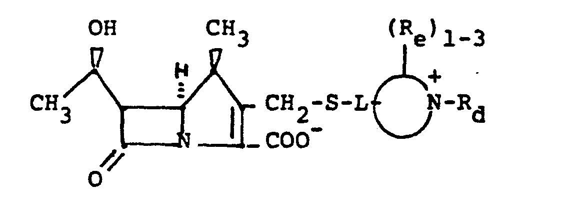
or, when W is other than COO⁻, salts with an external, physiologically acceptable counterion Z⁻ such as Cl⁻, Br⁻, I , OCH⁻₃, OSO₂CF⁻₃, φP(O)(O phenyl)⁻₂ and the like.

The inner salts are preferred.

Again, the compounds of Formula I include the stereoisomers as mixtures and as separate isomers.

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella Pneumoniae, Bacillus subtilis, Salmonella typhosa, Pseudomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the formula I antibiotic is parenteral by i.v. infusion, i.v. bolus, or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d.) three (t.i.d.) or four (q.i.d.) times per day. More specifically, for mild infections, and particularly urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or 1-carbade- thiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102616.4 filed July 24, 1979 (Patent Number 10573): 79102615.6, filed July 24, 1979 (application no. 15573); and No. 82107174.3, filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.) disclose suitable inhibitors, combination compositions and methods of treatment, they are incorporated herein by reference. A preferred weight ratio of I compound:DHP inhibitor in the combination compositions is about 1:1. A preferred DHP inhibitor is 7-(L-2-amino-2-carboxyethylthio)-2-(2,2-dimethyl- cyclopropanecarboxamide)-2-heptenoic acid or a useful salt thereof.

These combination compositions and their use is another embodiment of the present invention.

The compounds of Formula I may be prepared by any convenient process.

### Preparation of Compounds of Formula I

Due to the varied nature of _{R}⁴ and _{R}⁵, several different synthetic approaches are used to prepare compounds of type I. These are summarized below. Implicit in these synthetic routes is the fact that when R contains a functional group which might interfere with the intended course of the reaction, the offending group is blocked. For example, when a basic nitrogen group is present as -NHR or -NH₂, it is usually protected by acylation (-C0₂-allyl or -C0₂PNB) or silylation. In the case of a carboxyl group, protection is achieved via use of a suitable ester, such as allyl or PNB.

In many instances, the actual order of carrying out the individual synthetic transformations in a synthetic scheme can be permuted for reasons of Synthetic expediency. Thus, many of the examples listed below could proceed with equal facility by having the order of operations changed.

### Synthetic Methodology

A. Key Intermediate II: 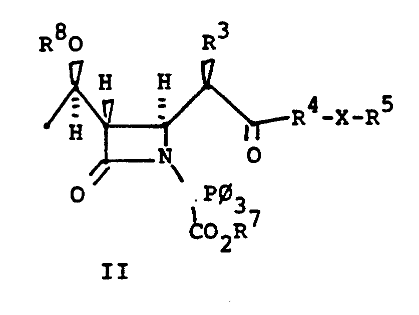
X, R³, R⁴ and R⁵ are as defined above;
R⁷ is a suitable ester protecting group such as p-nitrobenzyl, allyl, or the like;
_{R}⁸ is a suitable alcohol protecting group such as (CH₃)₃Si-, t-BuMe₂Si, p-nitrobenzyloxycarbonyl, allyloxycarbonyl, or the like.

### Method IIa

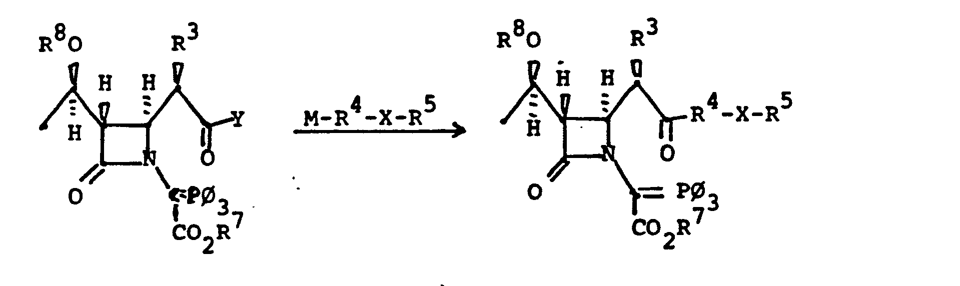

deblock if required II 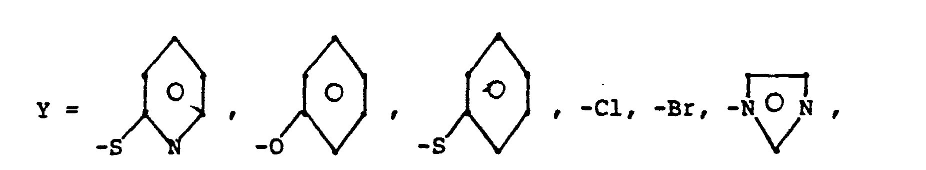
or the like;
_{R}⁵, _{R}³, _{R}⁷, and _{R}⁸ are as defined above;
R⁴=-CH₂-, 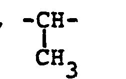
M = Li, MgX, Cu or the like.

### Method IIb

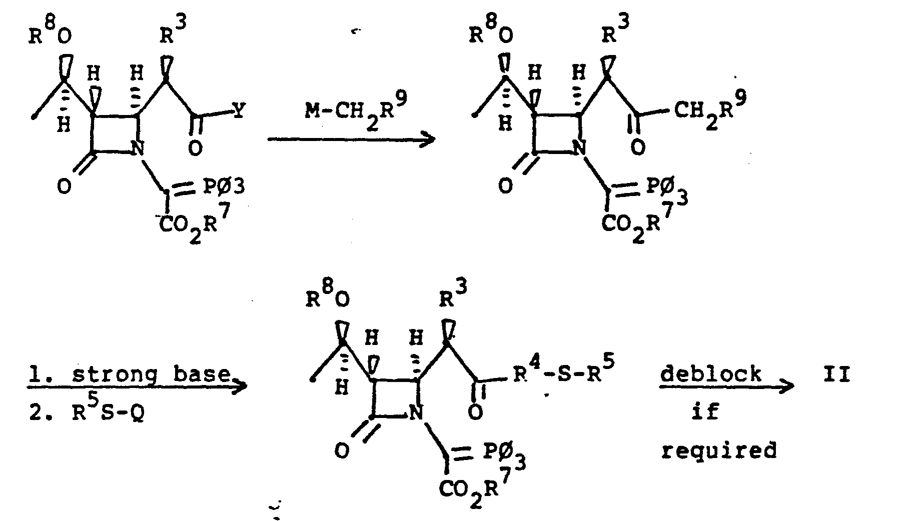

_{Y}, _{M}, and R are as defined above;
R⁸ = triorganosilyl, allyloxycarbonyl, or the like;
R⁷ = allyl, benzyl or the like;
Q = -Cl, -Br, -SR, -SO₂R or the like;
R⁹ = H, CH₃;
R⁴ = -CH₂-, 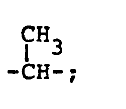
strong base = LiNiPr₂, LiN(TMS)₂, 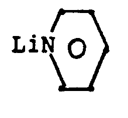 etc.

### Method IIc

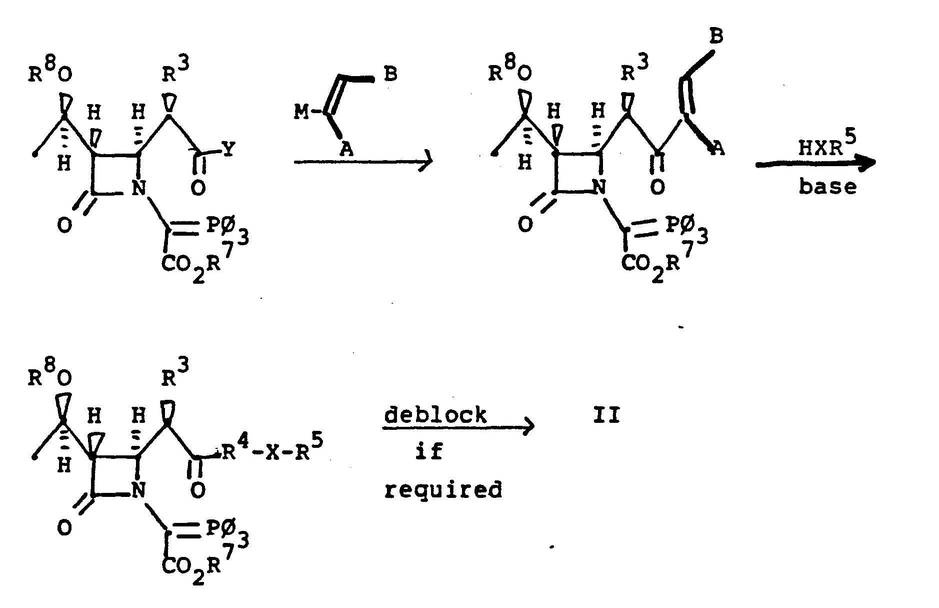

_{X}, _{M}, Y, _{R}³, and R⁵ are as defined above;
R⁸ = triorganosilyl, allyloxycarbonyl or the like;
R⁷ = allyl, benzyl or the like;
A, B = H or CH₃ (both not CH₃); 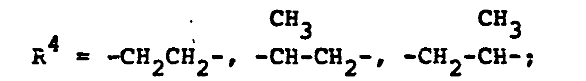
base = Et₃N, iPr₂NEt, NaH, n-BuLi, LiNiPr₂ or the like.

### Method IId

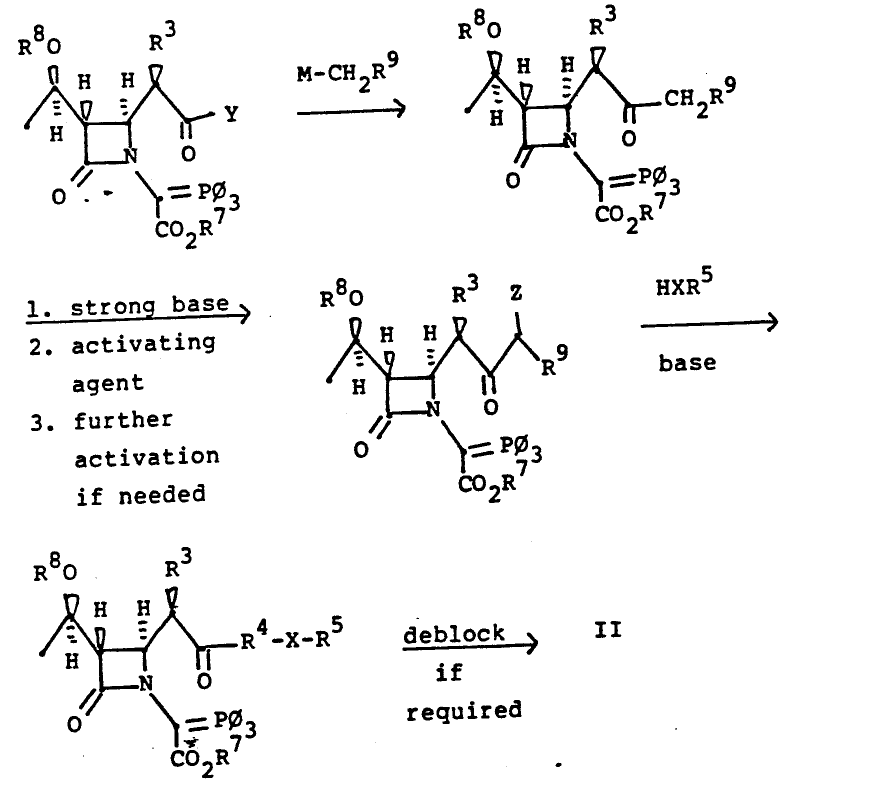

X, M, Y, R³ and R⁵ are as defined above; R⁷ = allyl, benzyl or the like;
R⁸ = triorganosilyl, allyloxycarbonyl, or the like;
R⁴ = -CH₂-, 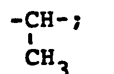
strong base is as defined previously; R⁹ = H, CH₃;
_{Z =} OSO₂CH₃, 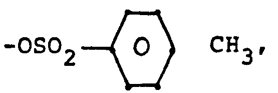 -Cl, Br, I or the like;
activating agent = O₂, MoOPH, Br₂, I₂, BrSO₂Ar etc.
The -OH product of reaction with 0₂ or MoOPH can be further activated with ClSO₂CH₃, 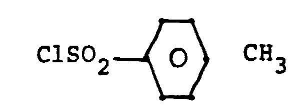 etc.

### Method IIe

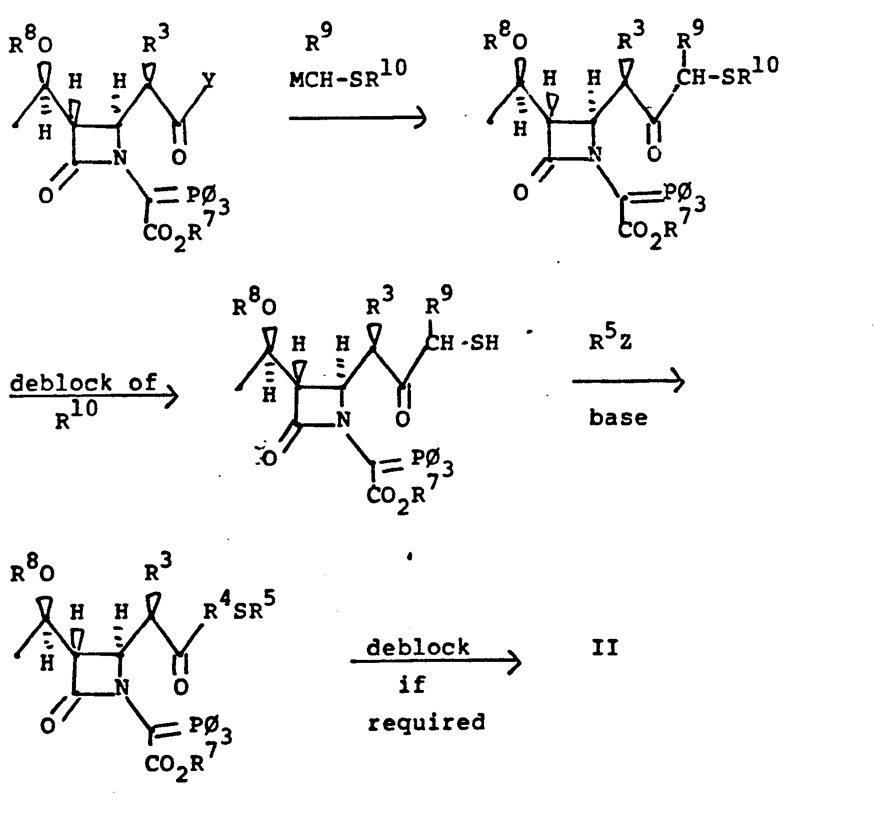

_{Z}, _{R}³, _{R}⁵, _{M} and Y are as defined previously;
R⁷ = allyl, benzyl or the like;
_{R}⁸ = triorganosilyl, allyloxycarbonyl or the like;
R⁴ = -CH₂-, -C^{H-}; ^{C}H₃
R¹⁰ = -Cφ₃, -tetrahydropyranyl or the like;
R⁹ = H, C^{H}₃^{:}
- in addition, Z can be an activated olefin or alkyne 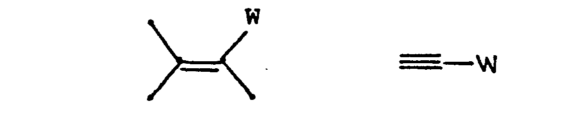
   W = electron withdrawing group such as 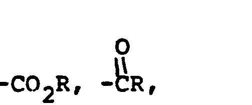 -CN, -SO₂R, etc.

Variations of the above methodologies involving permutation of steps is also possible. For example, the phosphorane group on nitrogen can be introduced at a later stage by "standard" procedures (see below). 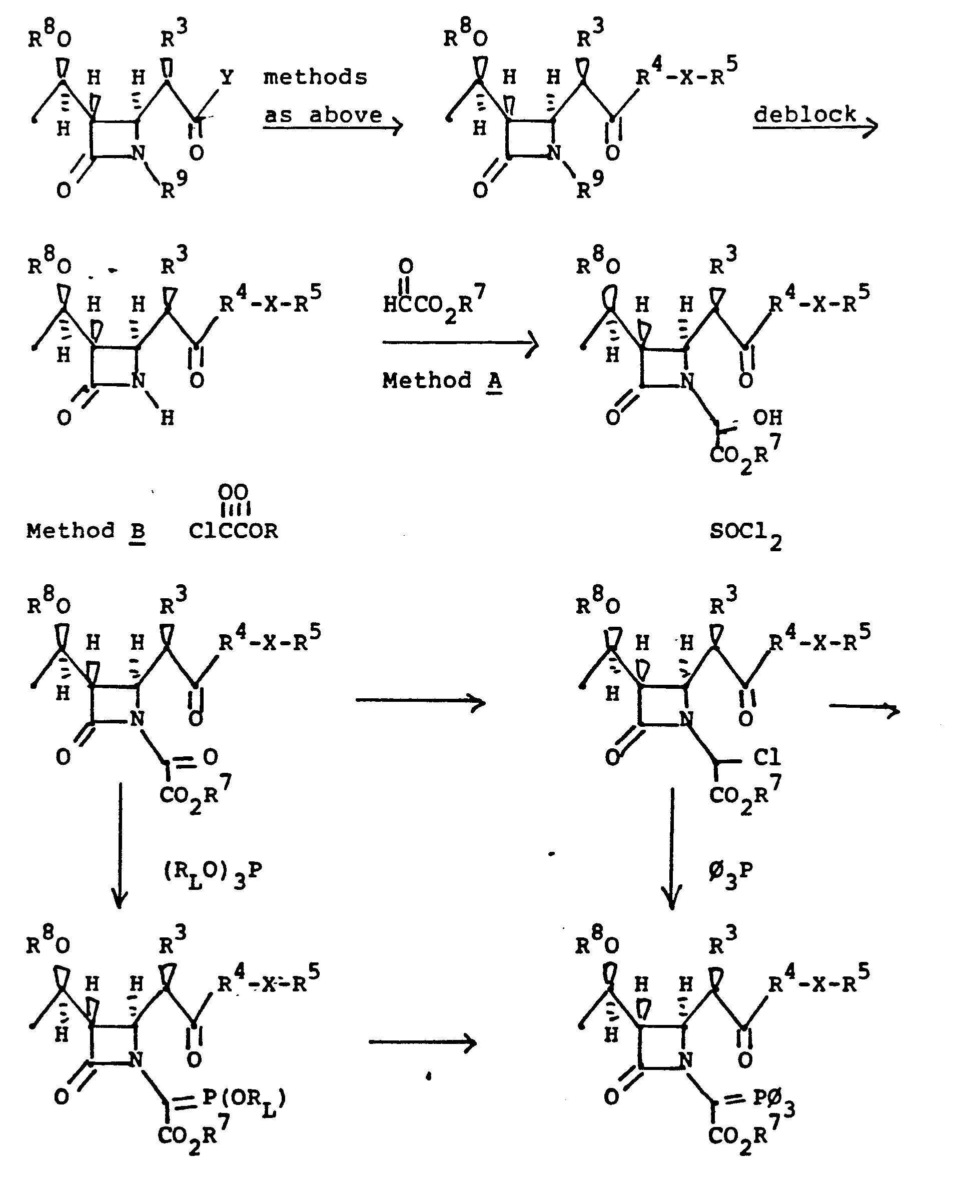
L=CH₃, CH₂CH₃, etc.

One example of such a route is shown in Method IIf.

### Method IIf

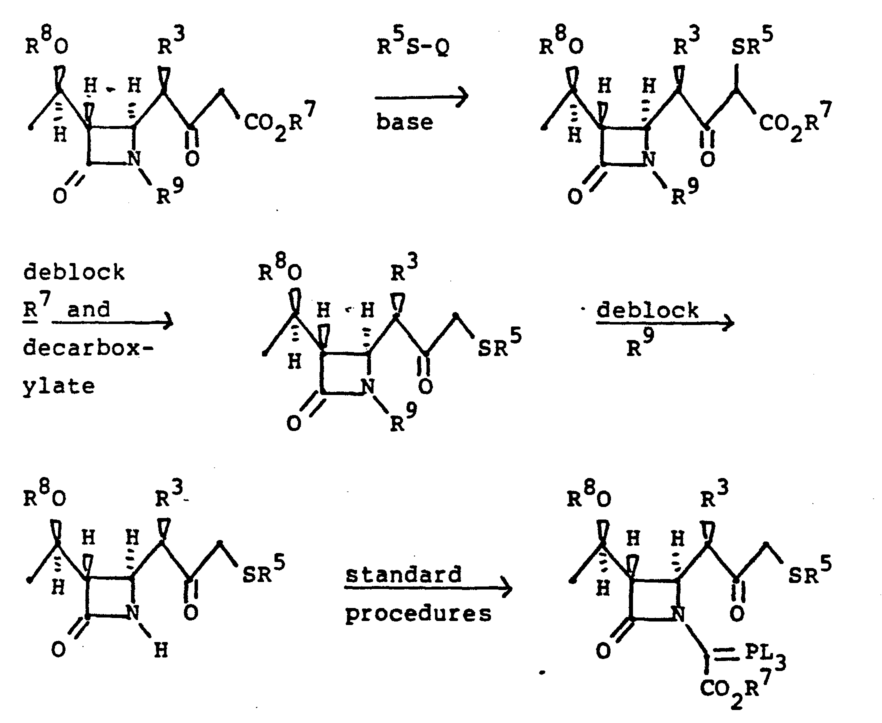

### L=φ, OEt, etc.

### Method IIg

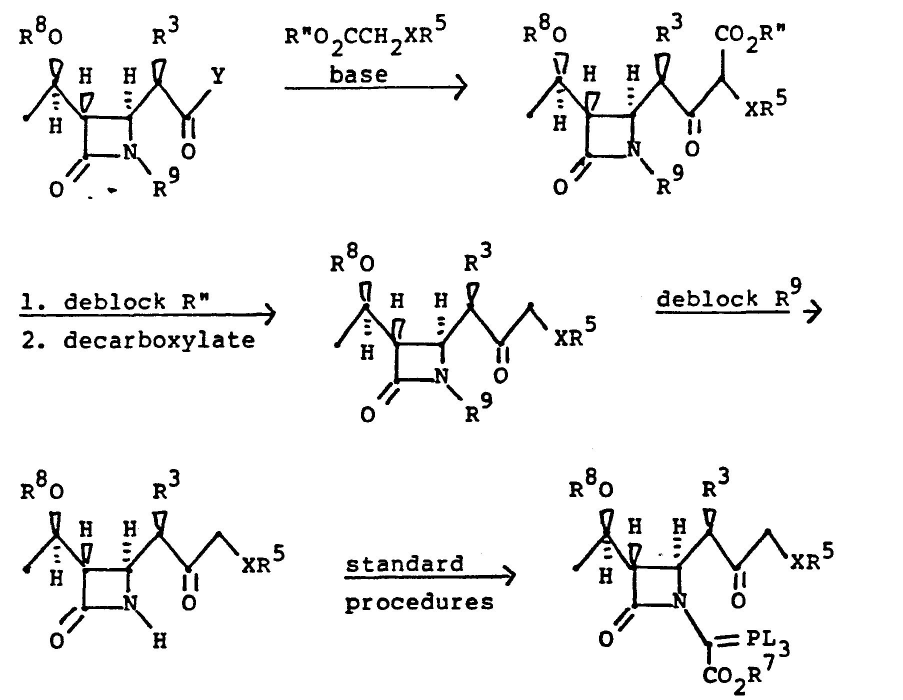

_{Y}, _{R}³, _{R}⁸, _{R}⁹ are as previously defined; R" = t-Bu, R₃Si such as Me₃Si, t-BuMe₂Si; 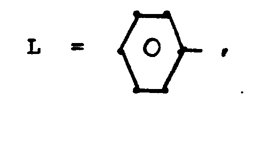
-OR such as OEt, OMe, OiPr, etc.

### Method IIh

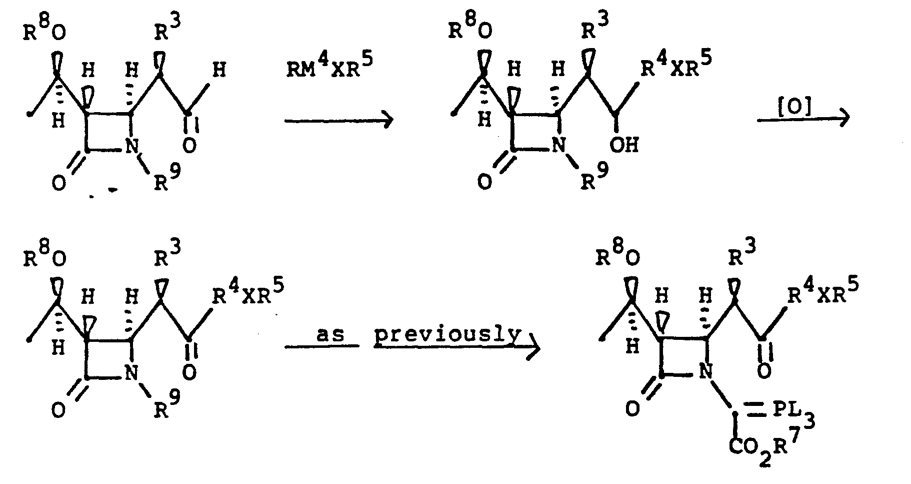

- all substituents as previously defined
Method IIi

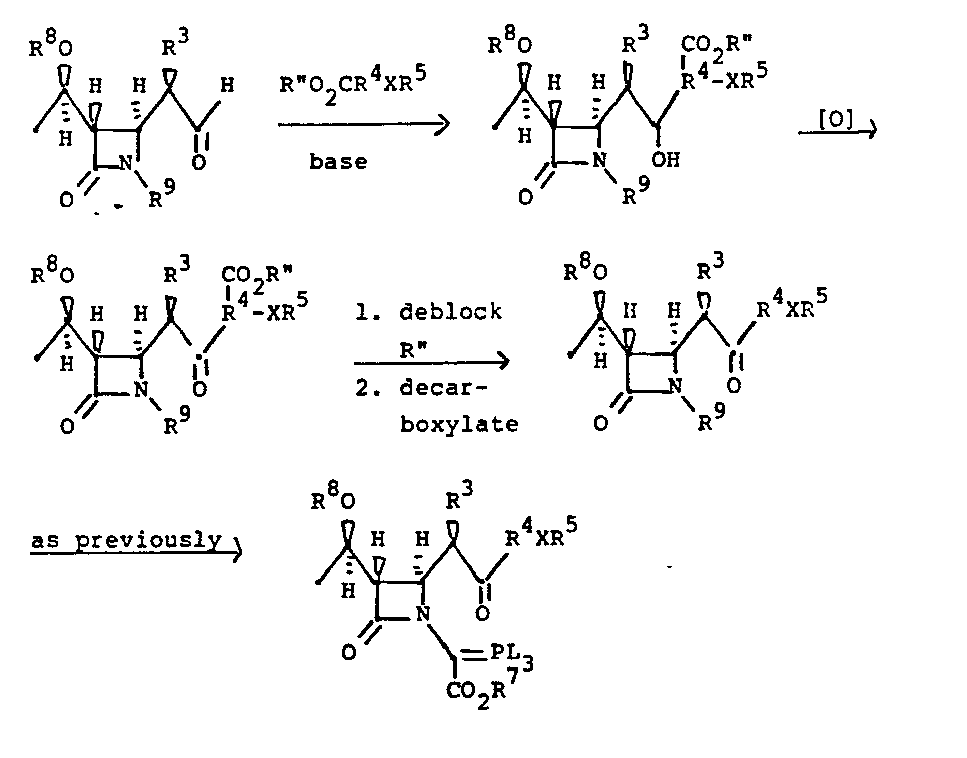

Completion of the Synthesis from Intermediate II - Methods a-i

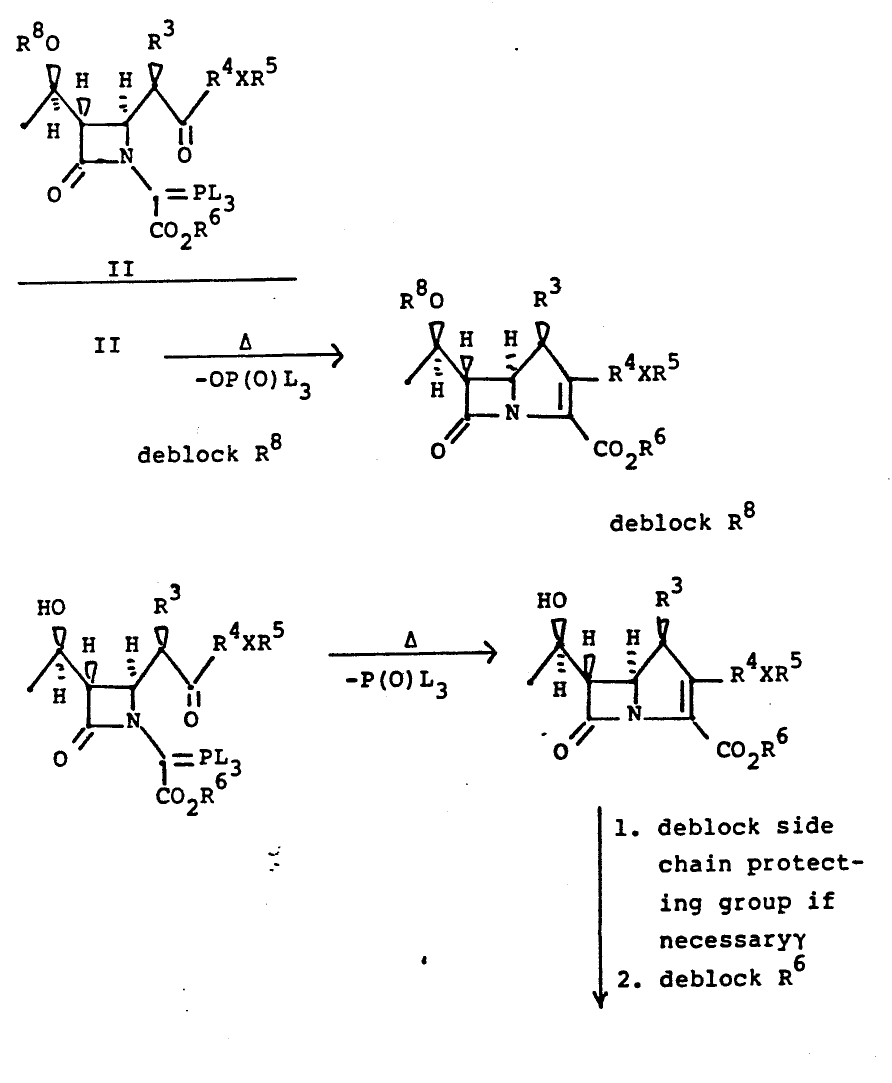

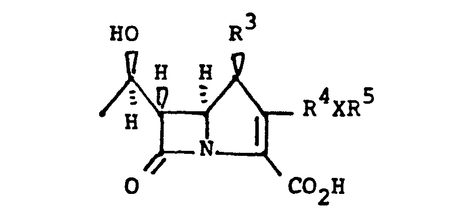

_{Y} In those cases where the final product will contain a quaternized heterocyclic group, an additional alkylation step would be added.

### Method j

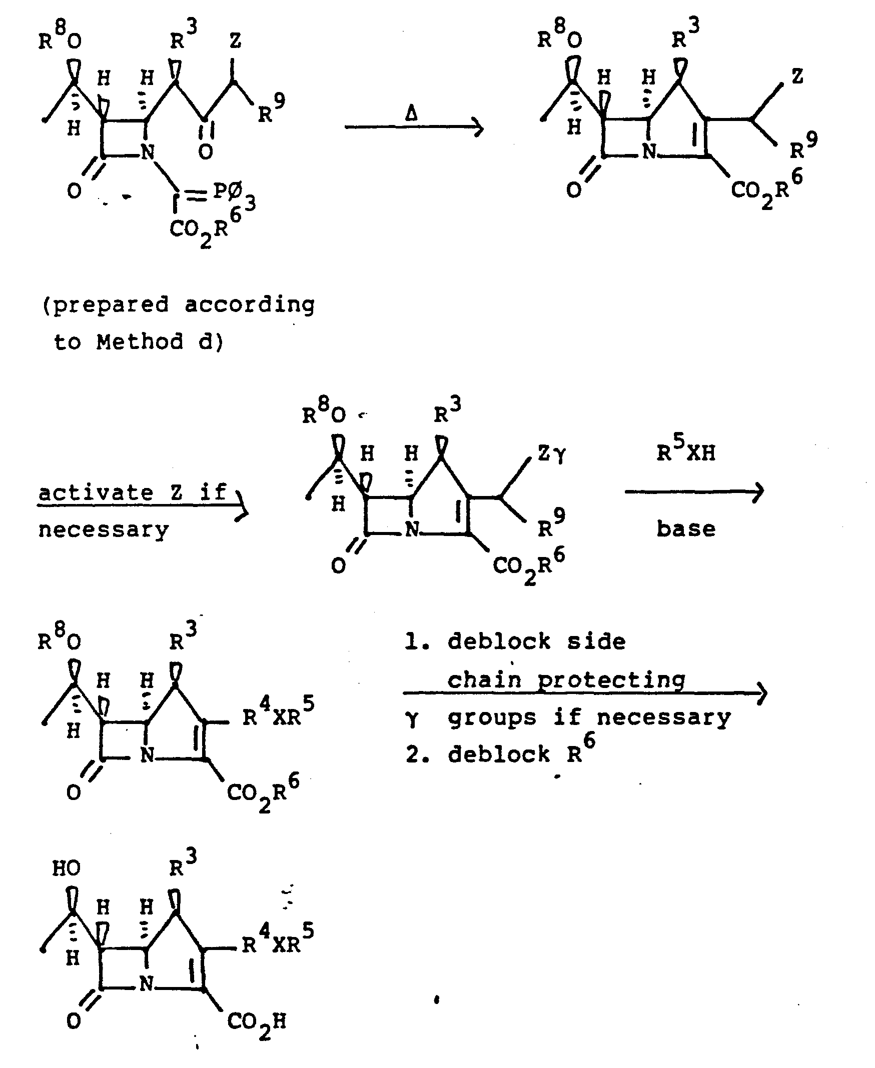

Y In those cases where the final product will contain a quaternized heterocyclic group, an additional alkylation step would be added.

The following examples illustrate the preparation of compounds of Formula I. The temperature is in degrees Celsius unless otherwise indicated.

### EXAMPLE 1

Preparation of (1R,5S,6S,8R)-2-[[(1-methyl-1H-tetrazol-5-yl]-thio]-methyl]-6-(hydroxyethyl)-1-methylcarbapen-2-em-3-carboxylic acid, potassium salt, 6

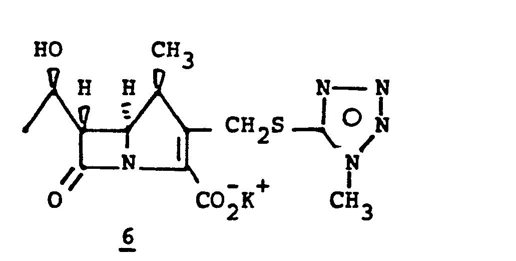

Step A:

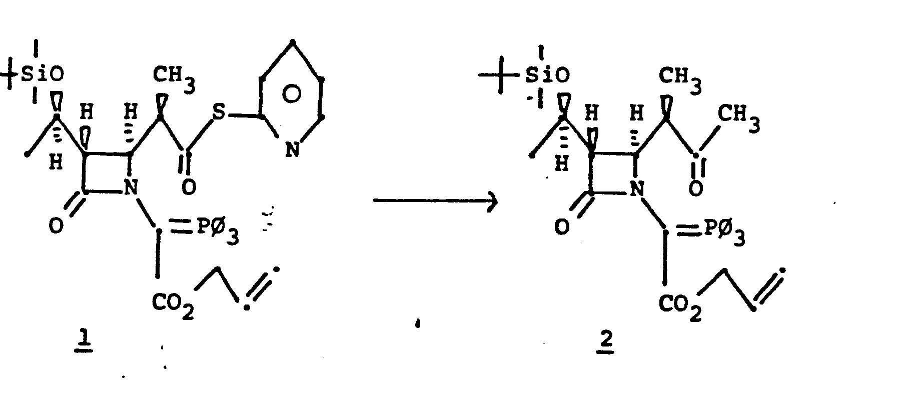

A commercial sample of 3M methylmagnesium bromide in ether (690 ul, 2.07 mmol) is added dropwise to a stirred solution of thiopyridyl ester 1 (1.06 g, 1.41 mmol) in anhydrous tetrahydrofuran (25 ml) at -78° under nitrogen. After 5 minutes, work-up of a small aliquot shows on TLC the less polar product as well as residual starting material. An additional amount of methyl magnesium bromide (140 µl, 0.42 mmol) is added, and stirring is continued at -78° for 10 minutes. The reaction mixture is then added to a saturated NH₄Cl solution (35 ml), H₂0 (10 ml), and ethyl acetate (40 ml). After phase separation, the aqueous layer is extracted with an additional amount of ethyl acetate. The combined organic layers are washed with cold 1N HC1 (35 ml), cold 10% NaHCO₃ (35 ml), and then brine (35 ml). After drying over MgSO₄, the organic layer is concentrated in vacuo to a yellow foam. Chromatography on Baker's silica gel (eluting with 0 10% ethyl acetate in methylene chloride) affords ca. 90% pure methyl ketone (800 mg). Preparative thin layer chromatography on silica gel (eluting with 1:1 ethyl acetate-hexane and extracting with 1:1 ethyl acetate-methylene chloride) provides pure methyl ketone 2 (650 mg, 70% yield).
NMR (CDC1₃) selected absorbances: 8.0-7.4 (aromatic protons), 6.0 (m, -CH₂CH=CH₂), 2.2 .0 (-C-CH3), 0.8 ( C(CH3)3) in ppm downfield from TMS.
IR (CH₂C1₂ solution) 1750, 1720, 1650, 1625 cm⁻¹. MS (FAB) 658 (M+1).

### Step B:

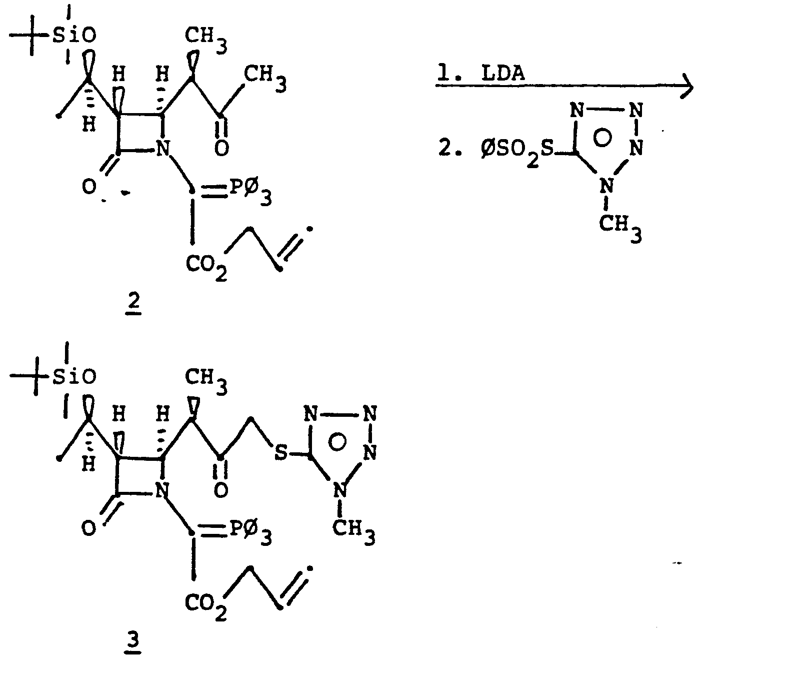

To a solution of diisopropylamine (22 µl, 0.16 mmol) in anhydrous tetrahydrofuran (1.5 ml) at 0° under nitrogen, 1.55 M BuLi in hexane (100 µl, 0.16 mmol) is added with stirring. After 15 minutes, 1 ml of the above LDA solution (0.1 mmol) is added all at once to a solution of methyl ketone 2 (32 mg, 0.049 mmol) in tetrahydrofuran (500 µl) at -78°. A solution of the tetrazolthiosulfonate (12 mg, 0.047 mmol) in tetrahydrofuran (500 µl) is immediately added to the orange-red enolate solution. The temperature is raised to 0° for 1 hour. The reaction mixture is added to a saturated NH₄Cl solution, H₂0, and ethyl acetate. After phase separation, the aqueous layer is again extracted with ethyl acetate. The combined organic layers are washed with brine, dried over MgSO₄, and concentrated to give the crude sulfinylated product (38 mg). Preparative thin layer chromatography on silica gel (eluting with 1:1 ethyl acetate-hexane and extracting with 1:1 ethyl acetate-methylene chloride) provides the tetrazole compound 3 (25 mg, 67% yield).

NMR (CDC1₃) selected absorbances: 7.98-7.40 (aromatic protons), 5.95 (-CH₂CH=CH₂), 3.97 (N-CH₃), 0.79 (C(CH3)3) in ^{pp}m downfield from TMS.

IR (CH₂Cl₂ solution) 1750, 1620, 1610 (sh) cm⁻¹. _{MS} (FAB) 772 (M+l), 262 (φ₃P).

### Step C:

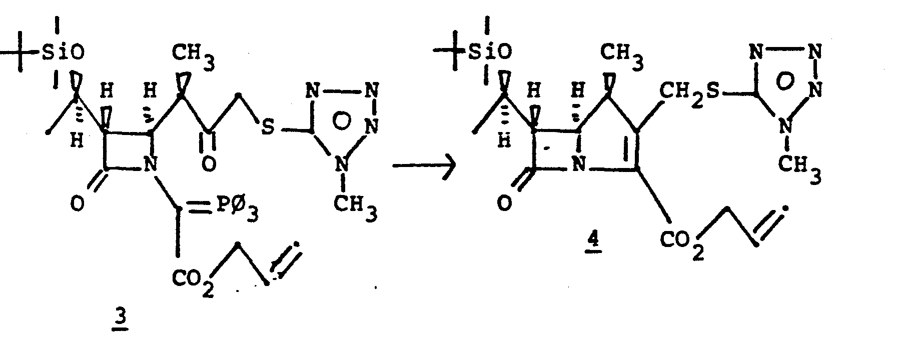

A solution of 3 (25 mg, 0.032 mmol) in benzene (1.7'ml) is heated at reflux under nitrogen for two hours and then concentrated in vacuo to a colorless oil. Preparative thin layer chromatography on silica gel (eluting with 1:1 ethyl acetate-hexane and extracting with 1:1 ethyl acetate-methylene chloride) provides the carbapenem 4 (13 mg, 80% yield).

NMR (CDC1₃) 5.98 (m, -CH₂CH=CH₂), 5.38 (m, -CH₂CH=CH₂), 4,87 (d, J=14Hz, 1 proton of AB pattern for -CH₂S-), 4.77 (m, -CH₂CH=CH₂), 4.21 (m, H₅+H₈), 4.16 (d, J=14Hz, 1 proton of AB pattern for -CH₂S-), 3.95 (s, N-CH₃), 3.31 (m, H₁), 3.27 (dd, J_{5,6}=3Hz, J_{6,8}=6Hz, H₆), 1.24 (d overlapping, 1-CH₃ and CH₃CHO-), 0.87 (s, C(CH₃)₃), 0.07 (s, -Si(CH₃)₂) in ppm downfield from TMS. IR (CH₂C1₂ solution) 1790, 1715 cm⁻¹. UV (dioxane) max = 295 nm. MS (FAB) 494 (M+1).

### Step D:

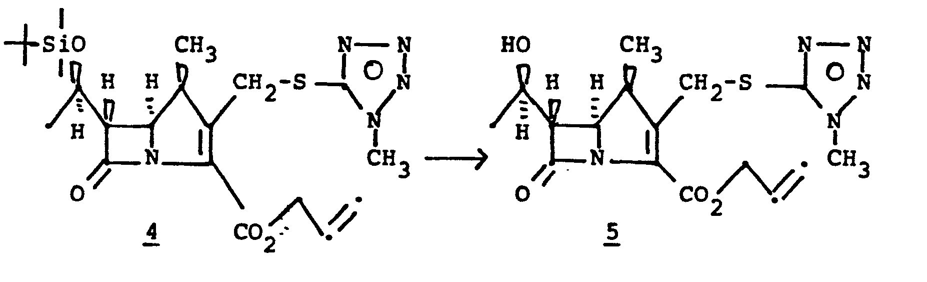

A solution of carbapenem 4 (31 mg, 0.063 mmol), acetic acid (54 µl, 0.94 mmol) and 1M tetrabutylammonium fluoride in tetrahydrofuran (386 µl, 0.39 mmol) is stirred at ambient temperature for thirty hours under nitrogen. The reaction mixture is then added to 0.5M pH 7 phosphate buffer (8 ml), H₂0 (8 ml), and ethyl acetate. Upon shaking a slight emulsion results which is broken by the addition of brine (5 ml). After separation of the layers, the aqueous one is re-extracted with ethyl acetate. The organic layers are combined and extracted with brine. After drying over MgSO₄ and concentrating in vacuo, preparative thin layer chromatography on silica gel (eluting with ethyl acetate and extracting with 1:1 ethyl acetate-methylene chloride) provides the carbinol 5 (16 mg, 68% yield).

NMR (CDC1₃) 5.98 (m, -CH₂-CH=CH₂), 5.38 (m, -CH₂CH=CH₂), 4.87 (d, J=14Hz, 1 proton of AB pattern for CH₂S), 4.76 (m, -CH₂CH⁼CH₂), 4.25 (m, H₅+H₈), 4.15 (d, J=14Hz, 1 proton of AB pattern for CH₂S), 3.96 (s, N-CH₃), 3.38 (m, H₁), 3.32 (dd, J_{5,6}=3Hz, J_{6,8}=6Hz, H₆), 1.34 + 1.25 (2d, 1-CH₃ + CH₃CHOH-) in ppm downfield from TMS. IR (CH₂C1₂ solution) 1780, 1730 cm⁻¹. UV (dioxane) max = 293 nm. MS (FAB) 380 (M+1).

Step E:

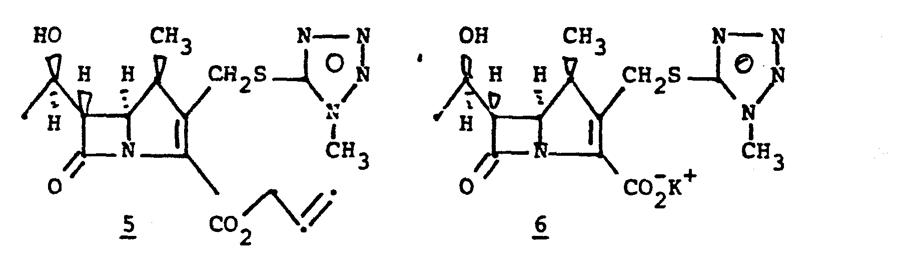

Triphenyl phosphine (2 mg, 0.008 mmol), tetrakis (triphenylphosphine) palladium (2.7 mg, 0.002 mmol) and 0.5M potassium 2-ethylhexanoate in ethyl acetate (93 ul, 0.05 mmol) are added to a stirred solution of the allyl ester carbapenem 5 (16 mg, 0.04 mmol) in 1:1 ethyl acetate-methylene chloride (1.6 ml) at ambient temperature under a nitrogen atmosphere. After 45 minutes the reaction mixture is concentrated in vacuo at ambient temperature. Two times the yellow powder is slurried in ether (1 ml) and the ether is pipetted off. The residue is dried in vacuo to an off-white powder. Water (400 µl) and 5% EtOH in water (400 ul) are added to this residue, and the clear supernatant (some yellow insoluble material settles to the bottom upon centrifugation) is applied to two 500 p RPS-F plates (Analtech). After elution in the cold with 5% EtOH in H₂0, the major band is extracted with 4:1 CH₃CN-H₂O (30 ml). Upon concentration (in vacuo below room temperature) to a volume of 5 ml, the solution is extracted 2 times with hexane (2 ml). The aqueous layer is then lyophilized at 0° to give 6, (1R,5S,6S,8R)-2-[[[1-methyl-1H-tetrazol-5-yl]-thio]-methyl]-6-(1-hydroxyethyl)-1-methylcarbapen-2- em-3-carboxylic acid, potassium salt (9.5 mg, 58% yield).

NMR (D₂0 with no internal standard) 4.80 (HDO), 4.78 (d, J=14_{H}z, 1 proton of AB pattern for CH₂S), 4.22 (m, H₈), 4.16 (dd, J_{5,6}=3Hz, J_{1,5}=10Hz, H₅), 4.06 (s, N-CH₃), 3.75 (dd, J_{5,6}=3Hz, J_{6,8}=Hz, H₆), 3.36 (m, H₁), 1.26 + 1.12 (2d, 1-CH₃ + CH₃CHOH-). UV (H₂0) max = 279 nm, =9,900.

### EXAMPLE 2

Alternate Preparation of Intermediate 3

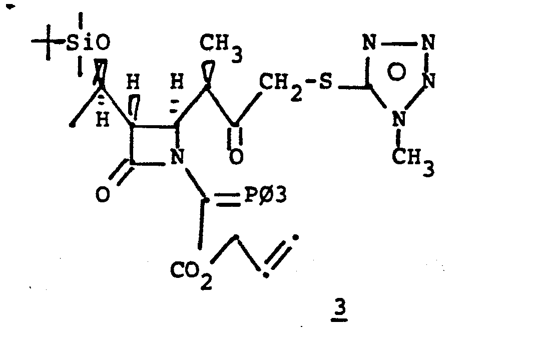

### Step A:

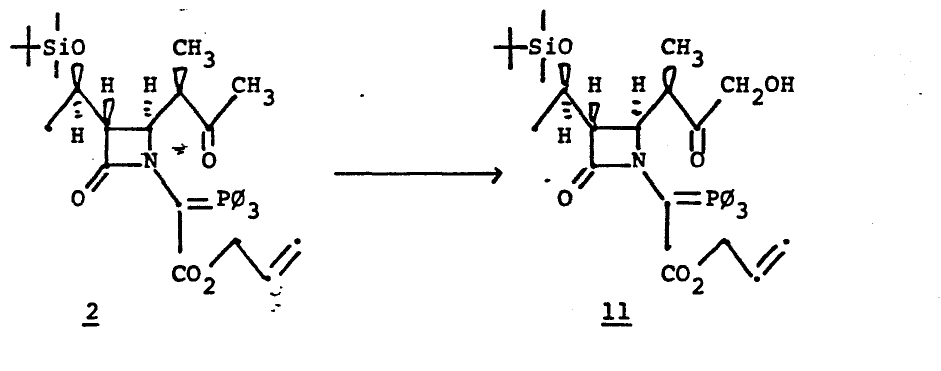

To diisopropyl amine (12 µl, 0.087 mmol) in tetrahydrofuran (1 ml) at 0° under nitrogen is added 1.3M BuLi in hexane (67 µl, 0.087 mmol). After 10 minutes at 0° the temperature is lowered to -73°, and a solution of methyl ketone 2 (38 mg, 0.058 mmol) in tetrahydrofuran (500 µl) is added. Following stirring 3 minutes at -78°, MOOS pyridine HMPA (MoOPH) (38 mg, 0.088 mmol) is added. The temperature is raised and held at -30° for 30 minutes. Upon addition of a saturated Na₂SO₃ solution (600 µl), 1M KH₂PO₄ (240 µl), water (2 ml) and ethyl acetate (2 ml) the cooling bath is removed, and the reaction mixture is stirred vigorously for 15 minutes. After phase separation, the aqueous layer is re-extracted with ethyl acetate. The combined organic layers are washed with brine, dried over MgSO₄ and concentrated to give a green oil (47 mg). Preparative thin layer chromatography on silica gel (plate eluted with 1:1 ethyl acetate-hexane and extracted with 1:1 ethyl acetate-methylene chloride) provides recovered starting material (10 mg) and the hydroxymethyl ketone 11 (13 mg, 32% yield).

NMR (CDC1₃) selected absorbances 8-7.4 (aromatic protons), 6.0 (m, -CH₂CH=CH₂), 4.4 (m, _{CH20H)}, ₀.₈ (C(CH₃)₃) in ppₘ downfield from TMS.

IR (CH₂Cl₂ solution) 1755, 1725, 1650, 1625 cm⁻¹. MS (FAB) 674 (M+l), 262 (φ₃P).

### Step B:

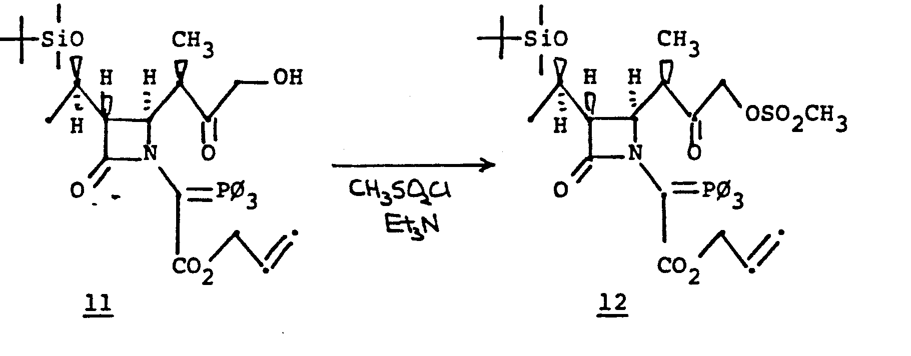

Triethylamine (2.9 ul, 0.021 mmol) and methanesulfonyl chloride (1.6 ul, 0.021 mmol) are added to the hydroxymethylketone 11 (13 mg, 0.019 mmol) in anhydrous methylene chloride (500 ul) at 0° under nitrogen. After stirring for one hour the reaction mixture is added to brine (2 ml) and methylene chloride (2 ml) containing pH 7 phosphate buffer (200 µl, 0.5M). After phase separation, the aqueous layer is again extracted with methylene chloride. The combined organic layers are washed with brine, dried over MgSO₄ and concentrated to give 12 mg crude mesylate. Preparative thin layer chromatography on silica gel (eluting with 1:1 ethyl acetate-hexane and extracting with 1:1 ethyl acetate-methylene chloride) provides the mesylate 12 (7 mg, 50% yield).

NMR (CDC1₃) selected absorbances 7.9-7.4 (aromatic protons), E.0 (m, -CH₂CH-CH₂), 3.22 (OSO₂CH₃), 0.8 (C(CH₃)₃) in ppm downfield from TMS.

IR (CH₂C1₂ solution) 1740, 1640, 1620 (sh) cm⁻¹.

### Step C:

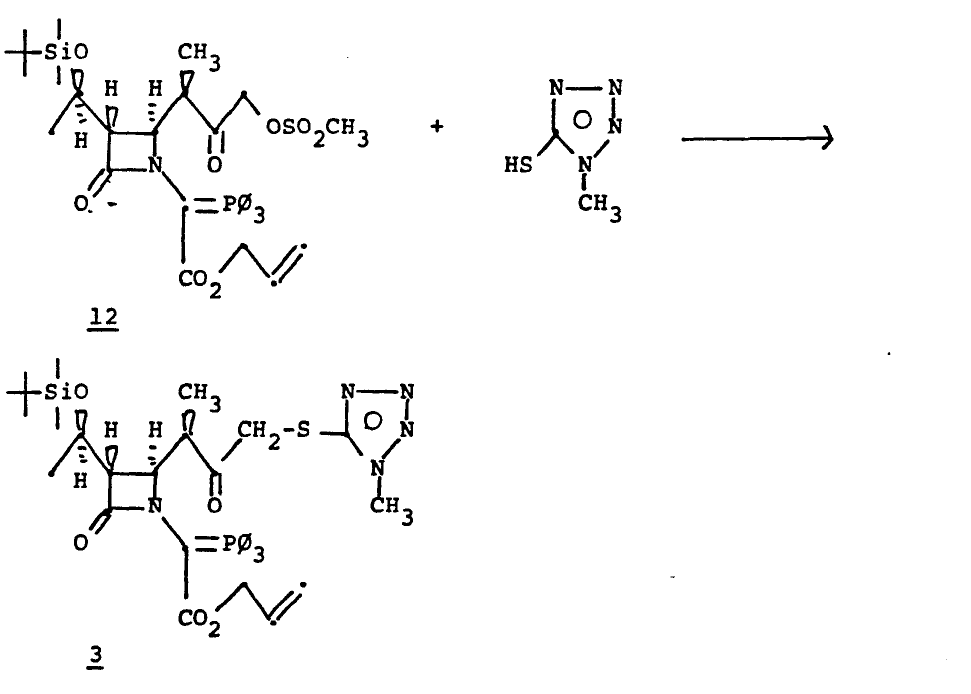

Triethylamine (1.6 ul, 0.012 mmol) and 5-mercapto-l-methyltetrazole (1.3 mg, 0.011 mmol) are added to the mesylate 12 (7 mg, 0.01 mmol) in dimethylformamide (100 µl). After stirring at ambient temperature for 3.5 hours, the reaction mixture was added to 1M K₂HP0₄ (100 µl), brine (2 ml) and ethyl acetate (2 ml). After phase separation, the aqueous layer is,washed again with ethyl acetate. The combined organic layers are washed with brine, dried over MgSO₄ and concentrated to give the crude product (8 mg). Preparative thin layer chromatography on silica gel (eluting with 1:1 ethyl acetate-hexane and extracting with 1:1 ethyl acetate-methylene chloride) provides the purified tetrazole 3 (4 mg, 56% yield) identical to that prepared in

### Example 1, Step B.

### EXAMPLE 3

### Preparation of (1R,5S,6S,8R)-2-phenylthiomethyl-6-(1- hyroxyethyl)-1-methylcarbapen-2-em-3-carboxylic acid, potassium salt

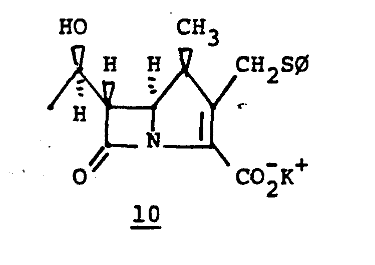

Step A:

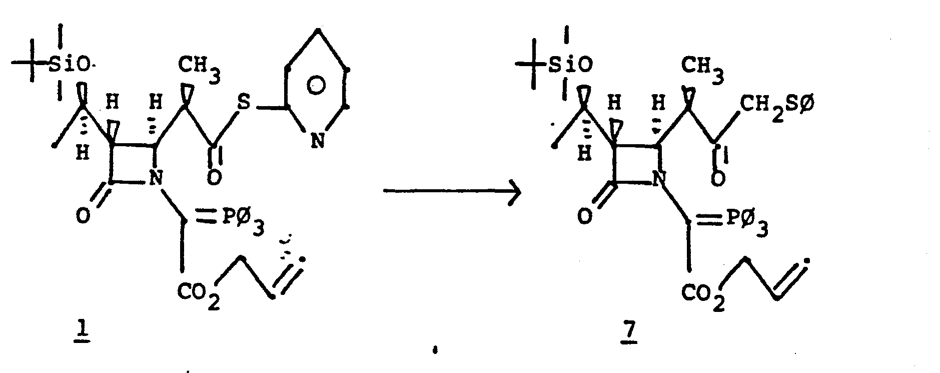

Chloromethylphenylsulfide (269 ul, 2.0 mmol) is added to magnesium shavings (53 mg, 2.2 mmol) stirred in anhydrous tetrahydrofuran (4 ml) under a nitrogen atmosphere. After 1.25 hours at ambient temperature, the reaction mixture is heated at 50° under a reflux condenser for 30 minutes. After cooling to ambient temperature, the above Grignard solution (250 µl, 0.12 mmol) is added to a solution of thiopyridyl ester 1 (81 mg, 0.11 mmol) in anhydrous tetrahydrofuran (1.75 ml) at 0° under nitrogen with stirring. After 15 minutes, the reaction mixture is worked up as Example 1-Step A to give the crude product (97 mg). Preparative thin layer chromatography on silica gel (eluting with 1:1 ethyl acetate-hexane and extracting with 1:1 ethyl acetate-methylene chloride) provides the phenylthiomethyl ketone 7 (59 mg, 75% yield).

NMR (CDC1₃) selected absorbances 8.0-7.0 (aromatic protons), 5.97 (m, -CH₂CH=CH₂), 3.82 (m, CH2Sφ), 0.82 (C(CH₃)₃) in ppm downfield from TMS.

IR (CH₂C1₂ solution) 1750, 1725, 1650, 1625 cm⁻¹. MS (FAB) 766 (M+1).

### Step B:

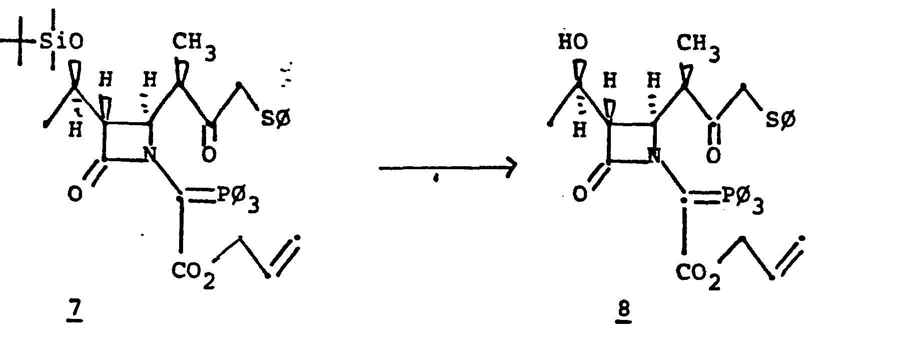

The silyl-protected material 7 (33 mg, 0.043 mmol) is stirred at ambient temperature under nitrogen in a solution of 0.2M HCI in 9:1 MeOH H₂0 (1.8 ml) for 6 hours. The reaction mixture is added to 1M K₂HPO₄ (860 µl), 1M KH₂ PO₄ (500 µl), H₂0, and ethyl acetate. After separation of the layers the aqueous layer is re-extracted with ethyl acetate. The combined organic layers are washed with brine, dried over MgSO₄ and concentrated in vacuo to give the crude material (33 mg). Preparative thin layer chromatography on silica gel (eluting with 1:1 ethyl acetate-methylene chloride and extracting with 1:9 methanol-methylene chloride) provides the carbinol 8 (21 mg, 75% yield).

NMR (CDC1₃) selected absorbances 8.0-7.2 (aromatic

protons), 6.02 (m, -CH₂CH=CH₂), 3.74 (CH₂Sφ) in_ppm downfield from TMS. IR (CH₂C1₂ solution) 1750, 1700, 1650, 16₂₅ cm⁻¹. MS (FAB) 652 (M+l), 262 (φ₃P).

### Step C:

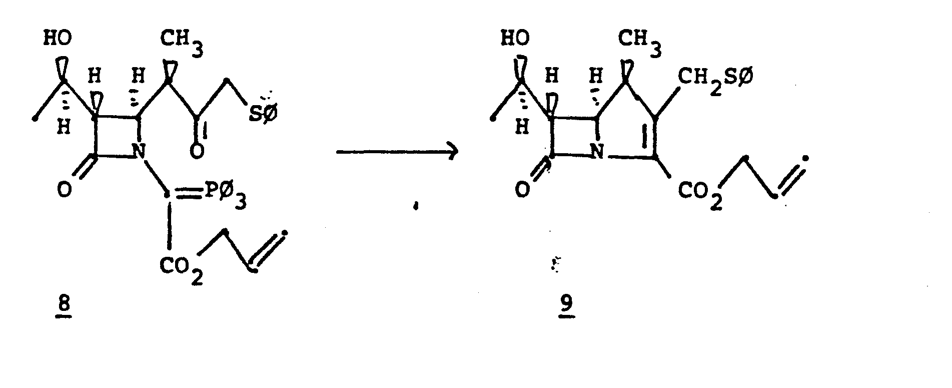

A solution of 8 (20 mg, 0.03 mmol) in toluene (2 ml) is heated at reflux under nitrogen for 6 hours and then concentrated in vacuo to a colorless oil. Preparative thin layer chromatography on silica gel (eluting with 1:4 ethyl acetate-methylene chloride and extracting with 1:9 methanol-methylene chloride) provides the carbapenem 9 (8 mg, 70% yield).

NMR (CDC1₃) 7.5-7.2 (aromatic protons), 5.92 (m, -CH₂CH=CH₂), 5.33 (m, -CH₂CH=_{CH2}), ₄.84 (d, J=14Hz, 1 proton of AB pattern for CH₂S), 4.64 (m, CH₂CH=CH₂), 4.24 (m, H₈), 4.16 (dd, J_{5,6}=3Hz, J_{1,5}=10Hz, H₅), 3.44 (m, H₁), 3.24 (^{dd,} J_{5,6}=3Hz, J_{6, 8}=6Hz, H₆), 1.8 (d, OH), 1.33 + 1.15 (2d, 1-CH₃ + CH₃CHOH-) in ppm downfield from TMS.

IR (CH₂Cl₂ solution) 1780, 172₅ cm⁻¹. MS (FAB) 374 (M+1).

### Step D:

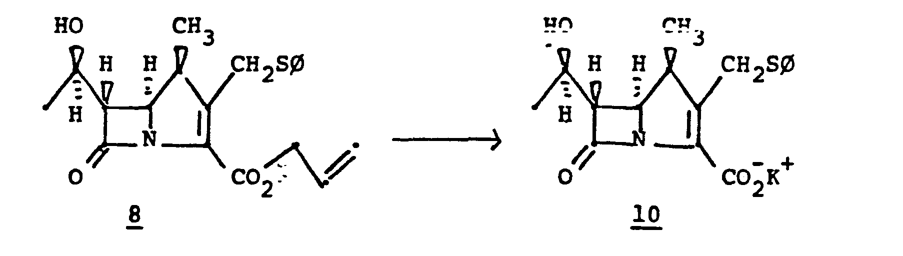

The allyl ester 9 (19 mg, 0.05 mmol) is deblocked in a manner analogous to Example 1, Step E. The reaction mixture is concentrated in vacuo at ambient temperature. The residue is dissolved in methylene chloride (400 ul) and applied to a 500 p RPS-F plate (Analtech). Elution with 20% EtOH in H₂0 in the cold is followed by extraction of the major band with 4:1 CH₃CN-H₂0 (25 ml). The extracting solution is treated in a manner analogous to Example 1, Step E and lyophilized at 0° to give (1R,5S.,6S,8R)-2-phenylthiomethyl-6-(1-hydroxyethyl)-l-methylcarbapen-2-em-3-carboxylic acid, potassium salt (14 mg, 75% yield).

NMR (D₂0 with no internal standard) 7.6-7.3 (aromatic protons), 4.74 (d, J=14Hz, 1 proton of AB pattern for CH₂S), 4.2 (m, H₈), 4.02 (dd, J_{5,6}=2.5Hz, J_{1,5}=9.5HZ, H₅), 3.46 (d, J=14Hz, 1 proton of AB pattern for CH₂S), 3.37 (m, H₁+H₆), 1.26 + 1.10 (2d, 1-CH₃ + CH₃CHOH-).

UV (H₂0) 273 nm ( =9,400) 248 nm ( =8,300).

### EXAMPLE 4

### Preparation of Allyl (1R,5S,6S,8R)-2-hydroxvmethyl-6-(1-t-butyldimethylsilyloxyethyl)-1-methylcarbapen-2- em-3-carboxylate

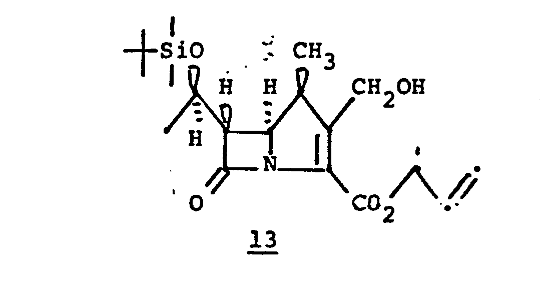

Step A:

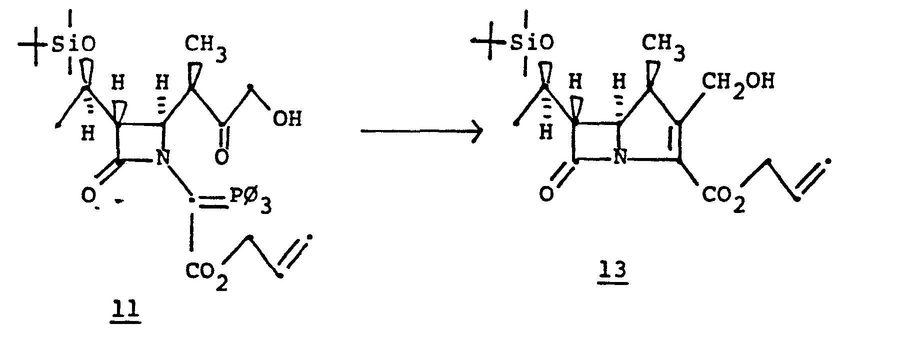

A solution of 11 (12 mg, 0.018 mmol) in toluene (1 ml) is heated at reflux under nitrogen for one hour and then concentrated to a colorless oil. Preparative thin layer chromatography on silica gel (plate eluted with 1:1 ethyl acetate-hexane and extracted with 1:1 ethyl acetate-methylene chloride) gives the carbapenem 13 (5 mg, 70% yield).

NMR (CDC1₃) 5.98 (m, -CH₂CH=CH₂), 5.39 (m, CH2CH=CH2), 4.80 (m, -CH₂CH=CH₂), 4.47 (m, CH₂OH), 4.22 (m, H₅⁺H₈), 3.25 (^{m, H}₁, H₆+OH), 1.26 and 1.21 (sd, 1-CH₃ and CH₃CHO-), 0.9 (C(CH₃)₃), 0.07 (Si(CH₃)₂) in ppm downfield from TMS.

IR (CH₂Cl₂ solution) 1775, 1710 cm⁻¹. UV (dioxane) max = 283 nm/ MS (FAB).

Compound 13 may be activated using the methodology described in Example 2, Step B. The resulting activated intermediate may be further elaborated via methodology in Example 2, Step C to provide compound 4 and similar resulting thio analogs.

## Claims

1. A compound having the formula: 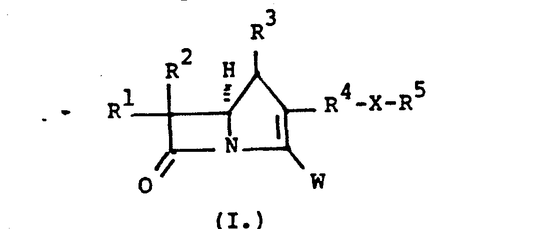
wherein R1 and R2 are independently H, CH₃-, CH₃CH₂-, (CH₃ ) ₂CH-, HOCH₂-, CH₃CH(OH)-, (CH₃)₂C(OH)-, FCH₂-, F₂CH-, F₃C-, CH₃CH(F)-, CH₃CF₂-, or (CH₃)₂C(F)-:
_{R}³ is C^{H}₃^{;}
R⁴ is a bridging group comprising substituted or unsubstituted C₁-C₄ straight, C₂-C₆ branched or C₃-C₇ cycloalkyl groups wherein the substituents are selected from C₁-C₆ alkyl, 0-C₁-C₆ alkyl, S-C₁= C₆ alkyl, CF₃, N(C₁-C₆ ^{alkyl})₂:
X is -S-, 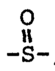
, -SO₂-, -O-, or -NH-;
R is selected from the group consisting of:

1. Aliphatic
-C₁-C₁₀ straight or branched alkyl; C₃-C₈ cycloalkyl; C₂-C₁₀ straight or branched alkenyl;
C₃-C₈ cycloalkenyl; or C₂-C₁₀ straight or branched alkynyl;

2. Substituted Aliphatic
-C₁-C₁₀ branched or unbranched alkyl; C₂-C₁₀ branched or unbranched alkenyl, or akynyl; or C₃-C₈ cycloalkyl or cycloalkenyl; wherein all of the above groups are substituted by one or more halo, ORₐ, 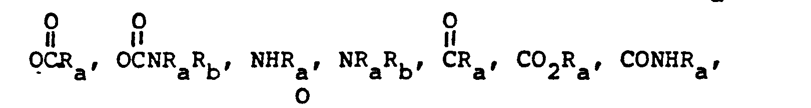 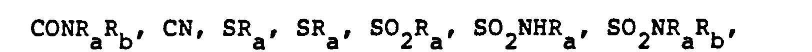 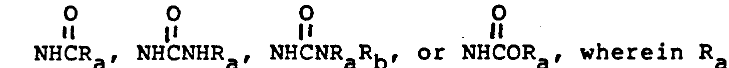
and Rb are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms;
heteroalkyl, heteroaralkyl, heterocyclyo and heterocyclyoalkyl and wherein the heteroatom or atoms in the above-named heterocyclic moieties are selected from the group consisting of 1-4 oxygen, nitrogen or sulfur atoms and wherein the alkyl moieties associated with said heterocyclic moieties have 1-6 carbon atoms;

3. Aryl
-phenyl; or phenyl substituted with 1-3 substituents selected from the group consisting of those substituents set forth above under "Substituted Aliphatic";

4. Heterocyclic
-mono- or bicyclic heterocyclic group containing from 5 to 11 total atoms of which 1 to 5 are heteroatoms selected from nitrogen, oxygen, and sulfur; or substituted heterocyclic where heterocyclic is as defined immediately above and the substituents are 1 to 3 in number and are selected from the group consisting of those substituents set forth above under "Substituted Aliphatic";

5. Arylaliphatic
-the group: -Y-Aryl, where "Aryl" is as defined above, including substituted aryl; and Y is C₁-C₆ straight or branched alkyl, C₃-C₈ cycloalkyl, or C₂-C₆ alkenyl or alkynyl;

6. Heterocycloaliphatic
-the group: -Y-Heterocyclic, where "Heterocyclic" is as defined above, including substituted heterocyclic; and Y is as defined above;

7. Alkyl-Heteroatom-Alkyl
-the group:-(CH₂)ₙ X (CFl₂)_{n'} R_{c'} where n and n' are independently 2-4; X is NR, O, or S where R is _{H}, _{CH3}, _{CH2CH3}, _{CH2CH20H}, or CH₂CH₂NH_{2;} and R is OH, NH₂, NHCH₃, N(CH₃)₂, 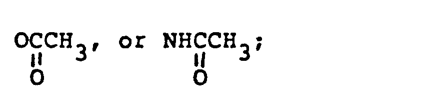
provided that the methylene carbons of the group formula may additionally be substituted with C₁-C₃ alkyl to form branched alkyl groups;

8. Heteroarylium
-the group: 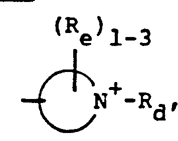 which is a mono- or bicyclic heteroarylium group containing from 5-11 ring atoms of which up to 5 are heteroatoms wherein R_{d} is:
1) an unsubstituted or substituted C₁-C₆ alkyl radical;
2) an unsubstituted or substituted ^{C}₂-C₆ alkenyl radical;
3) an unsubstituted or substituted C₂-C₆ alkynyl radical;
4) a C₃-C₇ cycloalkyl radical in which the ring is substituted or unsubstituted and one or more atoms may be replaced by a heteroatom;
5) a C₃-C₇ cycloalkyl methyl radical in which the ring may be substituted and one or more atoms may be replaced by a heteroatom;
6) an unsubstituted or substituted C_{S}-C₇ cycloalkenyl radical;
7) an unsubstituted or substituted bivalent C₂-C₆ alkylidene radical, optionally interrupted by a heteroatom, and joined to the heteroarylium group to form a ring which is carbocyclic or in which one or more atoms is replaced by a heteroatom. The new ring may contain one or more double bonds;
8) an unsubstituted or substituted phenyl or heteroaryl radical;
9) an unsubstituted or substituted phenyl (C₁-C₄ alkyl) or heteroaryl (C₁-C₄ alkyl) radical;
10) a cyano (C₁-C₄ alkyl) radical;
11) a carboxy (C₁-C₄ alkyl) radical;
12) a sulfo (C₁-C₄ alkyl) radical;
13) a carbamoyl (C₁-C₄ alkyl) radical;
14) a phosphonyl (C₁-C₄ alkyl) radical;
15) a hydroxy (C₁-C₄ alkyl) radical;
16) an amino (C₁-C₄ alkyl) radical in which the nitrogen atom is unsubstituted or substituted with one to three C₁-C₄ alkyl groups; wherein the substituents in the above definitions of R_{d} are independently selected from the group consisting of the definitions of Rₑ set out below;
Rₑ is independently selected from:
a) a trifluoromethyl group;
b) a halogen atom;
c) an unsubstituted or substituted C₁-C₄ alkoxyl radical;
d) a hydroxy group;
e) an unsubstituted or substituted (C₁-C₆ alkyl) carbonyloxy radical;
f) a carbamoyloxy radical which is unsubstituted or substituted on nitrogen with one or two Cₗ-C₄ alkyl groups;
g) a Cₗ-C₆ alkylthio radical, C₁-C₆ alkylsulfinyl radical or Cₗ-C₆ alkylsulfonyl radical, each of which is unsubstituted or substituted on the alkyl group;
h) a sulfo group;
i) a sulfamoyl group which is unsubstituted or substituted on nitrogen by one or two C₁-C₄ alkyl groups;
j) an amino group;
k) a mono (C₁-C₄ alkyl) amino or di(C₁-C₄ alkyl)amino group, each of which is unsubstituted or substituted on the alkyl group;
1) a formylamino group;
m) an unsubstituted or substituted (Cₗ-C₆ alkyl)carbonylamino radical;
n) a (C₁-C₄ alkoxyl) carbonylamino radical;
o) a ureido group in which the terminal nitrogen is unsubstituted or substituted with one or two C₁-C₄ alkyl groups;
p) an arylsulfonamido or a (C₁-C₆ alkyl)sulfonamido group;
q) a cyano group;
r) a formyl or acetalized formyl radical;
s) an unsubstituted or substituted (C₁-C₆ alkyl)carbonyl radical wherein the carbonyl is free or acetalized;
t) an unsubstituted or substituted phenylcarbonyl or heteroarylcarbonyl radical;
u) a hydroximinomethyl radical in which the oxygen or carbon atom is optionally substituted by a Cₗ-C₄ alkyl group;
v) a carboxyl group;
w) a (Cₗ-C₆ alkoxy)carbonyl radical;
x) a carbamoyl radical which is unsubstituted or substituted on nitrogen by one or two C₁-C₄ alkyl groups;
y) an N-hydroxycarbamoyl or N(Cₗ-C₄ alkoxy)carbamoyl radical in which the nitrogen atom may be additionally substituted by a Cₗ-C₄ alkyl group;
z) a thiocarbamoyl group;
aa) a 5-(lH)-tetrazolyl group;
ab) an amidino group 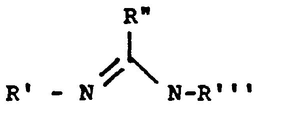 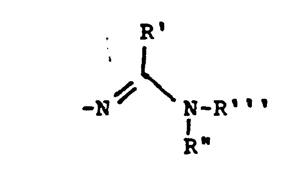 where R', R" and R"' are independently hydrogen, Cₗ-C₄alkyl or wherein two of the alkyl groups together form a C₂-C₆alkylidene radical optionally interrupted by a heteroatom and joined together to form a ring;
ac) a carboxamidino group 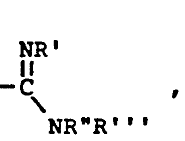 where R', R" and R"' are as defined above;
ad) a guanidinyl group where R" in ab) above is NR^{iv}R^{v} and R^{iv} and R^{v} are as defined for R' through R"' above;
ae) a phosphonate group -P(O)(O⁻)OR^{vi} where _{R}^{vi} is C₁-C₃alkyl;
af) an alkyl phosphonate group -(CH2)ₙP(O)(O⁻)(OR^{vi})where n =1 to 3 and R^{vi} is C₁-C₃alkyl;
ag) hydrogen;
ah) an unsubstituted or substituted C₁-C₆ alkyl radical;
ai) an unsubstituted or substituted C₂-C₆ alkenyl radical;
aj) an unsubstituted or substituted C₂-C₆ alkynyl radical;
ak) a C₃-C₇ cycloalkyl radical in which the ring is substituted or unsubstituted and one or more atoms may be replaced by a heteroatom;
al) a C₃-C₇ cycloalkyl methyl radical in which the ring may be substituted and one or more atoms may be replaced by a heteroatom;
am) an unsubstituted or substituted C₅-C₇ cycloalkenyl radical;
an) an unsubstituted or substituted phenyl or heteroaryl radical;
ao) an unsubstituted or substituted phenyl (C₁-C₄ alkyi) or heteroaryl (^{C}₁-C₄ alkyl) radical; and

9. Heteroaryliumaliphatic
-the group: -Y-Heteroarylium, where Y is as defined above; and "Heteroarylium" is as defined above and additionally includes the group: 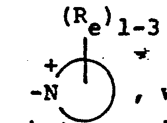
which is a mono- or bicyclic
heteroarylium group containing from 5-11 ring atoms of which up to 5 are heteroatoms, where Rₑ is as defined above;

10. Amidino and Amidinium 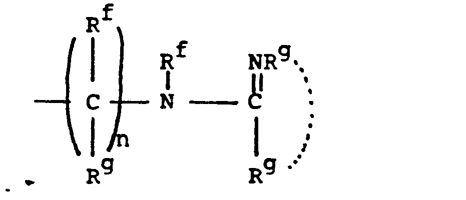 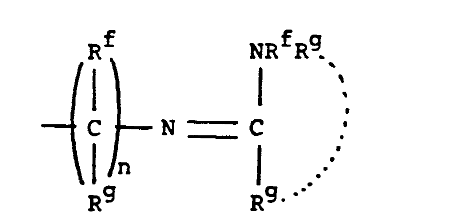 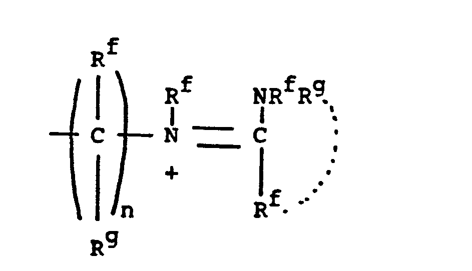
and wherein n = 2-6; R^{f} and R⁹ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heterocyclic groups as defined above, including heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; and the dotted line indicates provision for the ring formed by the joinder of substituents carried by the imino carbon atoms; 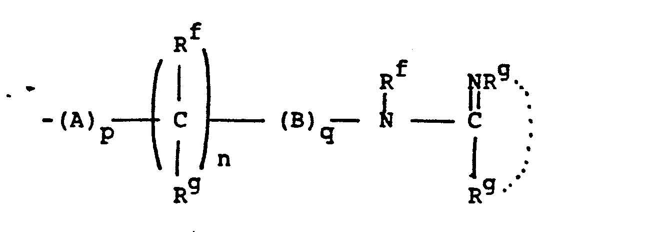 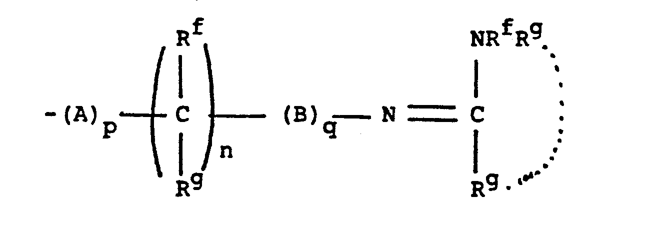 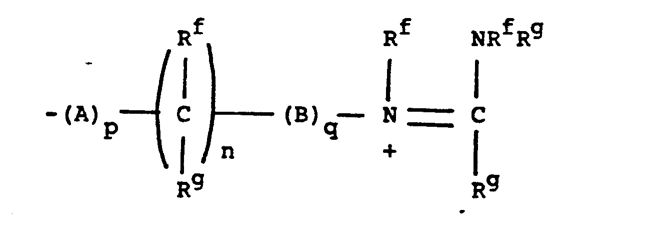
wherein R^{f}, R⁹ and n are as defined immediately above; p and q are 0 or 1; A and B are in bivalent form and are selected from the following definitions above of R⁵: cycloalkyl, alkenyl, cycloalkenyl, alkynyl; substituted: cycloalkyl, alkenyl, cycloalkenyl, alkynyl; phenyl and substituted phenyl; substituted and unsubstituted heteroaryl; arylaliphatic; heteroarylaliphatic, heterocyclylo- aliphatic, and heterocyclic; and alkyl-heteroatom-alkyl; and B can also be selected from -0- and -NR^{f}-:

11. Guanidino and Guanidinium 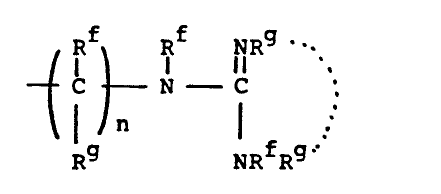 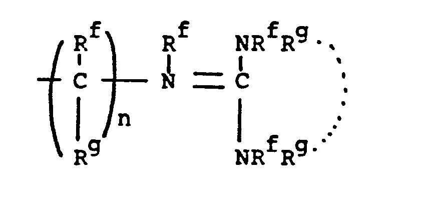 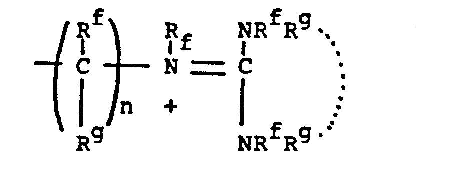
and wherein n = 2-6; R^{f} and R⁹ are as initially defined above under "Amidino and Amidinium"; and the dotted line indicates provision for the ring formed by the joinder of substituents carried by the imino carbon atoms; 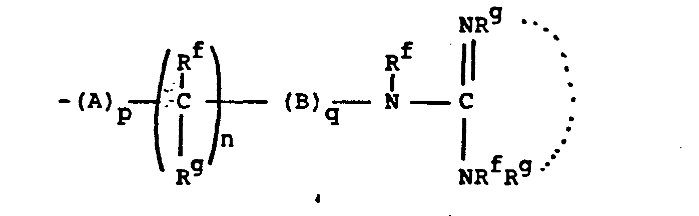 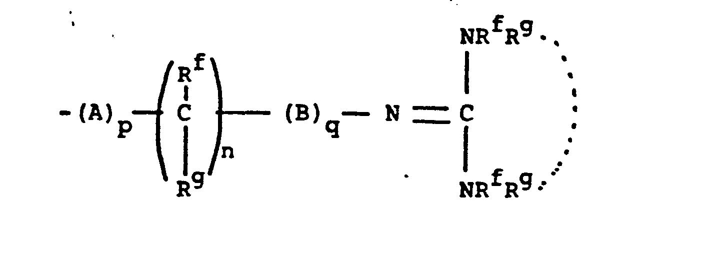 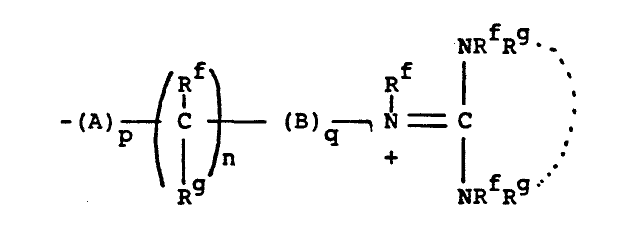
wherein R^{f} and R^{g} and n are as defined immediately above; p and q are 0 or 1; A and B are as defined above under "Amidino and Amidinium";

12. Carbamimidoyl and Carbamimidinium 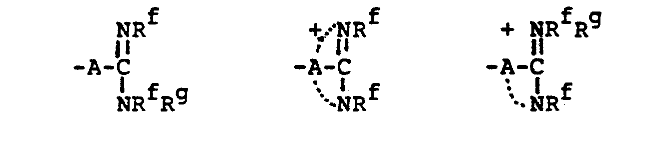 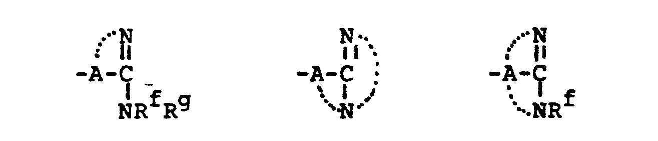 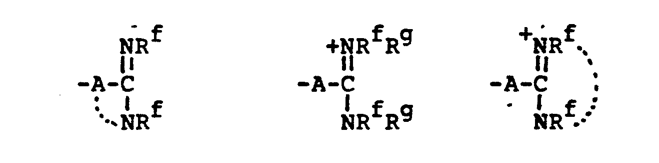 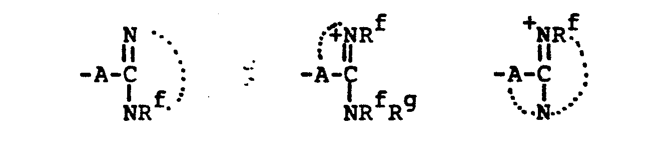
and wherein R^{f} and R^{g} are as defined above under "Amidino and Amidinium''; the two nitrogen atoms demonstrated in the above structure may participate in cyclic structures which are indicated by the dotted lines; and A is a connecting group between the sulfur atom and carbamimidoyl function which is bivalent, cyclic or acyclic, is to be read both left to right and right to left, and is selected from: substituted and unsubstituted: loweralkyl having from 1-6 carbon atoms; cycloalkyl having from 3-10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms, and the alkyl moiety comprises 1 to 6 carbon atoms; alkynyl having from 2 to 10 carbon atoms; aryl, which is defined as phenyl and naphthyl; arylalkyl and alkylaryl; heteroalkyl, alkylheteroalkyl, arylheteroalkyl and alkylheteroaryl wherein the heteroatoms are selected from the group of sulfur, oxygen and nitrogen, and the alkyl moiety has 1 to 6 carbon atoms, and the aryl moiety is phenyl; heterocyclyo comprising mono- and bicyclic structures having 5 to 10 ring atoms wherein one or more of the heteroatoms is selected from oxygen, nitrogen, or sulphur; heterocycloalkyl wherein the heterocyclyo moiety comprises from 3 to 10 atoms and the alkyl moiety comprises from 1 to 6 atoms; the substituent (or substituents) relative to the above-named radicals are selected from the group consisting of amino, hydroxyl, cyano, carboxyl, nitro, chloro, bromo, fluoro; alkoxy having from 1 to 6 carbon atoms, mercapto, haloloweralkyl, and alkylthio having from 1-6 carbon atoms; and
W is selected from: i) COOH or a pharmaceutically acceptable ester or salt thereof,
ii) COOR wherein R is a removable carboxy protecting group,
iii) COOM wherein M is an alkali metal, or
iv) COO⁻; provided that when W is other than iv) a counterion Z is provided.

2. A compound of Claim 1. having the configuration and formula:

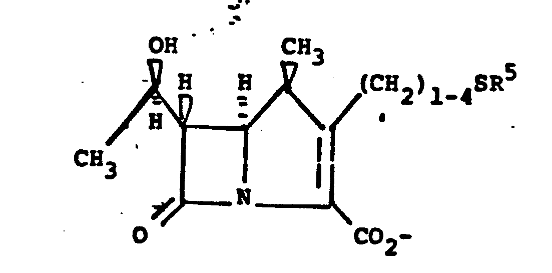

3. A compound of Claim 2 wherein said R⁵ is 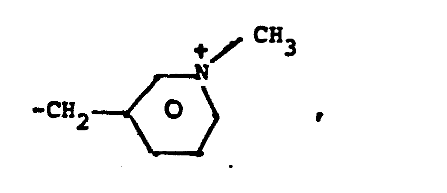 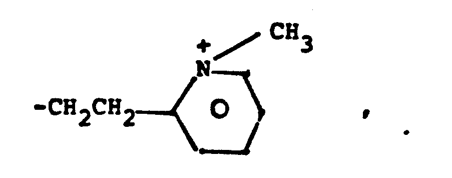 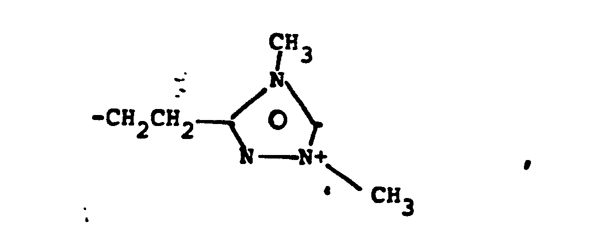 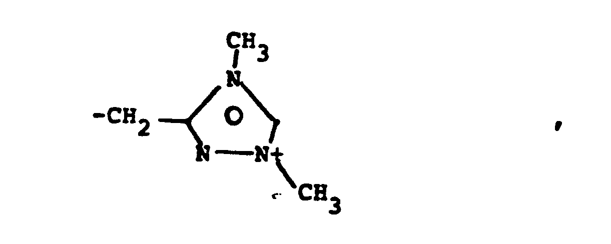 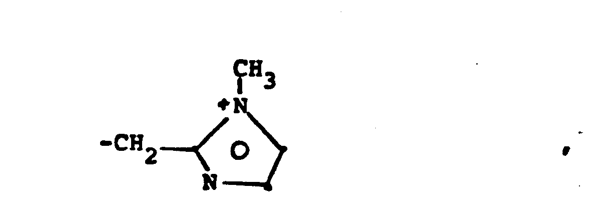 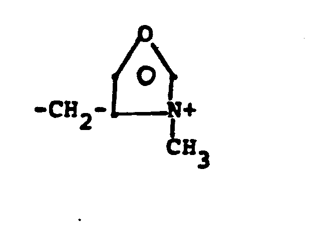 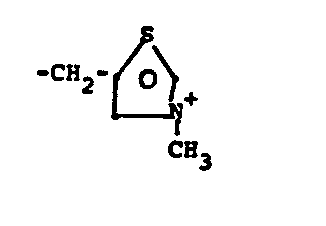 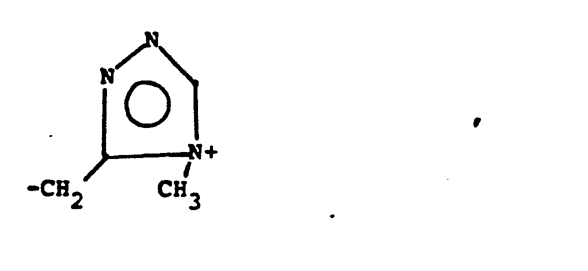 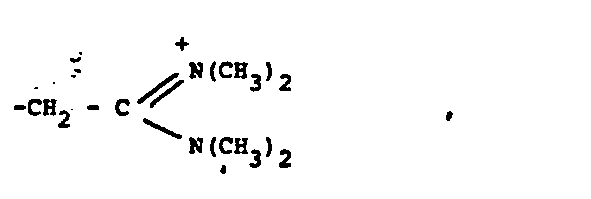 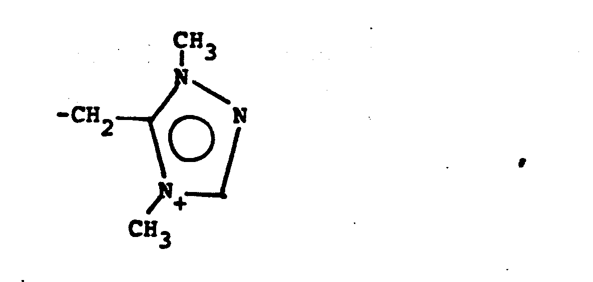 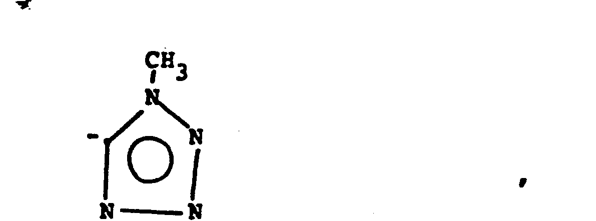 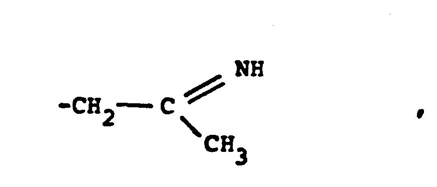
or

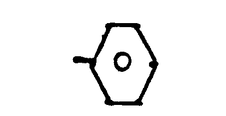

4. The combination of a compound of Claim I and a DHP fnhibitor.

5. A combination of Claim 4 wherein the DHP inhibitor is 7-(L-2-amino-2-carboxyethylthio)-2-(2,2-dimethylcyclopropanecarboxamfdo)-2-heptertoic acid.

6. A pharmaceutical composition for antibiotic use comprising an antibacterially effective amount of a compound of Claim 1, an inhibitorily effective amount of a DHP inhibitor, and optionally, a pharmaceutically acceptable carrier.

7. A pharmaceutical composition for antibiotic use comprising an antibacterially effective amount of a compound of Claim 1, and, optionally, a pharmaceutically acceptable carrier.
